# EUROPEAN PATENT SPECIFICATION

(11) **EP 4 041 729 B1**
(45) Date of publication and mention of the grant of the patent: **27.09.2023**
(21) Application number: 20792740.1
(22) Date of filing: 06.10.2020
(51) Int. Cl.: C07D 471/04, C07D 401/14, C07D 403/14, A61P 31/18, A61K 31/519

(54) **INHIBITORS OF HUMAN IMMUNODEFICIENCY VIRUS REPLICATION**
INHIBITOREN DER NEUBILDUNG MENSCHLICHER IMMUNDEFEKTVIREN
INHIBITEURS DE LA RÉPLICATION DU VIRUS DE L'IMMUNODÉFICIENCE HUMAINE

(30) Priority: 08.10.2019 US 201962912215 P
(43) Date of publication of application: 17.08.2022
(73) Proprietor: VIIV Healthcare UK (No.5) Limited, Stevenage SG1 2NY (GB)
(72) Inventor: BOWSHER, Michael S., Wallingford, Connecticut 06492 (US); PARCELLA, Kyle E., Branford, Connecticut 06405 (US); IWUAGWU, Christiana, Wallingford, Connecticut 06492 (US); GILLIS, Eric P, Branford, Connecticut 06405 (US)
(74) Representative: Matley, Joshua Ellis
(86) International application number: PCT/IB2020/059377
(87) International publication number: WO 2021/070054

(56) References cited:
- WO-A1-2018/203235
- WO-A1-2019/198024
- WO-A1-2020/084492

## Description

### FIELD OF THE INVENTION

The invention relates to compounds, compositions, and methods for the treatment of human immunodeficiency virus (HIV) infection. More particularly, the invention provides novel Capsid inhibitors, pharmaceutical compositions containing such compounds, and methods for using these compounds in the treatment of HIV infection. The invention also relates to methods for making the compounds hereinafter described.

### BACKGROUND OF THE INVENTION

Acquired immunodeficiency syndrome (AIDS) is the result of infection by HIV. HIV continues to be a major global public health issue. In 2015, an estimated 36.7 million people were living with HIV (including 1.8 million children) - a global HIV prevalence of 0.8%. The vast majority of this number live in low- and middle- income countries. In the same year, 1.1 million people died of AIDS-related illnesses.

Current therapy for HIV-infected individuals consists of a combination of approved anti-retroviral agents. Close to four dozen drugs are currently approved for HIV infection, either as single agents, fixed dose combinations or single tablet regimens; the latter two containing 2-4 approved agents. These agents belong to a number of different classes, targeting either a viral enzyme or the function of a viral protein during the virus replication cycle. Thus, agents are classified as either nucleotide reverse transcriptase inhibitors (NRTIs), non-nucleotide reverse transcriptase inhibitors (NNRTIs), protease inhibitors (PIs), integrase strand transfer inhibitors (INSTIs), or entry inhibitors (one, maraviroc, targets the host CCR5 protein, while the other, enfuvirtide, is a peptide that targets the gp41 region of the viral gp160 protein). In addition, a pharmacokinetic enhancer (cobicistat or ritonavir) can be used in combinations with antiretroviral agents (ARVs) that require boosting.

Despite the armamentarium of agents and drug combinations, there remains a medical need for new anti-retroviral agents. High viral heterogeneity, drug-associated toxicity, tolerability problems, and poor adherence can all lead to treatment failure and may result in the selection of viruses with mutations that confer resistance to one or more antiretroviral agents or even multiple drugs from an entire class (Beyrer, C., Pozniak A. HIV drug resistance - an emerging threat to epidemic control. N. Engl. J. Med. 2017, 377, 1605-1607; Gupta, R. K., Gregson J., et al. HIV-1 drug resistance before initiation or re-initiation of first-line antiretroviral therapy in low-income and middle-income countries: a systematic review and meta-regression analysis. Lancet Infect. Dis. 2017, 18, 346-355; Zazzi, M., Hu, H., Prosperi, M. The global burden of HIV-1 drug resistance in the past 20 years. PeerJ. 2018, DOI 10.7717/peerj.4848). As a result, new drugs are needed that are easier to take, have high genetic barriers to the development of resistance and have improved safety over current agents. In this panoply of choices, novel mechanisms of action (MOAs) that can be used as part of the preferred antiretroviral therapy (ART) can still have a major role to play since they should be effective against viruses resistant to current agents.

Certain potentially therapeutic compounds have now been described in the art and set forth in Blair, Wade S. et.al. Antimicrobial Agents and Chemotherapy (2009), 53(12), 5080-5087, Blair, Wade S. et al. PLoS Pathogens (2010), 6(12), e1001220, Thenin-Houssier, Suzie; Valente, Susana T. Current HIV Research, 2016, 14, 270-282, and PCT Patent applications with the following numbers: WO 2012065062, WO 2013006738, WO 2013006792, WO 2014110296, WO 2014110297, WO 2014110298, WO 2014134566, WO 2015130964, WO2015130966, WO 2016033243, WO2018035359, WO2018203235, WO 2019161017, and WO 2019161280.

What is now needed in the art are additional compounds which are novel and useful in the treatment of HIV. Additionally, these compounds should provide advantages for pharmaceutical uses, for example, with regard to one or more of their mechanisms of action, binding, inhibition efficacy, target selectivity, solubility, safety profiles, bioavailability or reduced frequency of dosing. Also needed are new formulations and methods of treatment which utilize these compounds.

### SUMMARY OF THE INVENTION

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

Briefly, in one aspect, the present invention discloses a compound or salt selected from the group consisting of: 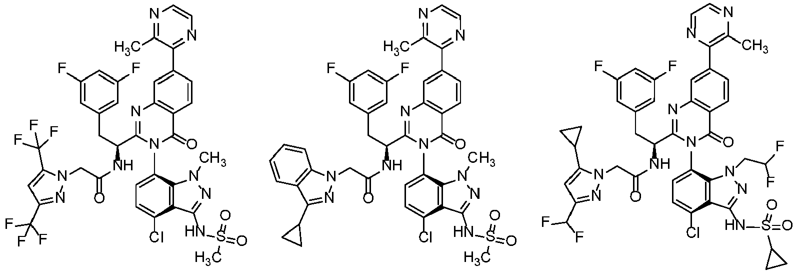 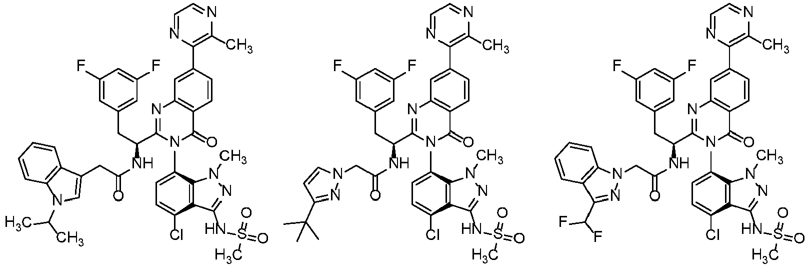 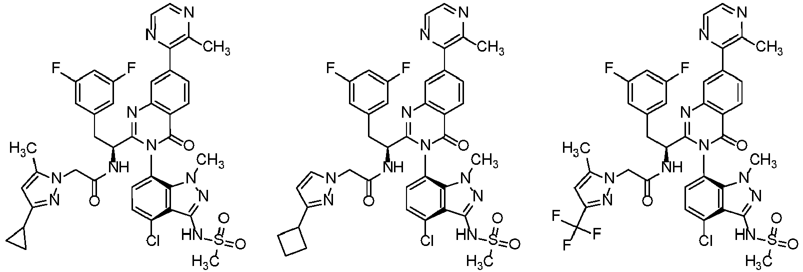 and pharmaceutically acceptable salts thereof.

In another aspect, the present invention discloses a compound or salt selected from the group consisting of: 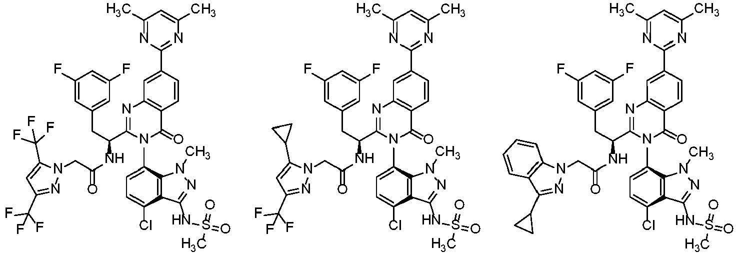 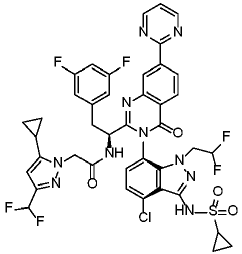 and pharmaceutically acceptable salts thereof.

In another aspect, the present invention discloses a compound or salt selected from the group consisting of: 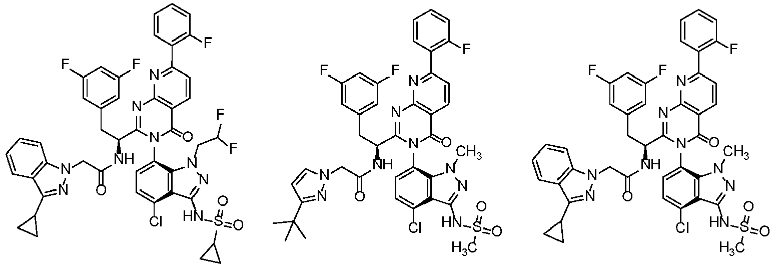 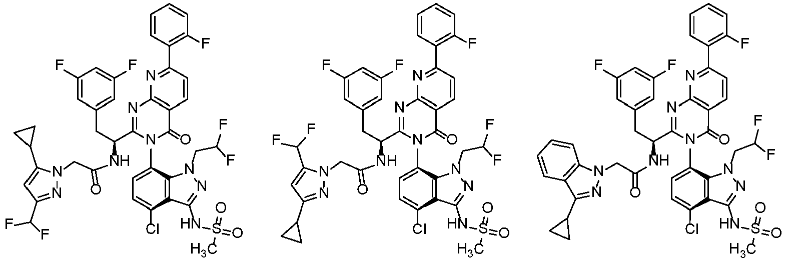 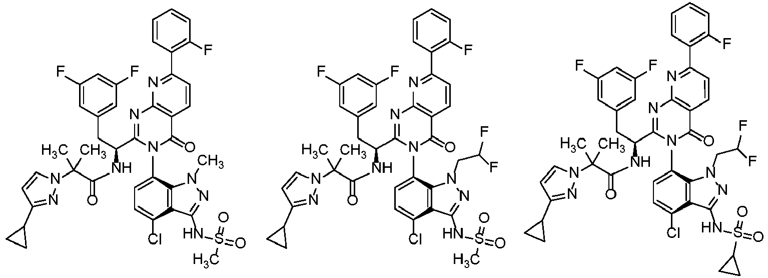 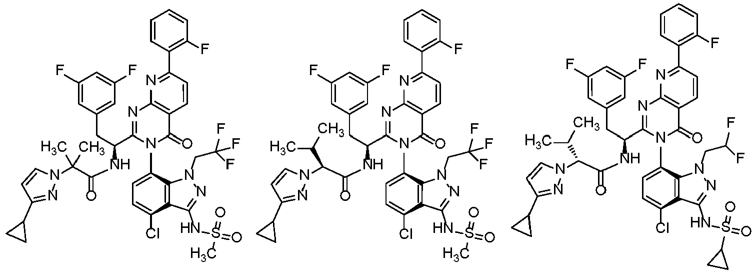 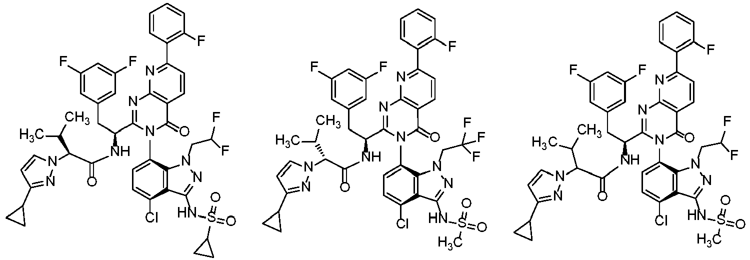 and pharmaceutically acceptable salts thereof.

In another aspect, the present invention discloses a compound or salt selected from the group consisting of: 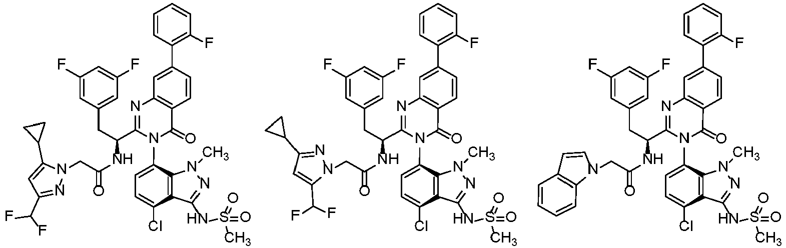 and pharmaceutically acceptable salts thereof.

In another aspect, the present invention discloses a compound or salt selected from the group consisting of: 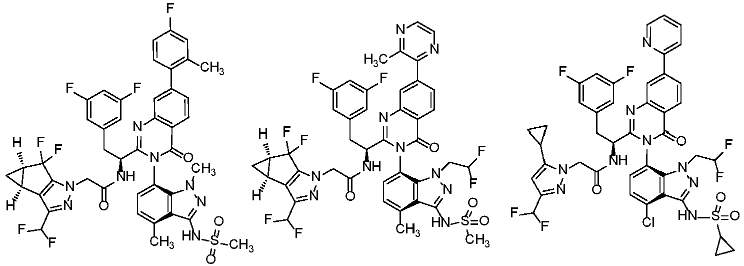 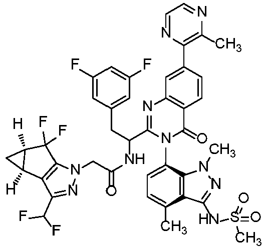 and pharmaceutically acceptable salts thereof.

In another aspect, the present invention discloses a composition comprising a compound or salt of the invention.

In another aspect, the present invention discloses a method of treating HIV infection in a human comprising administering a compound or salt of the invention.

In another aspect, the present invention discloses a compound or salt of the invention for use in therapy.

In another aspect, the present invention discloses a compound or salt of the invention for use in treating HIV infection in a human.

In another aspect, the present invention discloses the use of a compound or salt of the invention in the manufacture of a medicament for the treatment of HIV infection in a human.

### DETAILED DESCRIPTION OF THE INVENTION

The salts of the invention are pharmaceutically acceptable. Such salts may be acid addition salts or base addition salts. For a review of suitable pharmaceutically acceptable salts see, for example, Berge et al, J. Pharm, Sci., 66, 1-19, 1977.

Representative pharmaceutically acceptable acid addition salts include, but are not limited to, 4-acetamidobenzoate, acetate, adipate, alginate, ascorbate, aspartate, benzenesulfonate (besylate), benzoate, bisulfate, bitartrate, butyrate, calcium edetate, camphorate, camphorsulfonate (camsylate), caprate (decanoate), caproate (hexanoate), caprylate (octanoate), cinnamate, citrate, cyclamate, digluconate, 2,5-dihydroxybenzoate, disuccinate, dodecylsulfate (estolate), edetate (ethylenediaminetetraacetate), estolate (lauryl sulfate), ethane-1,2-disulfonate (edisylate), ethanesulfonate (esylate), formate, fumarate, galactarate (mucate), gentisate (2,5-dihydroxybenzoate), glucoheptonate (gluceptate), gluconate, glucuronate, glutamate, glutarate, glycerophosphorate, glycolate, hexyl resorcinate, hippurate, hydrabamine (*N,N'*-di(dehydroabietyl)-ethylenediamine), hydrobromide, hydrochloride, hydroiodide, hydroxynaphthoate, isobutyrate, lactate, lactobionate, laurate, malate, maleate, malonate, mandelate, methanesulfonate (mesylate), methylsulfate, mucate, naphthalene-1,5-disulfonate (napadisylate), naphthalene-2-sulfonate (napsylate), nicotinate, nitrate, oleate, palmitate, p-aminobenzenesulfonate, p-aminosalicyclate, pamoate (embonate), pantothenate, pectinate, persulfate, phenylacetate, phenylethylbarbiturate, phosphate, polygalacturonate, propionate, p-toluenesulfonate (tosylate), pyroglutamate, pyruvate, salicylate, sebacate, stearate, subacetate, succinate, sulfamate, sulfate, tannate, tartrate, teoclate (8-chlorotheophyllinate), thiocyanate, triethiodide, undecanoate, undecylenate, and valerate.

Representative pharmaceutically acceptable base addition salts include, but are not limited to, aluminium, 2-amino-2-(hydroxymethyl)-1,3-propanediol (TRIS, tromethamine), arginine, benethamine (*N*-benzylphenethylamine), benzathine (*N,N'*-dibenzylethylenediamine), *bis*-(2-hydroxyethyl)amine, bismuth, calcium, chloroprocaine, choline, clemizole (1-*p* chlorobenryl-2-pyrrolildine-1'-ylmethylbenzimidazole), cyclohexylamine, dibenzylethylenediamine, diethylamine, diethyltriamine, dimethylamine, dimethylethanolamine, dopamine, ethanolamine, ethylenediamine, L-histidine, iron, isoquinoline, lepidine, lithium, lysine, magnesium, meglumine (*N*-methylglucamine), piperazine, piperidine, potassium, procaine, quinine, quinoline, sodium, strontium, *t*-butylamine, and zinc.

In one embodiment, the compositions of this invention further comprise a pharmaceutically acceptable excipient. In the method of this invention, preferred routes of administration are oral and by injection to deliver subcutaneously or intramuscularly. Therefore, preferred pharmaceutical compositions include compositions suitable for oral administration (for example tablets) and compositions suitable for subcutaneous or intramuscular injection.

In another aspect the present invention discloses methods of preventing HIV infection in a human or reducing the risk of infection, comprising administering a compound or salt of this invention. Pre-exposure prophylaxis (or PrEP) is when people at risk for HIV infection take daily medicine to lower their chances of getting HIV infection. PrEP has been shown to be effective in reducing the risk of infection. As used herein, "HIV" or "Human Immunodeficiency Virus" refers to HIV-1 and/or to HIV-2.

The compounds and salts of this invention are believed to have as their biological target the HIV capsid and thus their mechanism of action is to modify in one or more ways the function of the HIV capsid.

The compounds and salts of the present invention may be employed alone or in combination with other therapeutic agents. Combination therapies according to the present invention thus comprise the administration of at least one compound or salt of the invention, and the administration of at least one other agent which may be useful in the treatment of HIV infection. A compound or salt of the present invention, and the other agent may be formulated and administered together in a single pharmaceutical composition or may be formulated and administered separately. When formulated and administered separately, administration may occur simultaneously or sequentially in any order. Suitable other agents include, for example, abacavir, atazanavir, bictegravir, cabotegravir, darunavir, delavirdine, didanosine, dideoxyinosine, dolutegravir, doravirine, efavirenz, elvitegravir, emtricitabine, etavirine, fosamprenavir, fostemsavir, GSK3640254, the antibody N6LS, GSK3739937/VH3739937 and GSK4000422/VH4000422, indinavir, lamivudine, lopinavir, maraviroc, nelfinavir, nevirapine, raltegravir, rilpiverine, ritonavir, saquinavir, slatravir, stavudine, tipranavir, tenofovir, tenofovir alafenamide, tenofovir disoproxil fumarate, zalcitabine, zidovudine, and S-648414. Preferred agents include, for example, bictegravir, cabotegravir, dolutegravir, fostemsavir, islatravir, and lamivudine. Particularly preferred agents include, for example, bictegravir, cabotegravir, dolutegravir, fostemsavir, and lamivudine.

### EXAMPLES

### LCMS Method A:

Column = Acquity UPLC BEH C18, 2.1 × 30 mm, 1.7 µm particles; Solvent A = 0.1% Formic acid in 100% Water; Solvent B = 0.1% Formic Acid in 100% Acetonitrile; Flow Rate = 0.8 mL/min.; Start % B = 5, Final % B = 95; Gradient Time = 1.6 min, then a 0.25 min hold at 95% B. Detection = 215 nm.

### LCMS Method B:

Column = Acquity BEH C18, 2.1 × 30 mm, 1.7 µm particles; Solvent A = 0.1% Formic acid in 100% Water; Solvent B = 0.1% Formic Acid in 100% Acetonitrile; Flow Rate = 0.8 mL/min.; Start % B = 5, Final % B = 95; Gradient Time = 1.7 min, then a 0.2 min hold at 95% B. Detection = 215 and 254 nm.

### LCMS Method E:

Column = Zorbax Eclipse Plus C18, 2.1 × 50 mm, 1.7 µm particles; Solvent A = 0.1% Formic acid in 100% Water; Solvent B = 0.1% Formic Acid in 100% Acetonitrile; Flow Rate = 1 mL/min; Start % B = 5, Final % B = 95; Gradient Time = 2.1 min, then a 0.3 min hold at 95% B. Detection = 215 and 254 nm.

### LCMS Method F:

Column = Waters XTerra C18, 4.6 × 50 mm, 5 µm particles; Solvent A = 0.1% NH₄OH in 100% Water; Solvent B = Acetonitrile; Flow Rate = 2.5 mL/min.; Start % B = 5, Final % B = 95; Gradient Time = 4 min, then a 1 min hold at 95% B. Detection = 215 nm and 254 nm.

### LCMS Method H:

Column = Acquity CSH C18, 2.1 × 30 mm, 1.7 µm particles; Solvent A = 0.1% Formic acid in 100% Water; Solvent B = 0.1% Formic Acid in 100% Acetonitrile; Flow Rate = 0.8 mL/min.; Start % B = 5, Final % B = 95; Gradient Time = 1.7 min, then a 0.2 min hold at 95% B. Detection = 215 and 254 nm.

### LCMS Method I:

Column = Acquity BEH C18, 2.1 × 100 mm, 1.7 µm particles; Solvent A = 0.05% TFA in water; Solvent B = Acetonitrile; Flow rate = 0.45 mL/min.; Start % B = 3, Final % B = 98; Gradient Time = 7.5 min., then a 2 min. hold at 98% B; Column Temp = 35°C.

### LCMS Method 1:

Column = Acquity BEH C18, 2.1 × 50 mm, 1.7 µm particles; Solvent A = 0.1% Formic Acid in water; Solvent B = 0.1% Formic Acid in Acetonitrile; Flow rate = 0.6 mL/min.; Start % B = 3, Final % B = 98; Gradient Time = 3.2 min., then a 0.6 min hold at 98% B; Column Temp = 35°C.

### LCMS Method K:

Column = Xbridge C18, 4.6 × 50 mm, 2.5 µm particles; Solvent A = 5 mM Ammonium Bicarbonate in water; Solvent B = Acetonitrile; Flow rate = 1.3 mL/min.; Time (min) / % Solvent B = 0/5, 0.5/5, 1.5/15, 7/98, 9/98, 9.5/5, 10/5; Column temp =35°C.

### General Procedure A:

To a stirred solution of (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-methyl-1H-indazol-3-yl)methanesulfonamide (or a different amine as indicated) (0.043-0.061 mmol, 1 equiv.) in N,N-dimethylformamide (1 mL) were added the carboxylic acid (1 equiv), 2-(3H-[1,2,3]triazolo[4,5-b]pyridin-3-yl)-1,1,3,3-tetramethylisouronium hexafluorophosphate(V) ("HATU", 1.2 equiv.) and DIPEA (2.5 equiv.). The mixture was stirred for 18 h and then concentrated under reduced pressure. The resulting residue was dissolved in DMF, the solution was filtered, and the filtrate was subjected to prep-HPLC to afford the indicated product.

### General Procedure B:

To a vial equipped with a stir bar was added N-((S)-1-((3P)-3-(4-chloro-1-methyl-3-(methylsulfonamido)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1 equiv, typically 25-50 mg), tribasic potassium phosphate (3 equiv), dichloro[9,9-dimethyl-4,5-bis(diphenylphosphino)xanthene]palladium(II) (0.05 equiv), and the appropriate aryl/heteroaryl halide (2-3 equiv). The vial was sealed with a septum cap and then was purged with argon. To the vial was added THF:water (4:1) to afford a reaction volume 0.05M in boronic ester. The reaction mixture was stirred at room 20-60 °C for 16-24 h. Upon cooling to ambient temperature, the reaction mixture was concentrated *in vacuo* and the resulting residue was subjected to HPLC purification to afforded the indicated product. Alternately, the reaction may be run under ambient atmosphere. Alternately, the reagents may be combined using stock solutions of THF and water to achieve the final concentrations indicated. Alternately, (S)-2-(3-cyclopropyl-1H-pyrazol-1-yl)-N-(2-(3,5-difluorophenyl)-1-(3-(4-(morpholinosulfonyl)phenyl)-4-oxo-7-(4,4,5,5-tetramethyl-1,3,2-dioxaborolan-2-yl)-3,4-dihydroquinazolin-2-yl)ethyl)acetamide or 2-((3bR,4aS)-3-(difluoromethyl)-5,5-difluoro-3b,4,4a,5-tetrahydro-1H-cyclopropa[3,4]cyclopenta[1,2-c]pyrazol-1-yl)-N-((S)-2-(3,5-difluorophenyl)-1-(3-(4-(morpholinosulfonyl)phenyl)-4-oxo-7-(4,4,5,5-tetramethyl-1,3,2-dioxaborolan-2-yl)-3,4-dihydroquinazolin-2-yl)ethyl)acetamide may be substituted for N-((S)-1-(3-(4-chloro-1-methyl-3-(methylsulfonamido)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

### General Procedure C:

To a solution of (S)-N-((6P)-7-(2-(1-amino-2-(3,5-difluorophenyl)ethyl)-7-(3-methylpyrazin-2-yl)-4-oxoquinazolin-3(4H)-yl)-4-chloro-1-methyl-1H-indazol-3-yl)methanesulfonamide (or a different amine if indicated) (0.025-0.053 mmol, 1 equiv.), the indicated carboxylic acid (1.1 equiv.), and HATU (1.2 equiv.) in tetrahydrofuran (THF) (0.5 mL) was added DIEA (3 equiv.) at RT. The resulting mixture was stirred at RT for 18 hrs. The reaction mixture was concentrated in vacuo. The residue was treated with ammonia in MeOH (2M, 1 mL) and the mixture was then stirred for 10 minutes. The reaction volatiles were removed under a stream of nitrogen gas. The resulting residue dissolved in DMF and then subjected to HPLC purification to afford the indicated product.

### General Procedure D:

To a vial equipped with a stir bar was added Pd(OAc)₂ (0.1 equiv), dicyclohexyl(2',6'-dimethoxy-[1,1'-biphenyl]-2-yl)phosphane (0.2 equiv), K₃PO₄ (3 equiv), and N-((S)-1-((3P)-3-(4-chloro-1-(2,2-difluoroethyl)-3-(methylsulfonamido)-1H-indazol-7-yl)-4-oxo-7-(4,4,5,5-tetra methyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1 equiv, typically 50-100 mg). To the vial was added the appropriate aryl halide or heteroaryl halide (3 equiv). The vial was capped with a septum cap and then placed under argon atmosphere (vac/fill × 3). To the vial was added THF:water (4:1) to afford a reaction volume 0.05M in boronic ester. The reaction mixture was degassed via vac/fill (x3) with argon. The reaction mixture was stirred at either ambient temperature, 45 °C, or 60 °C for overnight (~18 h). Upon cooling to ambient temperature, the reaction mixture was concentrated and the residue was subjected to HPLC purification to afford the indicated product.

### General Procedure I:

To a stirred solution of (S)-N-((6P)-7-(2-(1-amino-2-(3,5-difluorophenyl)ethyl)-4-oxo-7-(6-(trifluoromethyl)pyridin-2-yl)quinazolin-3(4H)-yl)-4-chloro-1-methyl-1H-indazol-3-yl)methanesulfonamide (or a different amine if indicated) (1 equiv, typically 0.023 - 0.036 mmol) in THF (volume necessary to achieve 0.1 M concentration in amine) was added the appropriate carboxylic acid (1.0 equiv), DIEA (3 equiv) and then HATU (1.0-1.5 equiv). The mixture was stirred at room temperature until judged complete by LCMS analysis (generally 18 h or less). The reaction mixture was concentrated under reduced pressure and the resulting residue was subjected to HPLC purification to afford the indicated product.

### General Procedure T:

To a solution of N-((S)-1-((3P)-7-bromo-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (typically 50 mg, 0.053 mmol, 1.0 equiv), in THF (80 volumes) and water (10 volumes) was added the appropriate boronic acid (1.5 equiv) and potassium phosphate tribasic (5 equiv) at 26 °C. The reaction mixture was degassed for 10 min with nitrogen bubbling. To the mixture was added dichloro[9,9-dimethyl-4,5-bis(diphenylphosphino)xanthene]palladium(II) (0.06 equiv). The mixture was stirred at room temperature for 8-16 h. The reaction mixture was filtered through a pad of Celite, extracting with ethyl acetate (30 mL). The organic layer was dried over anhydrous Na₂SO₄, filtered and concentrated under reduced pressure to afford the crude product. The crude material was subjected to HPLC purification to afford the indicated product.

### General HPLC conditions:

HPLC purification was performed using one of the conditions indicated below, optionally followed by a second HPLC purification using a different condition indicated below. Based on analytical HPLC data obtained on the crude reaction mixture, the purification condition was optimized for each target compound by modifying the initial Solvent A: Solvent B ratio, the gradient time, the final Solvent A:Solvent B ratio, and the hold time at the final Solvent A: Solvent B concentration.

HPLC Condition A: Column: Zorbax Eclipse Plus C18, 21.2 × 100 mm, 5 µm particles; Solvent A = 0.1% Formic Acid in 100% Water. Solvent B = Acetonitrile. Flow Rate = 40 mL/min. Wavelength = 215 and 254 nm. ESI+ Range: 150 to 1500 dalton.

HPLC Condition B: Column: Sunfire Prep C18 OBD, 30 × 100 mm, 5 µm particles; Solvent A: water:MeCN 95:5 w/ 0.1% TFA, Solvent B: MeCN:water 95:5 w/ 0.1% TFA. Flow Rate = 42 mL/min. Wavelength = 220 and 254 nm.

HPLC Condition C: Column: Waters Xterra C18, 19 × 100 mm, 10 µm particles; Solvent A = 0.1% NH4OH in 100% Water. Solvent B = Acetonitrile. Flow Rate = 40 mL/min. Wavelength = 215 and 254 nm. ESI + Range: 150 to 1500 dalton.

HPLC Condition D: Column: Waters XSelect CSH C18 , 19 × 100 mm, 5 µm particles; Solvent A = 0.1% Formic Acid in 100% Water. Solvent B = Acetonitrile. Flow Rate = 40 mL/min. Wavelength = 215 and 254 nm. ESI + Range: 150 to 1500 dalton.

### Preparation of bicyclo[3.1.0]hexan-3-ol

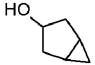

To a stirred solution of cyclopent-3-enol (130 g, 1545 mmol) in DCM (1200 mL) under N₂ atmosphere at 0-5 °C was added dropwise a solution of diethyl zinc in hexane (1.0 M, 3091 mL, 3091 mmol) over a period of 3 h. To the solution at 0 °C was added dropwise a solution of diiodomethane (249 mL, 3091 mmol) in DCM (300 mL) over a period of 1h. The reaction mixture was allowed to warm to 27 °C upon which formation of a white precipitation was observed. The mixture stirred for 16 h. Progress of the reaction was monitored by TLC (SiO₂, 20% EtOAc/pet, Rf = 0.3, UV-inactive, PMA-active). The reaction mixture was quenched via the careful addition of aq. saturated NH₄Cl solution (1.5 L). The mixture was filtered through pad of Celite. The aqueous layer was extracted with DCM (2 × 1L). The combined organic layers were dried over anhydrous Na₂SO₄, filtered and then concentrated under reduced pressure to afford crude bicyclo[3.1.0]hexan-3-ol as red liquid, 180 g. ¹H NMR (400 MHz, CDCl₃) δ = 4.41 - 4.35 (m, 1H), 2.18 - 2.05 (m, 2H), 1.73 (d, *J* = 13.9 Hz, 2H), 1.35 - 1.25 (m, 2H), 1.21 - 1.14 (m, 1H), 0.57 - 0.43 (m, 2H). GCMS: m/z = 98.1).

### Preparation of bicyclo[3.1.0]hexan-3-one

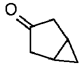

To a stirred solution of bicyclo[3.1.0]hexan-3-ol (210 g, 2054 mmol) in DCM (5000 mL) under N₂ atmosphere at 0 °C was added portion-wise Dess-Martin periodinane (954 g, 225 mmol). The mixture was allowed to warm to 27 °C and was then stirred for 16 h. Progress of the reaction was monitored by TLC (SiO₂, 20% Acetone/Hex, Rf = 0.3, UV inactive, PMA-active). The reaction mixture was filtered through pad of Celite and the filtrate was washed with aq. NaOH (1N, 8× 1 L). The combined aqueous phases were extracted with DCM (5 × 1 L). The combined organic layers were dried over anhydrous Na₂SO₄, filtered, and then concentrated under reduced pressure (bath temperature: 20 °C) to afford crude bicyclo[3.1.0]hexan-3-one as brown liquid. The liquid was further purified by downward distillation at 70 °C to afford bicyclo[3.1.0]hexan-3-one as a pale-yellow viscous liquid, 125 g (62%). ¹H NMR (400 MHz, CDCl₃) δ = 2.61 - 2.54 (m, 2H), 2.17 - 2.12 (m, 2H), 1.54 - 1.46 (m, 2H), 0.92 - 0.86 (m, 1H), -0.01 - -0.08 (m, 1H); GCMS: M/Z = 96.1.

### Preparation of 2-(2,2-difluoroacetyl)bicyclo[3.1.0]hexan-3-one

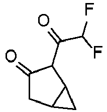

To a stirred solution of bicyclo[3.1.0]hexan-3-one (125 g, 1274 mmol) in THF (1500 mL) under N₂ atmosphere at -78 °C was added LDA (2.0 M in THF, 0.701 L, 1402 mmol). The solution was stirred for 1 h at -78 °C. To the solution was added slowly over 30 minutes a solution of ethyldifluoroacetate (174 g, 1402 mmol) in THF (300 mL) maintaining a temperature of -78 °C. The reaction mixture was allowed to warm to 27 °C and was then stirred for 1 h. Progress of the reaction was monitored by TLC (SiO₂, 20% Acetone/Hexane, Rf = 0.3, UV -active). The reaction mixture was quenched via the addition of aq. HCl (1N, 2000 mL). The mixture was stirred for 30 min. and then was extracted with EtOAc (3 × 1000 mL). The combined organic layers were washed with brine (1000 mL), dried over anhydrous Na₂SO₄ and filtered. The filtrate was concentrated under reduced pressure to afford 2-(2,2-difluoroacetyl)bicyclo[3.1.0]hexan-3-one as a pale-yellow viscous liquid, 180 g (71%). ¹H NMR (400 MHz, CDCl₃)δ = 6.18 (t, *J* = 54.8 Hz, 1H), 2.70 - 2.62 (m, 1H), 2.35 (d, *J* = 19.4 Hz, 1H), 2.14 (br s, 1H), 1.26 - 1.21 (m, 1H), 1.04-1.03 (m, 1H), 0.22-0.21 (m, 1H), LCMS: M/Z = 173.17).

### Preparation of ethyl 2-(3-(difiuoromethyi)-3b,4,4a,5-tetrahydro-1H-cyclopropa[3,4]cyclopenta[1,2-c]pyrazol-1-yl)acetate.

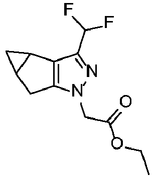

To a stirred solution of 2-(2,2-difluoroacetyl)bicyclo[3.1.0]hexan-3-one (180 g, 910 mmol) in ethanol (2 L) under N₂ atmosphere at 27 °C was added ethyl 2-hydrazinylacetate hydrochloride (422 g, 2729 mmol) followed by sulfuric acid (20 mL, 375 mmol). The mixture was stirred for 30 min. and then was heated to 100 °C and stirred for 16 h. Progress of the reaction was monitored by TLC (SiO₂, 20% Acetone/Hexane, Rf = 0.3, UV-active). The reaction mixture was concentrated under reduced pressure. The residue was dissolved in EtOAc (2000 mL) and was washed with water (2 × 1 L), brine (1.0 L), dried over anhydrous Na₂SO₄, filtered, and then was concentrated under reduced pressure. The resulting residue was subjected to silica gel column chromatography (pet.:acetone 100:0→98:2) to afford ethyl 2-(3-(difluoromethyl)-3b,4,4a,5-tetrahydro-1H-cyclopropa[3,4]cyclopenta[1,2-c]pyrazol-1-yl)acetate as an off-white solid, 110 g (46%). ¹H NMR (400 MHz, DMSO-d₆) δ = 6.86 (t, *J* = 54.8 Hz, 1H), 4.93 (s, 2H), 4.14 (q, *J* = 7.2 Hz, 2H), 2.88 - 2.79 (m, 1H), 2.76 - 2.68 (m, 1H), 2.14 - 2.04 (m, 2H), 1.19 (t, *J* = 7.2 Hz, 3H), 1.10 - 1.03 (m, 1H), 0.14 (q, *J* = 4.3 Hz, 1H).

### Preparation of ethyl 2-(3-(difluoromethyl)-5-oxo-3b,4,4a,5-tetrahydro-1H-cyclopropa[3,4]cyclopenta[1,2-c]pyrazol-1-y/)acetate.

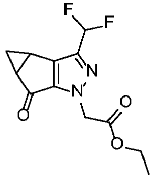

To a stirred solution of ethyl 2-(3-(difluoromethyl)-3b,4,4a,5-tetrahydro-1H-cyclopropa[3,4]cyclopenta[1,2-c]pyrazol-1-yl)acetate (110 g, 422 mmol) and Celite (395 g) in cyclohexane (3.5 L) at 0 °C was added portionwise pyridinium dichromate (794 g, 2110 mmol). To the mixture under nitrogen atmosphere was added dropwise tert-butyl hydroperoxide (355 mL, 2130 mmol) over a period of 10 min. The reaction mixture was warmed to 27 °C and was then stirred at that temperature for 48 h. Progress of the reaction was monitored by TLC (SiO₂, 30% Acetone/pet, Rf = 0.4, UV -active). The reaction mixture was filtered and the filter cake was extracted with EtOAc (1000 mL). The filtrate was washed with saturated aq. NazSzOs (2×500 mL); saturated aq. FeSO₄ (300 mL); and then brine (500 mL). The organic layer was dried over anhydrous Na₂SO₄, filtered and concentrated under reduced pressure to obtain the crude title compound (150 g).

### Preparation of ethyl 2-(3-(difluoromethyl)-4,4a-dihydrospiro[cyclopropa[3,4]cyclopenta[1,2-c]pyrazole-5,2'-[1,3]dithiolane]-1(3bH)-y/)acetate.

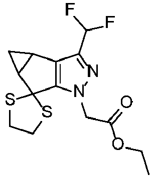

To a stirred solution of ethyl 2-(3-(difluoromethyl)-5-oxo-3b,4,4a,5-tetrahydro-1H-cyclopropa[3,4]cyclopenta[1,2-c]pyrazol-1-yl)acetate (75 g, 269 mmol) in DCM (1500 mL) at 27 °C under nitrogen atmosphere was added ethane-1,2-dithiol (43.0 mL, 511 mmol) followed by the addition of boron trifluoride acetic acid (72.6 mL, 511 mmol). The solution was stirred for 16 h. Progress of the reaction was monitored by TLC (SiO₂, 20% Acetone/Pet, Rf = 0.35, UV -Active). After completion, the reaction mixture was cooled to 0 °C and quenched via the addition of aq. saturated NaHCO₃ (500 mL). The mixture was extracted with DCM (2 × 1000 mL). The combined organics were washed with brine (1000 mL), dried over anhydrous Na₂SO₄, filtered and concentrated under reduced pressure to obtain a brown liquid. This material was subjected to silica gel column chromatography (Pet.:EtOAc 95:5→90:10) to afford ethyl 2-(3-(difluoromethyl)-4,4a-dihydrospiro[cyclopropa[3,4]cyclopenta[1,2-c]pyrazole-5,2'-[1,3]dithiolane]-1(3bH)-yl)acetate as an off-white solid, 80 g (74%). ¹H-NMR (400 MHz, CDCl₃)δ = 6.61 (t, *J* = 55.2 Hz, 1H), 5.00 - 4.85 (m, 2H), 4.29 - 4.19 (m, 2H), 3.55 - 3.46 (m, 4H), 2.63 - 2.53 (m, 1H), 2.49 - 2.38 (m, 1H), 1.30 - 1.24 (m, 4H), 0.65 - 0.60 (m, 1H). LCMS M+H = 346.9.

### Preparation of ethyl 2-(3-(difluoromethyl)-5,5-difluoro-3b,4,4a,5-tetrahydro-1H-cyclopropa[3,4]cyclopenta[1,2-c]pyrazol-1-yl)acetate

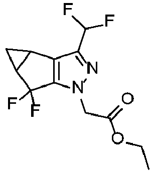

To a stirred solution of 1,3-dibromo-5,5-dimethylimidazolidine-2,4-dione (26.3 g, 92 mmol) in DCM (20 mL) at -70 °C under N₂ atmosphere was added HF-pyridine (2.460 g, 24.83 mmol). The solution was for 30 min. To the solution was added a solution of ethyl 2-(3-(difluoromethyl)-4,4a-dihydrospiro[cyclopropa[3,4]cyclopenta[1,2-c]pyrazole-5,2'-1,3]dithiolane]-1(3bH)-yl)acetate (10 g, 25 mmol) in DCM (20 mL). The reaction mixture was allowed to warm to -40 °C and then was stirred at that temperature for 1 h. Progress of the reaction was monitored by TLC (SiO2, 30% EtOAc/Pet, Rf = 0.3, UV in-active). The reaction mixture was quenched via the addition of aq. sat. NaHCOs (200 mL). The mixture was warmed to room temperature and was then extracted with EtOAc (2 × 100 mL). The combined organics were washed with brine (50 mL); dried over anhydrous Na₂SO₄; filtered; and were concentrated under reduced pressure to afford a brown solid. This material was subjected to silica gel column chromatography (Pet.:EtOAc 100:0→75-25) to afford ethyl 2-(3-(difluoromethyl)-5,5-difluoro-3b,4,4a,5-tetrahydro-1H-cyclopropa[3,4]cyclopenta[1,2-c]pyrazol-1-yl)acetate as a pale-yellow solid, 8.5 g (91%). ¹H NMR (400 MHz, CDCl₃)δ = 6.62 (t, J = 55.2 Hz, 1H), 4.82 (s, 2H), 4.30 - 4.18 (m, 2H), 2.51 - 2.37 (m, 2H), 1.42 - 1.35 (m, 1H), 1.31 - 1.23 (m, 3H), 1.14 - 1.08 (m, 1H). LCMS M+H = 293.07.

### Preparation of 2-(3-(difluoromethyl)-5,5-difluoro-3b,4,4a,5-tetrahydro-1H-cyciopropa[3,4]cyciopenta[1,2-c]pyrazol-1-yl)acetic acid

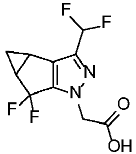

To a stirred solution of ethyl 2-(3-(difluoromethyl)-5,5-difluoro-3b,4,4a,5-tetrahydro-1H-cyclopropa[3,4]cyclopenta[1,2-c]pyrazol-1-yl)acetate (15 g, 50 mmol) in THF (17 mL) and MeOH (66 mL) at 0 °C under N₂ atmosphere was added a solution of LiOH (1.788 g, 74.7 mmol) in water (66 mL). The reaction mixture was allowed to warm to 27 °C and was then stirred for 3 h at that temperature. Progress of the reaction was monitored by TLC (SiO₂, 5% MeOH/DCM, Rf = 0.2, UV Active). After completion, the reaction mixture was concentrated under reduced pressure; diluted with water (50 mL); and washed with EtOAc (2 × 250 mL) to remove impurities. The aqueous layer was adjusted to pH 2-3 using aq. HCl (1M), then was extracted with EtOAc (3 × 1000 mL). The combined organics were dried over anhydrous Na₂SO₄; filtered; and concentrated under reduced pressure to afford 2-(3-(difluoromethyl)-5,5-difluoro-3b,4,4a,5-tetrahydro-1H-cyclopropa[3,4]cyclopenta[1,2-c]pyrazol-1-yl)acetic acid as an off white solid, 14 g (98%). LCMS M+H = 265.15.

### Separation affording 2-((3bS,4aR)-3-(difluoromethyl)-5,5difluoro-3b,4,4a,5-tetrahydro-1H-cyclopropa[3,4]cyclopenta[1,2-c]pyrazol-1-yl)acetic acid and 2-((3bR,4aS)-3-(difluoromethyl)-5,5-dif/uoro-3b,4,4a,5-tetrahydro-1H-cyclopropa[3,4]cyclopenta[1,2-c]pyrazol-1-y/)acetic acid

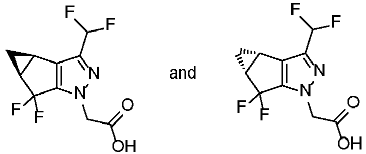

2-(3-(difluoromethyl)-5,5-difluoro-3b,4,4a,5-tetrahydro-1H-cyclopropa[3,4]cyclopenta[1,2-c]pyrazol-1-yl)acetic acid (5.5 g) was dissolved in isopropanol (20 mL). The solution was subjected portion-wise to SFC chiral separation as follows: Instrument = Thar 80; column = Chiralpak IC 30×250mm, 5 micron; solvent A = super critical COz; solvent B = isopropanol with 0.5% isopropylamine (v/v); eluent composition = 70%A:30%B; flow-rate = 65 g/min; back-pressure = 100 bar; temperature = 30 °C; injection volume = 2.5 mL; detection = 220 nm. 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid was collected as peak eluting from 7.5 min. to 14 min; 2-((3bR,4aS)-3-(difluoromethyl)-5,5-difluoro-3b,4,4a,5-tetrahydro-1H-cyclopropa[3,4]cyclopenta[1,2-c]pyrazol-1-yl)acetic acid was collected as a peak eluting from 2.7 min. to 5.8 min. For each enantiomer, the resulting solution was concentrated under reduced pressure and the resulting solids were dissolved in EtOAc, then twice washed with aq. citric acid (1M) followed by water followed by brine. The organic solution was dried over Na₂SO₄; filtered; then concentrated in vacuo to afford the separated enantiomer in 80-90% recovery.

### Preparation of N-(7-amino-4-chloro-1-methyl-1H-indazol-3-yl)-N-(4-methoxybenzyl)methanesulfonamide.

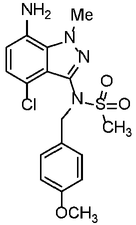

### Synthesis Scheme:

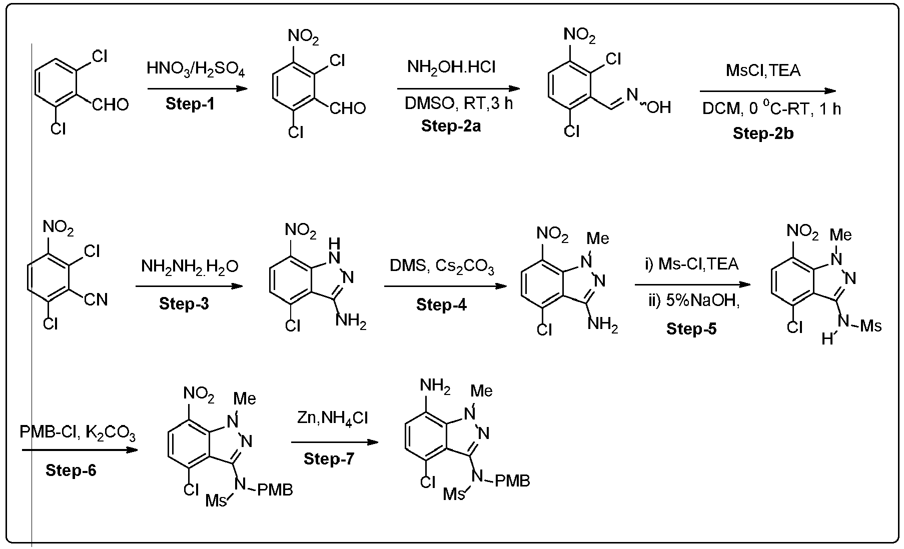

### Step 1: Preparation of 2,6-dichloro-3-nitrobenzaldehyde

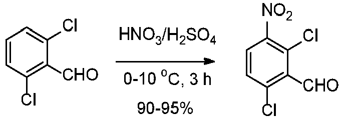

To a solution of sulfuric acid (H₂SO₄) (5.63 L, 4.5 V) in a round-bottom flask at 0-5 °C was added 2,6-dichlorobenzaldehyde (1.25 kg, 7.10 mol, 1.0 equiv.) in portions at below 15 °C. The reaction mass was stirred at 0-5 °C for 30 min. A solution of freshly prepared nitration mixture [Prepared from Conc. H₂SO₄ (0.425 L, 0.34 V) and 70% HNO₃ (0.85 kg, 13.49 mol, 1.30 equiv.) at 0 °C] was added to the above reaction mixture at below 10 °C **[Note:** Reaction is slightly exothermic (3-6 °C); so that addition is preferred at lower temperature]. The reaction mixture was stirred at 5-10 °C for 2-3 h. After completion of the reaction (monitored by TLC), it was quenched with ice cold water (18.75 L, 15 V) at below 25 °C. Then the reaction mass was allowed warm to room temperature and stirred for 2 h. The solids were isolated by filtration and then were washed with water (2.5 L, 2.0 V). Bulk residual water was removed from the solids by maintaining vacuum filtration for 60-90 min. The crude wet solid was initially dried under air atmosphere; then in a hot air oven at 50-55 °C for 10-12 h (until moisture content is not more than 5.0 %) to get the dried title product, 2,6-dichloro-3-nitrobenzaldehyde (1.44 kg, 92% yield) as a yellow solid. ¹H NMR (400 MHz, CDCl₃): *δ* 10. 44 (s, 1H), 7.88 (d, *J* = 8.4 Hz, 1H), 7.56 (d, *J* = 8.8 Hz, 1H).

### Step 2: Preparation of 2,6-dichloro-3-nitrobenzonitrile

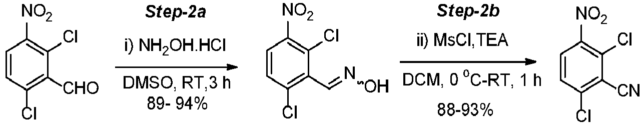

(Step-2a) To a solution of DMSO (5.9 L, 5.0 V)) in a round-bottom flask was added 2,6-dichloro-3-nitrobenzaldehyde (1.17 kg, 5.31 mol, 1.0 equiv.) at room temperature. After being stirred for 30 min at room temperature, hydroxylamine hydrochloride (0.63 kg, 9.04 mol, 1.70 equiv.) was added and the reaction mass was stirred at room temperature for 3 h. After completion of the reaction (monitored by TLC), the reaction mass was quenched by the addition of ice-cold water (18.0 L, 15.0 V) added at a rate sufficient to maintain the temperature below 30 °C (Observation: Solids formed upon water addition). The reaction mass was stirred at room temperature for 60-90 min. The solids were isolated by filtration; washed with water (2.5 L, 2.0 V); followed by washing with a mixture of acetone and hexanes (6.0 L, 1:1 ratio). Bulk residual water was removed from the solids by maintaining vacuum filtration for 60-90 min. The wet solid was initially air dried and then finally dried in a hot air oven at 50-55 °C for 10-12 h (until moisture content was not more than 1.0 %) to get the dried target product, 2,6-dichloro-3-nitrobenzaldehyde oxime (1.22 kg, 92% yield) as an off-white solid. The crude product (which contains 10-20% of 2,6-dichloro-3-nitrobenzonitrile) was used directly in the next step without further purification.
(Step-2b) To a stirred solution of the crude oxime (preparation described above, 1.13 kg, 4.80 mol, 1.0 equiv.) in DCM (9.04 L, 8.0 V) at 0-5 °C was added triethylamine ("TEA", 1.02 kg, 10.09 mol, 2.1 equiv.). After being stirred for 5 min, methanesulfonyl chloride (0.60 kg, 5.29 mol, 1.1 equiv.) was added (Observation: An exotherm is noted during the addition) slowly at 15 °C. Then the reaction mass was stirred at room temperature for 30-45 min. After completion of the reaction (progress of reaction was monitored by TLC; mobile phase: 20% ethyl acetate in hexanes), the reaction mass was diluted with water (6.78 L, 6.0 V); the organic layer was separated; and the aqueous layer was extracted with DCM (3.4 L, 3.0 V). The combined organic layers were washed with brine (5.65 L, 5.0 V); dried over Na₂SO₄; and concentrated under vacuum. The resulting crude solids were triturated with hexanes (4.50 L, 4.0 V) at room temperature. The wet material was dried in a hot air oven at 50-55 °C for 5- 6 h to get the dried product, 2,6-dichloro-3-nitrobenzonitrile (0.95 kg, 91% yield) as a yellow solid. ¹H NMR (400 MHz, CDCl₃): *δ*8.07 (d, *J* = 8.8 Hz, 1H), 7.63 (d, *J* = 8.8 Hz, 1H).

### Step 3: Preparation of 4-chloro-7-nitro-1H-indazol-3-amine

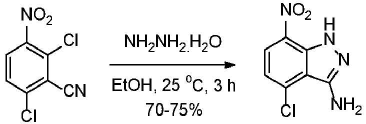

To a stirred solution of 2,6-dichloro-3-nitrobenzonitrile (750.0 g, 3.45 mol, 1.0 equiv.) in ethanol (7.5 L, 10.0 V) at 15-20 °C. was slowly added hydrazine hydrate (519.0 g, 10.36 mol, 3.0 equiv.) while maintaining the reaction mass below 25 °C (Observation: Addition is slightly exothermic and solid formation will begin upon addition). The reaction mixture temperature was slowly raised to room temperature and then the mixture was stirred for 3 h (Observation: the quantity of solids will increase during this time). After completion of the reaction (monitored by TLC), the mixture was diluted with water (7.5 L, 10.0 V) and further stirred for 1 h at room temperature. The solids were isolated via filtration and then were washed with water (2.25 L, 3.0 V). The wet solid was washed with a 1:1 ratio mixture of acetone (1.875 L, 2.5 V) and hexanes (1.875 L, 2.5 V). Bulk residual water was removed from the solids by maintaining vacuum filtration for 60-90 min. The wet solid was finally dried in a hot air oven for 7-8 h at 50 °C (until moisture content reaches below 1.5%) to get the dried product, 4-chloro-7-nitro-1*H*-indazol-3-amine (549.0 g, 75% yield) as a brick red-colored solid. ¹H NMR (400 MHz, CDCl₃): *δ*10.36 (bs, 1H), 8.20 (d, *J* = 8.4 Hz, 1H), 7.07 (d, *J* = 8.40 Hz, 1H), 4.73 (bs, 2H).

### Step 4: Preparation of 4-chloro-1-methyl-7-nitro-1H-indazol-3-amine

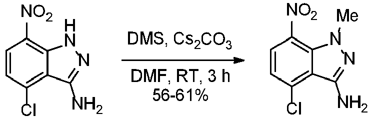

To a stirred solution of 4-chloro-7-nitro-1*H*-indazol-3-amine (500 g, 0.42 mol, 1.0 equiv.) in DMF (5.0 L, 10.0 V) at 5-10 °C was slowly added cesium carbonate (Cs₂CO₃) (1.91 kg, 5.88 mol, 2.5 equiv.) while maintaining the reaction mass below 10 °C. After being stirred for 5-10 min, dimethyl sulphate (326.3 g, 2.59 mol, 1.1 equiv.) was added while maintaining the reaction mass below 10 °C (Note: Slow addition is preferred for obtaining more favorable regio-selectivity). Then, the reaction temperature was slowly raised to room temperature and stirring was continued an additional 2 h at the same temperature. After completion of the reaction (monitored by TLC), the reaction mass was quenched by the addition of ice-cold water (15.0 L, 30.0 V) and the resulting mixture was then stirred for 6-8 h at room temperature. The solids were isolated via filtration and were then washed with water (1.5 L, 3.0 V). The wet solid was washed with IPA (1.5 L, 3.0 V) followed by hexanes (1.0 L, 2.0 V). Bulk residual water was removed from the solids by maintaining vacuum filtration for 60-90 min. The wet solid was dried in a hot air oven for 7-8 h at 50 °C (until moisture content is below 1.0%). The isolated material, 4-chloro-1-methyl-7-nitro-1*H*-indazol-3-amine (319.0 g, 60% yield), was used in the next step without further purification. ¹H NMR (400 MHz, CDCl₃): δ7.97 (d, *J* = 8.32 Hz, 1H), 6.97 (d, *J* = 8.24 Hz, 1H), 4.63 (bs, 2H), 3.96 (s, 3H).

### Step 5: Preparation of N-(4-chloro-1-methyl-7-nitro-1H-indazol-3-yl)methanesulfonamide

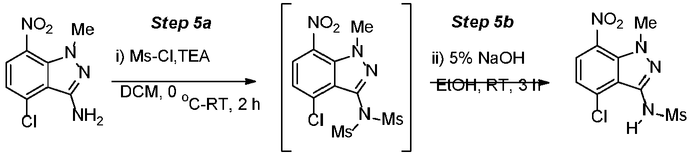

(Step 5a) To a solution of 4-chloro-1-methyl-7-nitro-1*H*-indazol-3-amine (625.0 g, 2.76 mol, 1.0 equiv.) in DCM (6.25 L, 10.0 V) at 0-5 °C. was added triethylamine (TEA) (837.0 g, 8.27 mol, 3.0 equiv.); followed by the addition of 4-dimethylaminopyridine (DMAP) (20.60 g, 0.165 mol, 0.06 equiv.). The reaction mass was stirred for 5-10 min., then methanesulfonyl chloride (MsCl) (790.0 g, 6.89 mol, 2.5 equiv.) added slowly while maintaining the reaction mass below 10 °C. The reaction mixture was allowed to warm to room temperature and was then stirred for 1.5-2.0 h. After completion of the reaction (monitored by TLC), the mixture was diluted with water (6.25 L, 10.0 V) and then stirred at room temperature for 15 min. The organic layer was separated, and the aqueous layer was extracted with DCM (6.25 L, 10.0 V). The combined organic layers were washed with brine (1.25 L, 2.0 V), dried over Na₂SO₄ and concentrated to get the crude solids. The solids were triturated with hexanes (1.25 L, 2.0 V) at room temperature to obtain the intermediate, N-(4-chloro-1-methyl-7-nitro-1H-indazol-3-yl)-N-(methylsulfonyl)methanesulfonamide, which was used directly in the next step. (ii) To a stirred solution of N-(4-chloro-1-methyl-7-nitro-1H-indazol-3-yl)-N-(methylsulfonyl)methanesulfonamide (prepared above) in ethanol (10.5 L, 20.0 V) at room temperature was added slowly an aq. 5% NaOH solution (4.38 L, 7.0 V) [Note: Slow addition is preferred via dropping funnel]. The reaction mass was stirred at the same temperature for 3 h. After completion of the reaction (monitored by TLC) [Sample preparation for TLC analysis: ~1.0 ml of sample acidified with aq. 2.0 N HCl to reach the pH: 2-3, extract it with ethyl acetate and analyze the organic layer by TLC], the reaction mass was cooled to 0-5 °C and the pH was adjusted to 2-3 by the addition of aq. 2.0 N HCl (3.13 L, 5.0 V) while maintain the reaction temperature below 10 °C [Note: Precipitation occurred upon addition of HCl and increased with stirring]. The reaction mixture was warmed to room temperature and then stirred for 1.5-2.0 h. Solids obtained were isolated via filtration and were then washed with water (1.25 L, 2.0 V); followed by washing with hexanes (1.25 L, 2.0 V). Bulk residual water was removed from the solids by maintaining vacuum filtration for 60-90 min. The wet material was dried in a hot air oven at 50 °C for 6-7 h (Until the moisture content is below 1.0%) to get the dried product, *N*-(4-chloro-1-methyl-7-nitro-1*H*-indazol-3-yl)methanesulfonamide (640.0 g, 76%) as a yellow solid. ¹H NMR (400 MHz, CDCl₃): δ8.05 (d, *J* = 8.32 Hz, 1H), 7.32 (bs, 1H), 7.17 (d, *J* = 8.28 Hz, 1H), 4.15 (s, 3H), 3.45 (s, 3H).

### Step 6: Preparation of N-(4-chloro-1-methyl-7-nitro-1H-indazol-3-yl)-N-(4-methoxybenzyl)methanesulfonamide

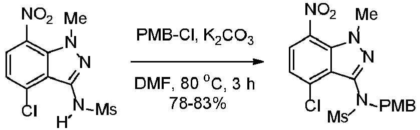

To a mixture of *N*-(4-chloro-1-methyl-7-nitro-1*H*-indazol-3-yl)methanesulfonamide (635.0 g, 2.08 mol, 1.0 equiv.) and 1-(chloromethyl)-4-methoxybenzene (359.0 g, 2.30 mol, 1.1 equiv.) in DMF (6.35 L, 10.0 V) at room temperature was added potassium carbonate (374.7 g, 2.70 mol, 1.3 equiv.). The reaction mixture was heated to 80-90 °C and maintained at that temperature for 3 h. After completion of the reaction (monitored by TLC), the mixture was poured into ice cold water (19.05 L, 30.0 V) [Note: Slow quenching with vigorous stirring is preferred to avoid clumping as the product precipitates]. The resulting solids were isolated via filtration and washed with water (1.90 L, 3.0 V); then the solids were washed with hexanes (1.27 L, 2.0 V). Bulk residual water was removed from the solids by maintaining vacuum filtration for 60-90 min. The isolated solid was dissolved in Ethyl acetate (12.7 L, 20.0 V) and charcoal was added (63.5 g). The mixture was heated to 60-70 °C and then stirred for 30-45 min. at that temperature. The mixture was filtered while still hot (40-50 °C) through a pad of Celite and the Celite pad was then extracted with ethyl acetate (3.17 L, 5.0 V). The combined filtrates were concentrated to dryness under reduced pressure at below 50 °C. Ethyl acetate (0.635 L, 1.0 V) was added to the solids at room temperature. The resultant solid suspension was stirred for 30 min. The solids were isolated via filtration and then were washed with hexanes (1.27 L, 2.0 V). Residual water was removed from the solids by maintaining vacuum filtration for 45-60 min. to afford the product *N*-(4-chloro-1-methyl-7-nitro-1*H*-indazol-3-yl)-*N*-(4-methoxybenzyl) methane sulfonamide (705.0 g, 80% yield) as a yellow solid. ¹H NMR (400 MHz, CDCl₃): δ7.99 (d, *J* = 8.24 Hz, 1H), 7.27 (d, *J* = 8.68 Hz, 2H), 7.19 (d, *J* = 8.24 Hz, 1H), 6.80 (d, *J* = 8.44 Hz, 2H), 4.95-4.76 (m, 2H), 4.17 (s, 3H), 3.76 (s, 3H), 3.01 (s, 3H).

### Step 7: Preparation of N-(7-Amino-4-chloro-1-methyl-1H-indazol-3-yl)-N-(4-methoxybenzyl)methanesulfonamide

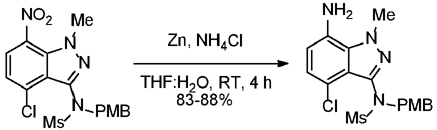

To a stirred suspension of zinc powder (540.0 g, 8.23 mol, 10.0 equiv.) in a mixture of THF (3.50 L, 10.0 V) and water (7.0 L, 20.0 V) at room temperature was added ammonium chloride (NH₄Cl) (449.0 g, 8.23 mol, 10.0 equiv.). To the mixture was added *N*-(4-chloro-1-methyl-7-nitro-1*H*-indazol-3-yl)-*N*-(4-methoxybenzyl)methanesulfonamide (350 g, 0.823 mol, 1.0 equiv.) in THF (7.0 L, 20.0 V). The reaction mixture was stirred at room temperature for 3-4 h. After completion of the reaction (monitored by in-process TLC/HPLC), the mixture was diluted with ethyl acetate (3.5 L, 10.0 V) and water (1.12 L, 2.5 V). The mixture was stirred for 15 min. The reaction mass was filtered through a pad of Celite bed washing with ethyl acetate (1.75 L, 5.0 V). The bi-phasic filtrate was collected, and the phases were separated. The aqueous layer was extracted with ethyl acetate (3.50 L, 10.0 V). The combined organic layers were washed with brine (3.50 L, 10 V), dried over Na₂SO₄, and then concentrated in *vacuo* to afford a crude solid. To the crude product was added MTBE (3.25 L, 10 V) and the suspension was stirred for 30 min at room temperature. The solids were isolated by filtration. Bulk residual water was removed from the solids by maintaining vacuum filtration for 30-45 min. The wet product was dried in a hot air oven (50 °C) for 2 h to afford the title product, *N-*(7-amino-4-chloro-1-methyl-1*H*-indazol-3-yl)-*N*-(4-methoxybenzyl)methanesulfonamide (276.0 g, 85% yield) as off-white solid. ¹H NMR (400 MHz, CDCl₃): *δ*7.29-7.26 (m, 2H), 6.86-6.79 (m, 2H), 6.42 (d, *J* = 7.80 Hz, 1H), 4.99-4.70 (m, 2H), 4.25 (s, 3H), 3.77 (s, 5H), 2.98 (s, 3H).

### Preparation of 2-amino-4-(4-(trifluoromethyl)pyrimidin-2-yl)benzoic acid:

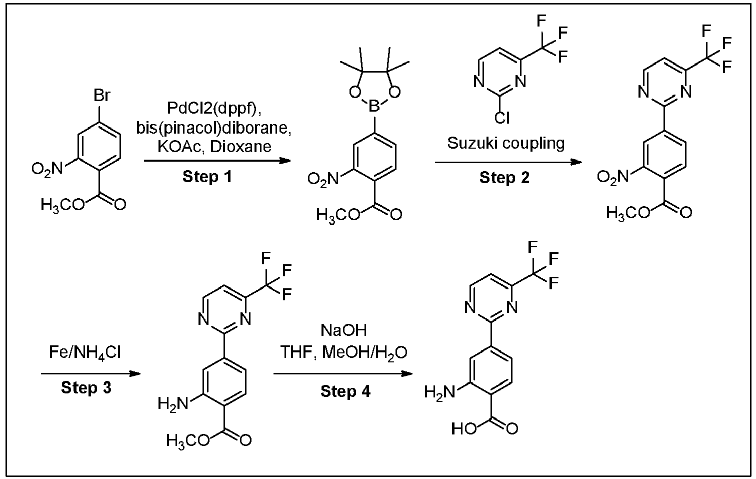

### Step 1: Preparation of methyl 2-nitro-4-(4,4,5,5-tetramethyl-1,3,2-dioxaborolan-2-yl)benzoate

To a stirred solution of methyl 4-bromo-2-nitrobenzoate (600 g, 2307 mmol) in 1,4-dioxane (6000 mL) at 26 °C under N₂ atmosphere was added bis(pinacol)diborane (615 g, 2423 mmol) and potassium acetate (679 g, 6922 mmol). The reaction mixture was degassed by bubbling N₂ gas through the mixture for 10 min. To the reaction mixture was added PdCl₂(dppf) (84 g, 115 mmol) and the mixture was then stirred at 80 °C for 3 hr. The progress of the reaction was monitored by TLC (SiO₂, 20% EtOAc/Pet, Rf = 0.4). On completion, the reaction mixture was concentrated under reduced pressure. The resulting residue was dissolved in EtOAc (6000 mL) and then diluted with water (2400 mL). The mixture was mixed and then filtered through a pad of Celite to remove solids. The Celite pad was extracted with EtOAc (2400 mL). The combined filtrate was partitioned, and the organic layer was isolated, dried over anhydrous Na₂SO₄, filtered, and concentrated under reduced pressure to afford the crude product. This material was triturated with diethyl ether as follows: First, the material was triturated with EtzO (2400 mL) and the resulting off-white solid was collected via filtration, washed with EtzO (2 × 900 mL), and reserved (480 g isolated). The combined filtrate was concentrated under a stream of N₂ gas to half of the original volume, upon which the solution cooled to below ambient temperature and an off-white solid precipitated. The precipitate was collected via filtration, washed with Et₂O (2 × 600 mL), and reserved (90 g isolated). The combined filtrate was concentrated under a stream of N₂ gas to half the original volume, upon which the solution cooled to below ambient temperature and an off-white solid precipitated. The precipitate was collected via filtration, washed with EtzO (2 × 600 mL), and this material (30 g isolated) was combined with the previously isolated solids to afford the product methyl 2-nitro-4-(4,4,5,5-tetramethyl-1,3,2-dioxaborolan-2-yl)benzoate as an off-white solid, 600g (83% yield). ¹H NMR (400 MHz, DMSO-d₆) *δ* = 8.20-8.14 (m, 1H), 8.10-8.05 (m, 1H), 7.87 (d, *J* = 7.5 Hz, 1H), 3.86 (s, 3H), 1.33 (s, 12H). GCMS Purity = 97.5%.

### Step 2: Preparation of methyl 2-nitro-4-(4-(trifluoromethyl)pyrimidin-2-yl)benzoate

To a solution of methyl 2-nitro-4-(4,4,5,5-tetramethyl-1,3,2-dioxaborolan-2-yl)benzoate (600 g, 1954 mmol) and 2-chloro-4-(trifluoromethyl)pyrimidine (357 mL, 2931 mmol) in tetrahydrofuran (THF) (5214 mL) at 26 °C under N₂ atmosphere was added slowly a solution of tribasic potassium phosphate (1244 g, 5861 mmol) in water (1307 mL). The reaction mixture was degassed by bubbling N₂ gas through the mixture for 10 min. To the mixture was added dichloro[9,9-dimethyl-4,5-bis(diphenylphosphino)xanthene]palladium(II) (73.8 g, 98 mmol), then the mixture was stirred under nitrogen atmosphere at 60 °C for 16 hr. The progress of the reaction was monitored by TLC (SiO₂, 20% EtOAc/Pet, Rf = 0.3). On completion, the reaction mixture was concentrated under reduced pressure and the resulting residue was diluted with ethyl acetate (5000 mL) and then washed with water (2 × 2000 mL). The organic layer was dried over anhydrous Na₂SO₄, filtered, and concentrated to dryness. The residue was mixed with diethyl ether (634 mL) and the solids were collected via filtration, washed with EtzO (634 mL), and then dried under vacuum to afford an off-white solid (420 g isolated) which was reserved. The combined filtrate (red in color) was concentrated under a stream of N₂ gas to half of the original volume upon which an off-white solid precipitated. The solids were collected via filtration, were washed with diethyl ether (2 × 634 mL), and the isolated material (80 g) was combined with the previously isolated solids. The blended material was dried under vacuum to afford methyl 2-nitro-4-(4-(trifluoromethyl)pyrimidin-2-yl)benzoate as an off-white solid, 500 g (75% yield). ¹H NMR (400 MHz, DMSO-d₆) *δ* = 9.39 (d, *J* = 5.0 Hz, 1H), 8.91 (d, *J* = 1.4 Hz, 1H), 8.82-8.78 (m, 1H), 8.14 (d, *J* = 5.1 Hz, 1H), 8.08 (d, *J* = 8.1 Hz, 1H), 3.91 (s, 3H). LCMS purity = 95.71%.

### Step 3: Preparation of methyl 2-amino-4-(4-(trifluoromethyl)pyrimidin-2-yl)benzoate

To a solution methyl 2-nitro-4-(4-(trifluoromethyl) pyrimidin-2-yl) benzoate (500 g, 1528 mmol) in ethanol (6945 mL) and water (695 mL) at 26 °C was added Fe powder (853 g, 15.3 mol) and NH₄Cl (817 g, 15.3 mol). The reaction mixture was stirred at 70 °C for 16 hr. The progress of the reaction was monitored by TLC (SiO₂, 20% EtOAc/Pet Rf: 0.4). On completion, the reaction mixture was filtered while hot through a Celite pad and the filtrate was concentrated under reduced pressure. The resulting residue was diluted with water (2500 mL) and then extracted with EtOAc (2 × 5000 mL). The combined organics were washed with brine solution (3000 mL) and then dried over anhydrous Na₂SO₄, filtered, and concentrated under reduced pressure to afford methyl 2-amino-4-(4-(trifluoromethyl)pyrimidin-2-yl)benzoate as a yellow solid, 330 g (69% yield). ¹H-NMR (400 MHz, DMSO-d₆) *δ* = 9.30-9.28 (m, 1H), 8.00 (d, *J* = 5.0 Hz, 1H), 7.93 (d, *J* = 1.5 Hz, 1H), 7.88 (d, *J* = 8.5 Hz, 1H), 7.55 (dd, *J* = 1.7, 8.5 Hz, 1H), 6.93 (s, 2H), 3.84 (s, 3H). LCMS purity = 95.51%.

### Step 4: Preparation of 2-amino-4-(4-(trifluoromethyl)pyrimidin-2-yl)benzoic acid

To a solution of methyl 2-amino-4-(4-(trifluoromethyl)pyrimidin-2-yl)benzoate (320 g, 1077 mmol) in methanol (1600 mL) and THF (1600 mL) at 20 °C was added dropwise aq. 5M sodium hydroxide (646 mL, 3230 mmol). The reaction mixture was stirred 60 °C for 16 hr. The progress of the reaction was monitored by TLC (SiO₂, 80% EtOAc/Pet. Rf: 0.3). On completion, reaction mixture was concentrated under reduced pressure to remove the volatile organics and the resulting residue was then acidified to pH~4 by the addition of aq. 1N HCl. The solids were collected via filtration and were washed with water (5000 mL). The solids were dried under vacuum and then further dried in a 50 °C oven for 24 hr to afford 2-amino-4-(4-(trifluoromethyl)pyrimidin-2-yl)benzoic acid as a yellow solid, 288 g (91% yield). ¹H-NMR (400 MHz, DMSO-d₆) *δ* = 9.31-9.26 (m, 1H), 7.99 (d, *J* = 5.0 Hz, 1H), 7.90-7.85 (m, 2H), 7.53 (dd, *J* = 1.7, 8.4 Hz, 1H). LCMS Purity = 96.71%.

### Preparation of (S)-N-((6P)-7-(2-(1-amino-2-(3,5-difluorophenyl)ethyl)-4-oxo-7-(4-(trifluoromethyl)pyrimidin-2-yl)quinazolin-3(4H)-yl)-4-chloro-1-methyl-1H-indazol-3-yl)methanesulfonamide:

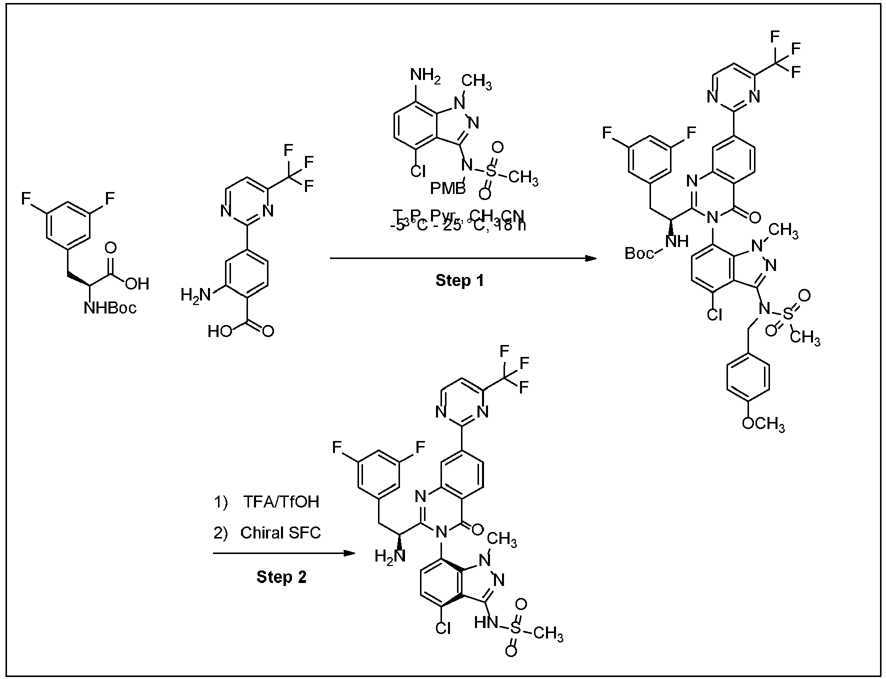

### Step 1: Preparation of tert-butyl (S)-(1-(3-(4-chloro-3-(N-(4-methoxybenzyl)methylsulfonamido)-1-methyl-1H-indazol-7-yl)-4-oxo-7-(4-(trifluoromethyl)pyrimidin-2-yl)-3,4-dihydroquinazolin-2-yl)-2-(3,5-difluorophenyl)ethyl)carbamate

To a stirred solution of (S)-2-((tert-butoxycarbonyl)amino)-3-(3,5-difluorophenyl) propanoic acid (76 g, 253 mmol) and 2-amino-4-(4-(trifluoromethyl)pyrimidin-2-yl)benzoic acid (79 g, 279 mmol) in acetonitrile (2560 mL) was added pyridine (49.2 mL, 608 mmol) and the mixture was then cooled to -5 °C and stirred at this temperature for 10 min. To the mixture at -5 °C was slowly added T3P (50% in EtOAc, 754 mL, 1266 mmol). The reaction mixture was stirred at -5 °C for 2 hr, then to the mixture was added N-(7-amino-4-chloro-1-methyl-1H-indazol-3-yl)-N-(4-methoxybenzyl)methanesulfonamide (100 g, 253 mmol) in one portion. The mixture was slowly warmed to 25 °C and was stirred for 18 hr. The progress of the reaction was monitored by TLC (SiO₂, 40% EtOAc/Pet. Rf = 0.2). On completion, the reaction mixture was concentrated under reduced pressure to remove acetonitrile and pyridine. The resulting residue was diluted in EtOAc (2500 mL) and was washed with aq. 2M sodium hydroxide (~4000 mL) followed by brine (4000 mL). The organic layer was dried over Na₂SO₄, filtered, and concentrated under reduced pressure to afford the crude product. This material was purified by silica gel chromatography eluting with 30-40% EtOAc/Pet. ether. The fractions containing the desired product were collected and concentrated under reduced pressure to afford tert-butyl (S)-(1-(3-(4-chloro-3-(N-(4-methoxybenzyl)methylsulfonamido)-1-methyl-1H-indazol-7-yl)-4-oxo-7-(4-(trifluoromethyl)pyrimidin-2-yl)-3,4-dihydroquinazolin-2-yl)-2-(3,5-difluorophenyl)ethyl)carbamate (215 g, 87% yield, a yellow solid) as a mixture of homochiral atropisomers (diastereomers). LCMS Purity = 94.91%.

### Step 2: Preparation of (S)-N-((6P)-7-(2-(1-amino-2-(3,5-difluorophenyl)ethyl)-4-oxo-7-(4-(trifluoromethyl)pyrimidin-2-yl)quinazolin-3(4H)-yl)-4-chloro-1-methyl-1H-indazol-3-yl)methanesulfonamide

To a stirred solution of tert-butyl (S)-(1-(3-(4-chloro-3-(N-(4-methoxybenzyl) methylsulfonamido)-1-methyl-1H-indazol-7-yl)-4-oxo-7-(4-(trifluoromethyl)pyrimidin-2-yl)-3,4-dihydroquinazolin-2-yl)-2-(3,5-difluorophenyl)ethyl)carbamate (425 g, 459 mmol) in TFA (1415 ml, 18.4 mol) at 27 °C was added slowly triflic acid (122 ml, 1378 mmol). The solution was and stirred under nitrogen atmosphere for 2 hr. The progress of the reaction was monitored by TLC (SiO₂, 50% EtOAc/Pet. Rf = 0.2). On completion, the volatiles were removed under a gentle stream of nitrogen gas. The resulting residue was dissolved in EtOAc (3000 mL). The organic solution was washed with and 2M sodium hydroxide (4000 mL, sufficient to achieve aq. phase pH >7), followed by brine (4000 mL). The organic layer was dried over Na₂SO₄, filtered and concentrated under reduced pressure to afford crude (S)-N-(7-(2-(1-amino-2-(3,5-difluorophenyl)ethyl)-4-oxo-7-(4-(trifluoromethyl)pyrimidin-2-yl)quinazolin-3(4H)-yl)-4-chloro-1-methyl-1H-indazol-3-yl)methanesulfonamide (425 g, 97% yield, a yellow solid) as a mixture of homochiral atropisomers (diastereomers). The above procedure was repeated and the product from both instances was combined (825 g total), blended, and then purified by silica gel chromatography eluting with 5-10% MeOH in DCM. Fractions containing the desired product were pooled and then concentrated under reduced pressure to afford a yellow solid, 580 g, a mixture of atropisomers (diastereomers). A portion of this material (490 g) was mixed with methanol (5000 mL, 10V) and then filtered to remove the solids which were reserved. The filtrate was concentrated under reduced pressure to afford 255 g of the product, a ~85:15 ratio of atropisomers favoring the desired atropisomer. This material was dissolved in methanol:acetonitrile (10:90, app. 1.5 L). The resulting solution was subjected to prep-SFC chromatography using the following method: Column = (R,R) Welk-01, 30 × 250 mm, 5µ; Eluent = CO₂:methanol (1:1); Flow-rate = 100.0 g/min.; Back-pressure = 100.0 bar; Detection = 254 nm (UV); Stack time = 14.0 min.; Load per injection = 1000 mg. The two peaks were collected separately and the major peak (second to elute) was concentrated under reduced pressure to afford (S)-N-((6P)-7-(2-(1-amino-2-(3,5-difluorophenyl)ethyl)-4-oxo-7-(4-(trifluoromethyl)pyrimidin-2-yl)quinazolin-3(4H)-yl)-4-chloro-1-methyl-1H-indazol-3-yl)methanesulfonamide as a yellow solid, 199 g. This material was contaminated with 0.5% of the other atropisomer (first peak to elute); therefore, a portion of the material (173 g) was subjected to a second round of prep-SFC purification following the method described above. Fractions corresponding to the major peak were pooled and concentrated under reduced pressure to obtain (S)-N-((6P)-7-(2-(1-amino-2-(3,5-difluorophenyl)ethyl)-4-oxo-7-(4-(trifluoromethyl)pyrimidin-2-yl)quinazolin-3(4H)-yl)-4-chloro-1-methyl-1H-indazol-3-yl)methanesulfonamide as a yellow solid, 160 g. The material is a single stereoisomer. ¹H-NMR (300 MHz, DMSO-d₆) δ = 9.42 (d, *J* = 5.1 Hz, 1H), 8.81 (d, *J* = 1.5 Hz, 1H), 8.59 (dd, *J* = 8.9, 2.9 Hz, 1H), 8.39 (d, *J* = 8.4 Hz, 1H), 8.13 (d, *J* = 5.1 Hz, 1H), 7.40-7.34 (m, 2H), 7.01 (tt, *J* = 9.5, 2.2 Hz, 1H), 6.81-6.72 (m, 2H), 3.71 (s, 3H), 3.62-3.57 (m, 1H), 3.38-3.34 (m, 1H), 3.23 (s, 3H), 2.92-2.85 (m, 1H). LCMS Purity = 95.86%.

### Preparation of N-(7-amino-4-chloro-1-(2,2-difluoroethyl)-1H-indazol-3-yl)-N-(4-methoxybenzyl)methanesulfonamide:

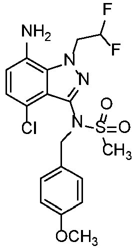

### Synthesis Scheme:

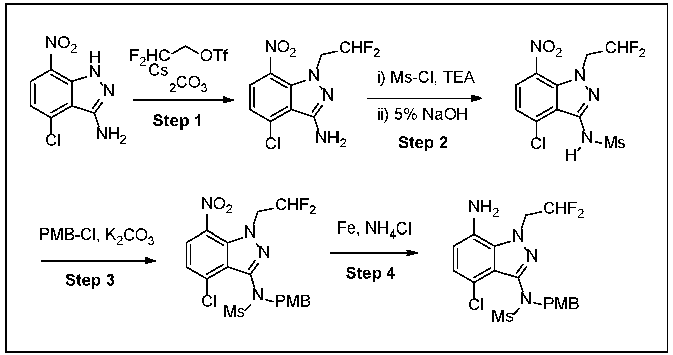

### Step 1: Preparation of 4-chloro-1-(2,2-difluoroethyl)-7-nitro-1H-indazol-3-amine

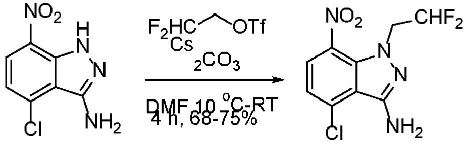

To a stirred solution of 4-chloro-7-nitro-1*H*-indazol-3-amine (180 g, 0.85 mol, 1.0 equiv.) in DMF (1.8 L, 10.0 V) at 10-15 °C was added cesium carbonate (Cs₂CO₃) (551 g, 1.70 mol, 2.0 equiv.) at a rate necessary to maintaining the reaction mass below 20 °C. The mixture was stirred for 5-10 min, then to the stirred mixture at 10-15 °C was added 2,2-difluoroethyl trifluoromethanesulfonate (133 mL, 0.93 mol, 1.1 equiv.) at a rate necessary to maintain the reaction mass below 20 °C (Note: Slow addition is preferred to obtain more favorable regio-selectivity). The reaction mass was allowed to slowly warm to room temperature and was then stirred at the same temperature for 3 h. After completion of the reaction (monitored by TLC), the reaction mass was quenched by the addition of ice-cold water (5.4 L, 30.0 V) and the resulting mixture was allowed to warm to room temperature with stirring for 6-8 h. The solids were isolated via filtration and were then washed with water (540 mL, 3.0 V). The wet solid was washed with hexanes (0.9 L, 5.0 V). Bulk residual water was removed from the solids by maintaining vacuum filtration for 60-90 min. The wet solid was dried in a hot air oven for 7-8 h at 50 °C (until the moisture content was below 1.0%). The isolated material, 4-chloro-1-(2,2-difluoroethyl)-7-nitro-1*H*-indazol-3-amine (160 g, 71% yield), was used in the next step without further purification. ¹H NMR (400MHz, CDCl₃): δ8.05 (d, *J* = 8.4 Hz, 1H), 7.07 (d, *J* = 8.4 Hz, 1H), 6.00 (tt, *J₁* = 3.9 Hz, *J₂* = 7.7 Hz, 1H), 4.76 - 4.84 (m, 4H).

### Step 2: Preparation of N-(4-chloro-1-(2,2-difluoroethyl)-7-nitro-1H-indazol-3-yl)methane sulfonamide

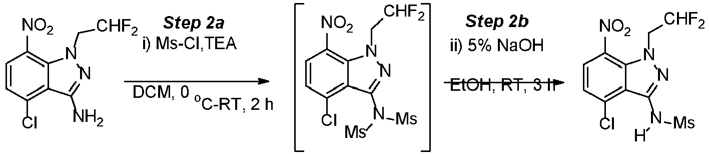

Step 2a: To a solution of 4-chloro-1-(2,2-difluoroethyl)-7-nitro-1*H*-indazol-3-amine (170.0 g, 0.96 mol, 1.0 equiv.) in DCM (1.7 L, 10.0 V) at 0-5 °C was added triethyl amine (264 mL, 2.88 mol, 3.0 equiv.), followed by 4-dimethylaminopyridine (3.4 g, 0.048 mol, 0.05 equiv.). The reaction mass was stirred for 5-10 min., then methanesulfonyl chloride (120 mL, 2.4 mol, 2.5 equiv.) was added slowly while maintaining the reaction mass below 10 °C. The reaction mixture was allowed to warm to room temperature and then was stirred for 1.5-2.0 h. After completion of the reaction (monitored by TLC), the mixture was diluted with water (1.7 L, 10.0 V) and then stirred at room temperature for 15 min. The organic layer was separated, and the aqueous layer was extracted with DCM (1.7 L, 10.0 V). The combined organic layers were washed with 10% brine solution (340 mL, 2.0 V), dried over Na₂SO₄ and concentrated to afford the product as a crude solid. The solids were triturated with hexanes (340 mL, 2.0 V) at room temperature to obtain *N*-(4-chloro-1-(2,2-difluoroethyl)-7-nitro-1*H-*indazol-3-yl)-*N*-(methylsulfonyl) methanesulfonamide which was used directly in the next step. Step 2b: To a stirred solution of *N*-(4-chloro-1-(2,2-difluoroethyl)-7-nitro-1*H*-indazol-3-yl)-*N*-(methylsulfonyl) methanesulfonamide (entirety of material prepared above) in ethanol (1.7 L, 10.0 V) at room temperature was added slowly aq. 5% NaOH solution (1.19 L, 7.0 V) [Note: Slow addition is preferred via dropping funnel]. The reaction mass was stirred at the same temperature for 3 h. After completion of the reaction [Sample preparation for TLC analysis: an aliquot of reaction solution (~1 mL) was acidified with aq. 2.0 N HCl to reach the pH 2-3; then the mixture was extracted with ethyl acetate and organic layer was analyzed by TLC], the reaction mass was cooled to 0-5 °C and the pH was adjusted to 2-3 by the addition of aq. 2.0 N HCl (~850 mL, 5.0 V) at below 10 °C [Note: Precipitation occurred upon addition of HCl and the solids increased gradually with stirring]. The reaction mixture was warmed to room temperature and then stirred for 1.5-2.0 h. Solids obtained were isolated via filtration and were then washed with water (340 mL, 2.0 V); followed by washing with hexanes (340 mL, 2.0 V). Bulk residual water was removed from the solids by maintaining vacuum filtration for 60-90 min. The wet material was dried in a hot air oven at 50 °C for 6-7 h (until the moisture content was below 1.0%) to afford the dried product, *N*-(4-chloro-1-(2,2-difluoroethyl)-7-nitro-1*H*-indazol-3-yl)methanesulfonamide (170.0 g, 75%) as a yellow solid. ¹H NMR (400MHz, CDCl₃): *δ*8.15 (d, *J* = 8.3 Hz, 1H), 7.52 (bs, 1H), 7.24 (d, *J* = 8.3 Hz, 1H), 6.04 (tt, *J₁* = 3.7 Hz, *J₂* = 7.9 Hz, 1H), 5.02 (td, *J₁* = 3.9 Hz, *J₂* = 14.3 Hz, 2H), 3.42 (s, 4H).

### Step 3: Preparation of N-(4-chloro-1-(2,2-difluoroethyl)-7-nitro-1H-indazol-3-yl)-N-(4-methoxy benzyl)methanesulfonamide

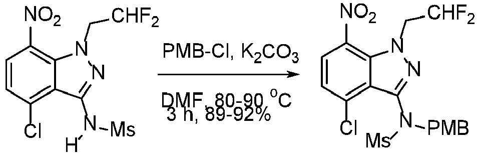

To a mixture of *N*-(4-chloro-1-(2,2-difluoroethyl)-7-nitro-1*H*-indazol-3-yl)methane sulfonamide (160.0 g, 0.45 mol, 1.0 equiv.) and 1-(chloromethyl)-4-methoxybenzene (67.6 mL, 0.5 mol, 1.1 equiv.) in DMF (1.6 L, 10.0 V) at room temperature was added potassium carbonate (93.8 g, 0.59 mol, 1.3 equiv.). The reaction mixture was heated to 80-90 °C and maintained at the same temperature for 3 h. After completion of the reaction (monitored by TLC), the mixture was poured into ice cold water (4.8 L, 60.0 V) [Note: Slow quenching with vigorous stirring is preferred to avoid clumping as the product precipitates]. The resulting solids were isolated via filtration and washed with water (480 mL, 3.0 V); then the solids were washed with hexanes (320 mL, 2.0 V). Bulk residual water was removed from the solids by maintaining vacuum filtration for 1-2 h. The isolated solid was dissolved in ethyl acetate (1.6 L, 10.0 V) and charcoal was added (16.0 g). The mixture was heated to 60-70 °C and then stirred for 30-45 min. at that temperature. The mixture was filtered while hot (40-50 °C) through a pad of Celite and the Celite pad was then extracted with ethyl acetate (800 mL, 5.0 V). The combined filtrates were concentrated to dryness under reduced pressure at below 50 °C. To the resulting solids at room temperature was added ethyl acetate (160 mL, 1.0 V). The suspension was stirred for 30 min. The solids were isolated via filtration and then were washed with hexanes (320 mL, 2.0 V). Residual water was removed from the solids by maintaining vacuum filtration for 45-60 min. to afford the product *N*-(4-chloro-1-(2,2-difluoroethyl)-7-nitro-1*H*-indazol-3-yl)-*N*-(4-methoxybenzyl)methanesulfonamide (180.0 g, 92% yield) as a yellow solid. ¹H NMR (400MHz, CDCl₃): *δ* 8.06 (d, *J* = 8.4 Hz, 1H), 7.52 (bs, 1H), 7.27 - 7.21 (m, 4H), 6.77 (d, *J* = 8.3 Hz, 2H), 6.01 (tt, *J₁* = 3.8 Hz, *J₂* = 7.9 Hz, 1H), 5.12 - 4.78 (m, 4H), 3.74 (s, 3H), 3.02 (s, 3H).

### Step 4: Preparation of N-(7-amino-4-chloro-1-(2,2-difluoroethyl)-1H-indazol-3-yl)-N-(4-methoxybenzyl)methanesulfonamide

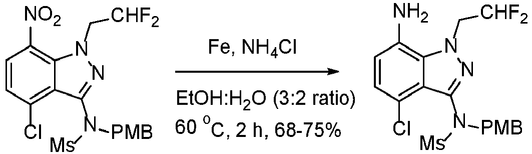

To a stirred suspension of iron powder (76.5 g, 1.37 mol, 5.0 equiv.) in a mixture of EtOH (650 mL, 5.0 V) and water (780 mL, 6.0 V) at room temperature was added ammonium chloride (118.0 g, 2.18 mol, 8.0 equiv.). To the mixture was added *N*-(4-chloro-1-(2,2-difluoroethyl)-7-nitro-1*H*-indazol-3-yl)-*N*-(4-methoxybenzyl)methanesulfonamide (130 g, 0.27 mol, 1.0 equiv.) in EtOH (520 mL, 4.0 V). The reaction mixture was heated to 60 °C and then stirred for 2 h. After completion of the reaction (monitored by in-process TLC/HPLC), the mixture was cooled to room temperature and diluted with ethyl acetate (1.3 L, 10.0 V) and water (390 mL, 3.0 V). The mixture was stirred for 15 min. The mixture was filtered through a pad of Celite and the Celite pad was then extracted with ethyl acetate (650 mL, 5.0 V). The bi-phasic filtrate was partitioned, and the organic phase was reserved while the aqueous layer was extracted with ethyl acetate (650 mL, 5.0 V). The combined organic layers were washed with brine (1.3 L, 10 V), dried over Na₂SO₄, and then concentrated *in vacuo* to afford a crude solid. To the crude product was added MTBE (650 mL, 5.0 V) and the suspension was stirred for 30 min. at room temperature. The solids were isolated via filtration. Bulk residual water was removed from the solids by maintaining vacuum filtration for 30-45 min. The wet product was dried in a hot air oven (50 °C) for 2 h to afford the title compound /IF(7-amino-4-chloro-1-(2,2-difluoroethyl)-1*H*-indazol-3-yl)-*N*-(4-methoxy benzyl)methanesulfonamide (100.0 g, 70% yield) as off-white solid. ¹H NMR (400MHz, CDCl₃): *δ*7.21 (d, *J* = 8.5 Hz, 2H), 6.87 (d, *J* = 8.4 Hz, 1H), 6.78 (d, *J* = 8.5 Hz, 2H), 6.52 (d, *J* = 8.3 Hz, 1H), 6.01 (tt, *J₁* = 3.8 Hz, *J₂* = 7.7 Hz, 1H), 4.98-4.69 (m, 4H), 3.75 (s, 3H), 2.98 (s, 3H).

### Preparation of N-(7-amino-4-chloro-1-(2,2-difluoroethyl)-1H-indazol-3-yl)-N-(4-methoxybenzyl)cyclopropanesulfonamide

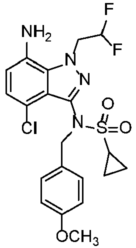

### Synthesis Scheme:

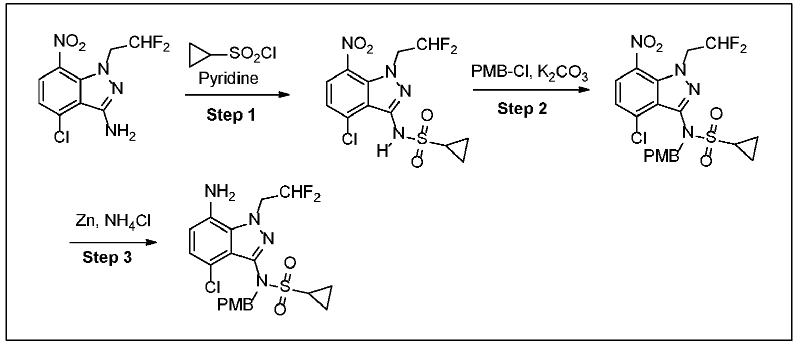

### Step 1: Preparation of N-(4-chloro-1-(2,2-difluoroethyl)-7-nitro-1H-indazol-3-yl)cyclopropanesulfonamide

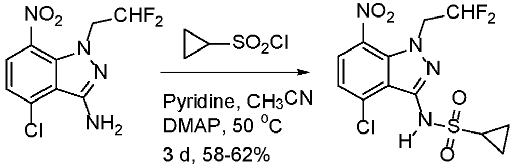

To a stirred solution of 4-chloro-1-(2,2-difluoroethyl)-7-nitro-1*H*-indazol-3-amine (150.0 g, 0.54 mol, 1.0 equiv.) in acetonitrile (600 mL, 4.0 V) at room temperature was added pyridine (600 mL, 4.0 V), followed by the addition of 4-dimethylaminopyridine (30.0 g, 0.27 mol, 0.5 equiv.). The reaction mass was stirred for 5-10 min., then cyclopropylsulfonyl chloride (114 mL, 1.08 mol, 2.0 equiv.) was added at room temperature. The reaction mixture was heated to 50 °C and then stirred at that temperature for 3 days. After completion of the reaction (monitored by TLC), the mixture was cooled to room temperature and diluted with water (1.5 L, 10.0 V) and ethyl acetate (1.5 L, 10.0 V), then stirred at room temperature for 15 min. The organic layer was separated, and the aqueous layer was extracted with EtOAc (300 mL, 2.0 V). The combined organic layers were washed with aq. 1.0 N HCl (600 mL, 4.0 V), followed by 10% brine solution (1.5 L, 10.0 V). The organic layer was dried over Na₂SO₄, filtered, and then concentrated under reduced pressure to afford *N*-(4-chloro-1-(2,2-difluoroethyl)-7-nitro-1*H*-indazol-3-yl)cyclopropanesulfonamide (124.0 g, 61%) as a viscous liquid. ¹H NMR (400MHz, CDCl₃): *δ*8.11 (d, *J* = 8.5 Hz, 1H), 7.25 (d, *J* = 8.5 Hz, 1H), 6.04 (tt, *J₁* = 3.8 Hz, *J₂* = 7.7 Hz, 1H), 5.05 (td, *J₁* = 3.8 Hz, *J₂* = 14.4 Hz, 2H), 3.06 - 3.00 (m, 1H), 1.65 - 1.42 (m, 2H), 1.19 - 1.13 (m, 2H).

### Step 2: Preparation of N-(4-chloro-1-(2,2-difluoroethyl)-7-nitro-1H-indazol-3-yl)-N-(4-methoxybenzyl)cyclopropanesulfonamide

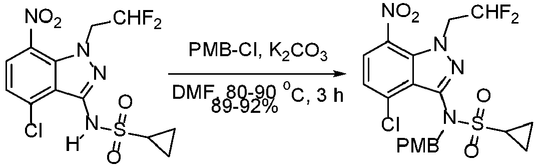

To a mixture of *N*-(4-chloro-1-(2,2-difluoroethyl)-7-nitro-1*H*-indazol-3-yl)cyclopropanesulfonamide (100.0 g, 0.20 mol, 1.0 equiv.) and 1-(chloromethyl)-4-methoxybenzene (39.2 mL, 0.22 mol, 1.1 equiv.) in DMF (1.0 L, 10.0 V) at room temperature was added potassium carbonate (128 g, 0.33 mol, 1.3 equiv.). The reaction mixture was heated to 80-90 °C and maintained at that temperature for 3 h. After completion of the reaction (monitored by TLC), the mixture was poured into ice cold water (3.0 L, 30.0 V) [Note: Slow quenching with vigorous stirring is preferred to avoid clumping as the product precipitates]. The resulting solids were isolated via filtration and washed with water (300 mL, 3.0 V); then the solids were washed with hexanes (300 mL, 3.0 V). Bulk residual water was removed from the solids by maintaining vacuum filtration for 1-2 h. The wet solid was dissolved in ethyl acetate (500 mL, 5.0 V) and charcoal was added (10.0 g). The mixture was heated to 60-70 °C and then stirred for 30-45 minutes at that temperature. The mixture was filtered while hot (40-50 °C) through a pad of Celite and the Celite pad was extracted with ethyl acetate (500 mL, 5.0 V). The combined filtrates were concentrated to dryness under reduced pressure at below 50 °C to afford *N*-(4-chloro-1-(2,2-difluoroethyl)-7-nitro-1*H*-indazol-3-yl)-/V-(4-methoxy- benzyl)cyclopropanesulfonamide (122.0 g, 92% yield) as a yellow solid. ¹H NMR (400MHz, CDCl₃): δ8.05 (d, *J* = 8.6 Hz, 1H), 7.26-7.22 (m, 3H), 6.73 (d, *J* = 8.5 Hz, 2H), 5.98 (tt, *J₁* = 3.7 Hz, *J₂* = 7.8 Hz, 1H), 5.09-4.88 (m, 4H), 3.72 (s, 3H), 2.65-2.60 (m, 1H), 1.15-1.06 (m, 2H), 0.89 - 0.86 (m, 2H).

### Step 3: Preparation of N-(7-amino-4-chloro-1-(2,2-difluoroethyl)-1H-indazol-3-yl)-N-(4-methoxybenzyl)cyclopropanesulfonamide

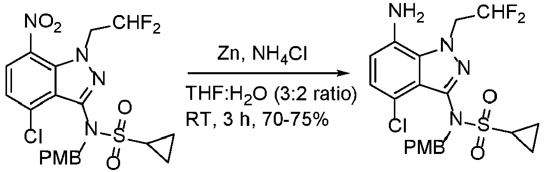

To a stirred suspension of zinc powder (156.0 g, 2.4 mol, 10.0 equiv.) in a mixture of THF (1.2 L, 10.0 V) and water (2.4 L, 20.0 V) at room temperature was added ammonium chloride (129.0 g, 2.40 mol, 10.0 equiv.). To the mixture was added *N*-(4-chloro-1-(2,2-difluoroethyl)-7-nitro-1*H*-indazol-3-yl)-*N*-(4-methoxybenzyl)cyclopropanesulfonamide (120 g, 0.2 mol, 1.0 equiv.) in THF (2.4 L, 20.0 V). The reaction mixture was stirred at room temperature for 2 h. After completion of the reaction (monitored by in-process TLC/HPLC), the mixture was diluted with ethyl acetate (1.2 L, 10.0 V) and water (360 mL, 3.0 V). The mixture was stirred for 15 min. The mixture was filtered through Celite and the Celite pad was extracted with ethyl acetate (600 mL, 5.0 V). The bi-phasic filtrate was partitioned, and the organic phase was reserved while the aqueous layer was extracted with ethyl acetate (600 mL, 5.0 V). The combined organic layers were washed with 10% brine solution (1.2 L, 10 V), dried over Na₂SO₄, filtered, and then concentrated *in vacuo* to afford a crude solid. To the crude product was added MTBE (600 mL, 5.0 V) and the suspension was stirred for 30-45 min. at room temperature. The solids were isolated by filtration and then bulk residual water was removed from the solids by maintaining vacuum filtration for 30-45 min. The wet product was dried in a hot air oven (50 °C) for 2 h to afford the product, *N*-(7-amino-4-chloro-1-(2,2-difluoroethyl)-1*H*-indazol-3-yl)-*N*-(4-methoxybenzyl)cyclopropanesulfonamide (81.0 g, 73% yield) as off-white solid. ¹H NMR (400MHz, CDCl₃): *δ* 7.25 (d, *J* = 8.5 Hz, 2H), 6.93 (d, *J* = 8.4 Hz, 1H), 6.75 (d, *J* = 8.3 Hz, 2H), 6.57 (d, *J* = 8.4 Hz, 1H), 6.03 (tt, *J₁* = 3.7 Hz, *J₂* = 7.9 Hz, 1H), 4.80-4.95 (m, 4H), 3.74 (s, 3H), 2.67-2.61 (m, 1H), 1.14 (d, *J* = 2.4 Hz, 2H), 0.96 (d, *J* = 2.3 Hz, 2H).

### Preparation of N-(7-amino-4-chloro-1-(2,2,2-trifluoroethyl)-1H-indazol-3-yl)-N-(4-methoxybenzyl)methanesulfonamide

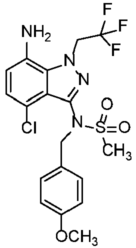

### Synthesis Scheme:

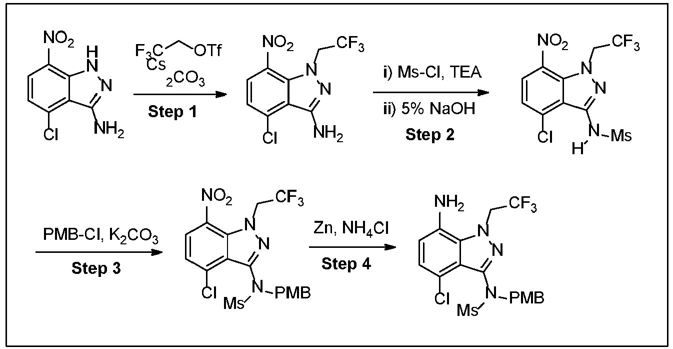

### Step 1: Preparation of 4-chloro-7-nitro-1-(2,2,2-trifluoroethyl)-1H-indazol-3-amine

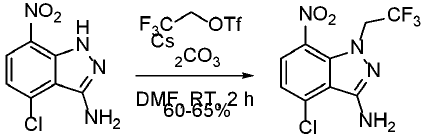

To a stirred solution of 4-chloro-7-nitro-1*H*-indazol-3-amine (50 g, 0.23 mol, 1.0 equiv.) in DMF (500 mL, 10.0 V) at 10-15 °C was added cesium carbonate (Cs₂CO₃) (153.3 g, 0.47 mol, 2.0 equiv.) at a rate sufficient to maintain the reaction mass below 20 °C. The mixture was stirred for 5-10 min, then to the stirred mixture at 10-15 °C was added 2,2,2-trifluoroethyl trifluoromethanesulfonate (60.18 g, 0.26 mol, 1.1 equiv.) at a rate sufficient to maintain the reaction mass below 20 °C (Note: slow addition is preferred for obtaining more favorable regio-selectivity). The reaction mass was allowed to slowly warm to room temperature and was then stirred at the same temperature for 2 h. After completion of the reaction (monitored by TLC), the reaction mass was quenched via the addition of ice-cold water (1.5 L, 30.0 V) and the resulting mixture was allowed to warm to room temperature with stirring for 6-8 h. The solids were isolated via filtration and were then washed with water (150 mL, 3.0 V). The wet solid was washed with hexanes (250 mL, 5.0 V) and then bulk residual water was removed from the solids by maintaining vacuum filtration for 60-90 min. The wet solid was dried in a hot air oven for 7-8 h at 50 °C (until the moisture content was below 1.0%). The isolated material, 4-chloro-7-nitro-1-(2,2,2-trifluoroethyl)-1*H*-indazol-3-amine (45.0 g, 60% yield), was used directly in the next step without further purification. ¹H-NMR (400 MHz, CDCl₃): δ8.09 (d, *J* = 8.40 Hz, 1H), 7.12 (d, *J* = 8.40 Hz, 1H), 5.14 (q, *J* = 8.52 Hz, 2H), 4.77 (bs, H).

### Step 2: Preparation of N-(4-chloro-7-nitro-1-(2,2,2-trifluoroethyl)-1H-indazol-3-yl)methanesulfonamide

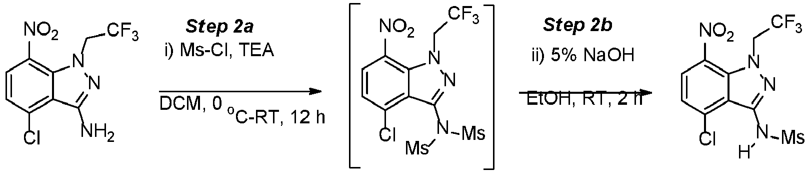

(Step 2a): To a solution of 4-chloro-7-nitro-1-(2,2,2-trifluoroethyl)-1*H*-indazol-3-amine (20.0 g, 0.068 mol, 1.0 equiv.) in DCM (200 mL, 10.0 V) at 0-5 °C. was added triethylamine (29.0 mL, 0.204 mol, 3.0 equiv.), followed by the addition of 4-dimethylaminopyridine (415 mg, 0.03 mol, 0.05 equiv.). The reaction mass was stirred for 5-10 min., then to the mixture was added methanesulfonyl chloride (13.25 mL, 0.17 mol, 2.5 equiv) at a rate sufficient to maintain the reaction mass below 10 °C. The reaction mixture was allowed to warm to room temperature with stirring for 12 h. After completion of the reaction (monitored by TLC), the mixture was diluted with water (200 mL, 10.0 V) and then stirred at room temperature for 15 min. The organic layer was separated, and the aqueous layer was extracted with DCM (200 mL, 10.0 V). The combined organic layers were washed with 10% brine solution (60 mL, 3.0 V), dried over Na₂SO₄, filtered, and concentrated to afford the crude solids. The solids were triturated with hexanes (60 mL, 3.0 V) at room temperature to obtain the intermediate, *N*-(4-chloro-7-nitro-1-(2,2,2-trifluoroethyl)-1*H*-indazol-3-yl)-*N*-(methylsulfonyl)methanesulfonamide, which was used directly in the next step.
(Step 2b): To a stirred solution of *N*-(4-chloro-7-nitro-1-(2,2,2-trifluoroethyl)-1*H*-indazol-3-yl)-*N*-(methylsulfonyl)methanesulfonamide (entirety of the material prepared above) in ethanol (200 mL, 10.0 V) at room temperature was added slowly aq. 5% NaOH solution (140 mL, 7.0 V) [Note: Slow addition is preferred via dropping funnel]. The reaction mass was stirred at the same temperature for 2 h. After completion of the reaction [Sample preparation for TLC analysis: An aliquot of the reaction solution (~1.0 ml) was acidified by the addition of aq. 2.0 N HCl to reach pH 2-3; then the mixture was extracted with ethyl acetate and the organic phase was analyzed by TLC], the reaction mass was cooled to 0-5 °C and the pH was adjusted to 2-3 by the addition of aq. 2.0 N HCl (100 mL, 5.0 V) while maintain the temperature below 10 °C [Note: Precipitation occurred upon addition of HCl and increased with stirring]. The reaction mixture was warmed to room temperature and then stirred for 1.5-2.0 h. The solids were isolated via filtration and were then washed with water (60 mL, 3.0 V), followed by washing with hexanes (60 mL, 3.0 V). Bulk residual water was removed from the solids by maintaining vacuum filtration for 60-90 min. The wet material was dried in a hot air oven at 50 °C for 6-7 h (until the moisture content was below 1.0%) to afford *N*-(4-chloro-7-nitro-1-(2,2,2-trifluoroethyl)-1*H*-indazol-3-yl)methanesulfonamide (22.1 g, 87%) as a yellow solid. ¹H NMR (400 MHz, CDCl₃): δ8.19 (d, *J* = 8.40 Hz, 1H), 7.56 (bs, 1H), 7.30 (d, *J* = 8.40 Hz, 1H), 5.34 (q, *J* = 8.30 Hz, 2H), 3.46 (s, 3H).

### Step 3: Preparation of N-(4-chloro-7-nitro-1-(2,2,2-trifluoroethyl)-1H-indazol-3-yl)-N-(4-methoxybenzyl)methanesulfonamide

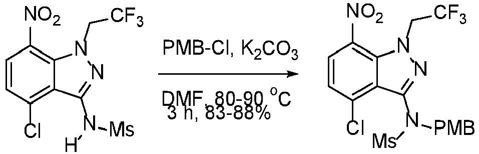

To a mixture of *N*-(4-chloro-7-nitro-1-(2,2,2-trifluoroethyl)-1*H*-indazol-3-yl)methanesulfonamide (50.0 g, 0.134 mol, 1.0 equiv.) and 1-(chloromethyl)-4-methoxybenzene (23.0 g, 0.147 mol, 1.1 equiv.) in DMF (500 mL, 10.0 V) at room temperature was added potassium carbonate (27.8 g, 0.201 mol, 1.5 equiv.). The reaction mixture was heated to 80-90 °C and maintained at that temperature for 3 h. After completion of the reaction (monitored by TLC), the mixture was poured into ice cold water (2.0 L, 40.0 V) [Note: Slow quenching with vigorous stirring is preferred to avoid clumping as the product precipitates]. The resulting solids were isolated via filtration and washed with water (150 mL, 3.0 V); then the solids were washed with hexanes (150 mL, 3.0 V). Bulk residual water was removed from the solids by maintaining vacuum filtration for 1-2 h. The solids were dissolved in ethyl acetate (500 mL, 10.0 V) and to the solution was added charcoal (5.0 g). The mixture was heated to 60-70 °C and then stirred at that temperature for 30-45 min. The mixture was filtered while hot (40-50 °C) through a pad of Celite and the Celite pad was extracted with ethyl acetate (250 mL, 5.0 V). The combined filtrate was concentrated to dryness under reduced pressure at below 50 °C. The solids were combined with ethyl acetate (50 mL, 1.0 V) at room temperature. The resulting suspension was stirred for 30 min. The solids were isolated via filtration and then were washed with hexanes (100 mL, 2.0 V). Residual water was removed from the solids by maintaining vacuum filtration for 45-60 min. to afford *N*-(4-chloro-7-nitro-1-(2,2,2-trifluoroethyl)-1*H*-indazol-3-yl)-*N*-(4-methoxybenzyl)methanesulfonamide (56.0 g, 85% yield) as a yellow solid. ¹H NMR (400 MHz, CDCl₃): δ8.12 (d, *J* = 8.36 Hz, 1H), 7.31 (d, *J* = 8.36 Hz, 1H), 7.22 (d, *J* = 8.44 Hz, 2H), 6.77 (d, *J* = 8.44 Hz, 2H), 5.50-5.25 (m, 2H), 4.94-4.79 (m, 2H), 3.75 (s, 3H), 3.02 (s, 3H).

### Step 4: Preparation of N-(7-amino-4-chloro-1-(2,2,2-trifluoroethyl)-1H-indazol-3-yl)-N-(4-methoxybenzyl)methanesulfonamide

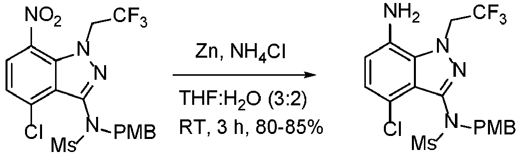

To a stirred suspension of zinc powder (66.31 g, 1.01 mol, 10.0 equiv.) in THF (500 mL, 10.0 V) and water (1.0 L, 20.0 V) at room temperature was added ammonium chloride (54.78 g, 1.01 mol, 10.0 equiv.). To the mixture was added a solution of *N*-(4-chloro-7-nitro-1-(2,2,2-trifluoroethyl)-1*H*-indazol-3-yl)-*N*-(4-methoxybenzyl)methanesulfonamide (50.0 g, 0.101 mol, 1.0 equiv.) in THF (1.0 L, 20.0 V). The reaction mixture was stirred at room temperature for 3 h. After completion of the reaction (monitored by in-process TLC/HPLC), the mixture was diluted with ethyl acetate (1.0 L, 20.0 V) and water (250 mL, 5.0 V). The mixture was stirred for 15 min. The mixture was filtered through a pad of Celite and the Celite pad was extracted with ethyl acetate (250 mL, 5.0 V). The bi-phasic filtrate was partition and the organic layer was reserved while the aqueous layer was extracted with ethyl acetate (500 mL, 10.0 V). The combined organic layers were washed with 10% brine solution (500 mL, 10.0 V), dried over Na₂SO₄, filtered, and then concentrated *in vacuo* to afford a crude solid. To the crude product was added MTBE (250 mL, 5.0 V) and the resulting suspension was stirred for 30 min. at room temperature. The solids were isolated by filtration and then bulk residual water was removed from the solids by maintaining vacuum filtration for 30-45 min. The wet product was dried in a hot air oven (50 °C) for 2 h to afford the title product *N*-(7-amino-4-chloro-1-(2,2,2-trifluoroethyl)-1*H*-indazol-3-yl)-*N*-(4-methoxybenzyl)methanesulfonamide (39.0 g, 83% yield) as off-white solid. ¹H NMR (400 MHz, CDCl₃): δ7.25 (d, *J* = 8.48 Hz, 2H), 6.98 (d, *J* = 7.80 Hz, 1H), 6.79 (d, *J* = 8.48 Hz, 2H), 6.66 (d, *J* = 7.84 Hz, 1H), 5.35-4.75 (m, 4H), 3.77 (s, 3H), 3.56 (bs, 2H), 2.98 (s, 3H).

### Preparation of 2-amino-6-(2-fluorophenyl)nicotinic acid

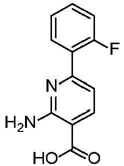

A solution of 2-amino-6-chloronicotinic acid (1.0 g, 5.79 mmol), Pd(dppf)₂Cl₂ (0.21 g, 0.29 mmol), (2-fluorophenyl)boronic acid (1.14 g, 8.11 mmol), and sodium carbonate (1.23 g, 11.59 mmol) in dioxane (46 mL) and water (12 mL) was degassed and heated to 100 °C for 3 h. After cooling to ambient temperature, the reaction mixture was added to water and washed with ether. The aqueous layer was then acidified with 0.5 M citric acid. The aqueous slurry was washed with 10% IPA/DCM (x3). The aqueous layer was then filtered to provide an off-white solid of semipure product of ~80% purity (0.87 g, 65%) which was used in the next reaction without further purification. ¹H NMR (500 MHz, DMSO-d6) δ 8.14 (br d, J=7.94 Hz, 1H), 7.90-7.97 (m, 1H), 7.48-7.54 (m, 1H), 7.31-7.37 (m, 2H), 7.04 (br d, J=8.24 Hz, 1H). LC/MS: m/z = 233.15 [M+1]⁺.

### Preparation of (S)-tert-butyl (1-(3-(4-chloro-3-(N-(4-methoxybenzyl)methylsulfonamido)-1-methyl-1H-indazol-7-yl)-7-(2-fluorophenyl)-4-oxo-3,4-dihydropyrido[2,3-d]pyrimidin-2-yl)-2-(3,5-difluorophenyl)ethyl)carbamate

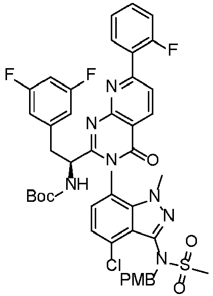

A mixture of (S)-2-((tert-butoxycarbonyl)amino)-3-(3,5-difluorophenyl)propanoic acid (0.908 g, 3.01 mmol), N-(7-amino-4-chloro-1-methyl-1H-indazol-3-yl)-N-(4-methoxybenzyl)methanesulfonamide (1.190 g, 3.01 mmol), and diphenyl phosphite (2.334 mL, 12.06 mmol) in pyridine (6.03 mL) in a pressure vial was heated in an aluminum block for 2 h at 75 °C and cooled to rt. The reaction diluted with EtOAc (~150 mL) and washed with 0.5 M citric acid, dried over Na₂SO₄, and concentrated in vacuo. The residue was purified silica gel flash column chromatography (220 g isco column) using 0-50 % ethyl acetate in hexanes, then 50-70% ethyl acetate in hexanes. The desired fractions were concentrated to give a yellow solid (1.37 g, 52%). LC/MS: m/z = 874.25 [M+1]⁺.

### Preparation of (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-methyl-1H-indazol-3-yl)-N-(4-methoxybenzyl)methanesulfonamide

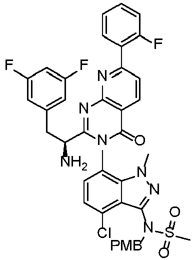

To a solution of tert-butyl (S)-(1-(3-(4-chloro-3-(N-(4-methoxybenryl)methylsulfonamido)-1-methyl-1H-indazol-7-yl)-7-(2-fluorophenyl)-4-oxo-3,4-dihydropyrido[2,3-d]pyrimidin-2-yl)-2-(3,5-difluorophenyl)ethyl)carbamate (1.37 g, 1.567 mmol) in DCM (3.92 mL) was added 4 N HCl in dioxane (3.92 mL, 15.67 mmol). After stirring for 1 h, the resultant pale-yellow suspension was diluted with DCM, washed with saturated aqueous NaHCO3, dried over Na2SO4, and concentrated in vacuo to give a yellow solid (1.16 g, 96%) which was carried on without further purification. LC/MS: m/z = 774.20 [M+1]⁺.

### Preparation of (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-methyl-1H-indazol-3-yl)methanesulfonamide

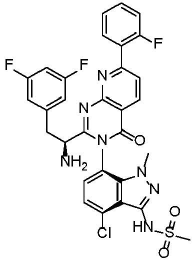

To a solution of (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-methyl-1H-indazol-3-yl)-N-(4-methoxybenzyl)methanesulfonamide (1.17 g, 1.511 mmol) in DCM (7.56 ml) and TFA (4.73 mL) was added TfOH (0.403 mL, 4.53 mmol). The reaction was stirred for 1 h and then concentrated in vacuo. The residue was partitioned between 1 M NaOH and EtOAc. The EtOAc layer was dried (Na₂SO₄) and concentrated in vacuo. The crude mixture of atropisomers was purified on silica gel (120 g isco column) using 20-100% ethyl acetate in hexanes. The desired fractions were concentrated to give an off-white solid. This solid was further purified on a 275 g C18 column using 10-60% 95:5 CH3CN:Water with 0.1% TFA in 95:5 Water:Acetonitrile with 0.1% TFA to separate the atropisomers. The second (major) eluting peak was concentrated (220 mg). The major atropisomer was chirally purified by SFC chromatography on a Chiralpak ID, 25 mm × 250 mm, 5u column, using a A:B gradient, solvent A 80 % heptane, 0.1% TFA solvent B 20% ethanol, 0.1% TFA to provide the desired product (176 mg, 18%, chiral purity 98.2%). LC/MS: m/z = 654.15 [M+1]⁺.

### Preparation of (S)-tert-butyl (1-(3-(4-chloro-1-(2,2-difluoroethyl)-3-(N-(4-methoxybenzyl)methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate

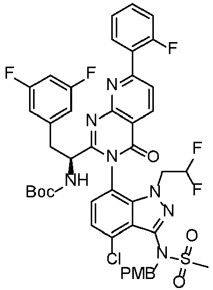

A mixture of 2-amino-6-(2-fluorophenyl)nicotinic acid (1.5 g, 6.46 mmol), (S)-2-((tert-butoxycarbonyl)amino)-3-(3,5-difluorophenyl)propanoic acid (1.752 g, 5.81 mmol), N-(7-amino-4-chloro-1-(2,2-difluoroethyl)-1H-indazol-3-yl)-N-(4-methoxybenzyl)methanesulfonamide (2.59 g, 5.81 mmol) and diphenyl phosphonate (4.95 mL, 25.8 mmol) in pyridine (13 mL) in a pressure vial was stirred for 6 hours at 75 °C. The mixture was cooled to rt and the mixture was diluted with EtOAc (~250 mL). The solution was washed with 0.5 M citric acid, dried over Na₂SO₄, filtered, and concentrated under reduced pressure. The resulting residue was purified by silica gel chromatography (330g RediSep column) eluting with 0-45% ethyl acetate in hexanes over 12 CV. Fractions containing the desired product were pooled and then concentrated under reduced pressure to afford (S)-tert-butyl (1-(3-(4-chloro-1-(2,2-difluoroethyl)-3-(N-(4-methoxybenzyl)methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate as a pale yellow foamy solid, 1.61 g (27% yield), a mixture of four stereoisomers. LC/MS: m/z = 946.15 [M+1]⁺.

### Preparation of (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)-N-(4-methoxybenzyl)methanesulfonamide

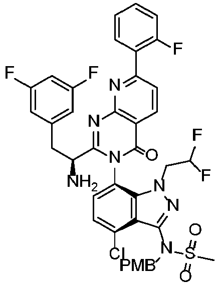

To a solution of tert-butyl (S)-(1-(3-(4-chloro-1-(2,2-difluoroethyl)-3-(N-(4-methoxybenzyl)methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate (1.93 g, 2.088 mmol) in dichloromethane (10.44 mL) was added HCl in dioxane (4M, 10.44 ml, 41.8 mmol). The mixture was stirred at rt for 1 h. The resulting pale yellow solution was concentrated under reduced pressure and the residue was dissolved in ethyl acetate. The solution was washed with sat. aq. NaHCOs, dried over Na₂SO₄, filtered, and concentrated under reduced pressure to afford a yellow solid. This material was purified by silica gel chromatography (220 g RediSep column) eluting with 10-100 % ethyl acetate in hexanes over 25 column volumes. Two peaks contained the desired product mass; fractions of the major peak (first to elute) were pooled and then concentrated to afford a pale yellow solid. The material was subjected to prep-SFC chromatography (to separate enantiomers) using the following method: Column = Chiralpak IB 20×250mm, 5u; Solvent = CO₂:EtOH (65:35); Flow rate = 50 g/min; Back pressure = 100 bar; UV wavelength = 220 nm; Temperature = 30 °C. The major peak was collected and concentrated to afford (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)-N-(4-methoxybenzyl)methanesulfonamide, 1.2 g, (69% yield, 100% chiral purity). LC/MS: m/z = 824.10 [M+1]⁺.

### Preparation of (S)-tert-butyl (1-(3-(4-chloro-1-(2,2-difluoroethyl)-3-(N-(4-methoxybenzyl)cyclopropanesulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate

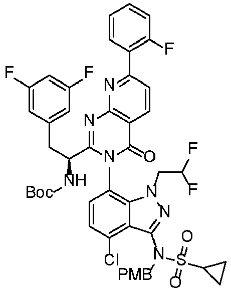

A mixture of 2-amino-6-(2-fluorophenyl)nicotinic acid (0.493 g, 2.124 mmol), (S)-2-((tert-butoxycarbonyl)amino)-3-(3,5-difluorophenyl)propanoic acid (0.640 g, 2.124 mmol), N-(7-amino-4-chloro-1-(2,2-difluoroethyl)-1H-indazol-3-yl)-N-(4-methoxybenzyl)cyclopropanesulfonamide (1 g, 2.124 mmol) and diphenyl phosphonate (1.627 ml, 8.49 mmol) in pyridine (4.25 mL) in a pressure vial was stirred at 75 °C for 8 h. The mixture was cooled to rt and then was diluted with EtOAc, washed with 0.5 M citric acid, dried over Na₂SO₄, filtered, and concentrated under reduced pressure. The resulting residue was purified by silica gel chromagraphy (220 g RediSep column) eluting with 0-40 % ethyl acetate in hexanes over 25 CV. The desired fractions were pooled and concentrated under reduced pressure to afford (S)-tert-butyl (1-(3-(4-chloro-1-(2,2-difluoroethyl)-3-(N-(4-methoxybenzyl)cyclopropanesulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate as a pale yellow foamy solid, 0.97 g (48%), a mixture of four stereoisomers. LC/MS: m/z = 950.10 [M+1]⁺.

### Preparation of (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)-N-(4-methoxybenzyl)cyclopropanesulfonamide

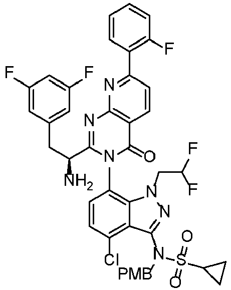

To a solution of tert-butyl (S)-(1-(3-(4-chloro-1-(2,2-difluoroethyl)-3-(N-(4-methoxybenzyl)cyclopropanesulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate (0.971 g, 1.022 mmol) in dichloromethane (5 mL) was added HCl in dioxane (4M, 5.11 mL, 20.43 mmol). The mixture was stirred at rt for 1 h. The resultant pale yellow solution was concentrated under reduced pressure. The residue was dissolved in ethyl acetate and then was washed with sat. aq. NaHCOs, dried over Na₂SO₄, filtered, and concentrated in vacuo afford a yellow solid. This material was purified by silica gel chromatography (220 g RediSep column) eluting with 10-100 % ethyl acetate in hexanes over 25 column volumes. Two peaks contained the desired product mass; fractions of the major peak (first to elute) were pooled and then concentrated to afford a pale yellow solid. This material was subjected to prep-HPLC (chiral) chromatography (to separate enantiomers) using the following method: Column = Chiralcel OD-H (30 mm × 250 mm), 5 microns; Eluent = n-heptane:ethanol (40:60) with 20 mM ammonium acetate; Flow rate = 45 ml/min.; Temperature = ambient temp.; Detection = 260 nm (UV). The major peak was collected and concentrated in vacuo to afford (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)-N-(4-methoxybenzyl)cyclopropanesulfonamide, 0.51 g (59% yield, 100% chiral purity). LC/MS: m/z = 850.10 [M+1]⁺.

### Preparation of (S)-tert-butyl (1-(3-(4-chloro-3-(N-(4-methoxybenzyl)methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate

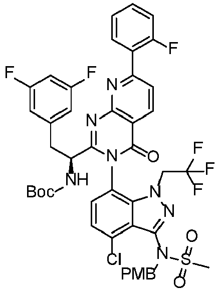

A mixture of 2-amino-6-(2-fluorophenyl)nicotinic acid (1 g, 4.31 mmol), (S)-2-((tert-butoxycarbonyl)amino)-3-(3,5-difluorophenyl)propanoic acid (1.297 g, 4.31 mmol), N-(7-amino-4-chloro-1-(2,2,2-trifluoroethyl)-1H-indazol-3-yl)-N-(4-methoxybenzyl)methanesulfonamide (1.993 g, 4.31 mmol) and diphenyl phosphonate (3.30 mL, 17.23 mmol) in pyridine (12.30 mL) in a pressure vial was stirred at 75 °C for 18 h. The mixture was cooled to rt and then was diluted with EtOAc and washed with aq. 0.5 M citric acid, dried over Na₂SO₄, filtered, and then concentrated under reduced pressure. The resulting residue was purified by silica gel chromatography (80g RediSep column) eluting with 0-45% ethyl acetate in hexanes over 25 CV. The desired fractions were pooled and then concentrated in vacuo to afford tert-butyl (S)-(1-(3-(4-chloro-3-(N-(4-methoxybenzyl)methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate as a pale yellow foamy solid, 1.44 g (36 %), a mixture of four stereoisomers. LC/MS: m/z = 942.10 [M+1]⁺.

### Preparation of (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2,2-trifluoroethyl)-1H-indazol-3-yl)methanesulfonamide

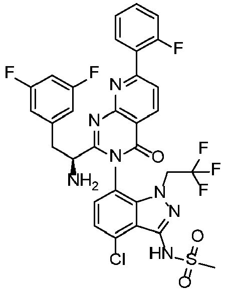

To a solution of tert-butyl (S)-(1-(3-(4-chloro-3-(N-(4-methoxybenryl)methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)carbamate (1.44 g, 1.528 mmol) in dichloromethane (5.0 ml) and trifluoroacetic acid (2.5 mL) was added trifluoromethanesulfonic acid (0.407 mL, 4.58 mmol). The solution was stirred at rt for 1 h and then the pale yellow solution was concentrated in vacuo. The resulting residue was dissolved in ethyl acetate, washed with aq. 1 N NaOH, dried over Na₂SO₄, filtered, and concentrated under reduced pressure to afford a yellow solid. The solid was purified by silica gel chromatography (120 g RediSep Gold column) eluting with 10-100% ethyl acetate in hexanes over 20 CV, then at 100 % ethyl acetate for 5 CV and finally at 12.5% methanol in ethyl acetate for 3 CV. Two peaks contained the desired product mass; fractions of the major peak (first to elute) were pooled and concentrated under reduced pressure to afford a white fluffy solid. This material was further purified by prep-HPLC (chiral, to separate enantiomers) using the following method: Column = Chiralpak IG 20 × 250mm, 5 micron; Eluent = heptane:ethanol (60:40), no modified was used during elution but the column was pre-conditioned with 100 mM NH₄OAc in ethanol; flow-rate = 20 mL/min.; Detection = 254 nm (UV); length of run = 10 min. Two peaks were observed with retention times of 6.5 min and 8.6 min. Fractions corresponding to the major peak were pooled and concentrated to afford (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2,2-trifluoroethyl)-1H-indazol-3-yl)methanesulfonamide, 0.41 g (37% yield, 100% chiral purity). LC/MS: m/z = 722 [M+1]⁺.

### Preparation of tert-butyl (S)-(1-(7-bromo-3-(4-chloro-3-(N-(4-methoxybenzy/)methylsulfonamido)-1-methyl-1H-indazol-7-y/)-4-oxo-3,4-dihydroquinazolin-2-yl)-2-(3,5-difluorophenyl)ethyl)carbamate

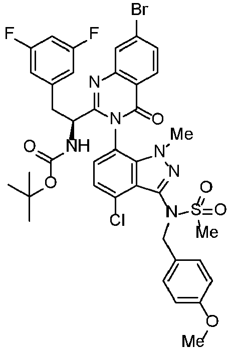

To a solution of (S)-2-((tert-butoxycarbonyl)amino)-3-(3,5-difluorophenyl)propanoic acid (3.82 g, 12.66 mmol), 2-amino-4-bromobenzoic acid (3.01 g, 13.93 mmol) and N-(7-amino-4-chloro-1-methyl-1H-indazol-3-yl)-N-(4-methoxybenzyl)methanesulfonamide (5 g, 12.66 mmol) in pyridine (50 mL) was added diphenyl phosphite (9.80 mL, 50.6 mmol). The resulting mixture was placed on a preheated oil bath (70 °C) and heated at 70 °C for 16 h. The mixture was cooled to room temperature and then concentrated under reduced pressure. The mixture was then diluted with EtOAc (approximately 500 mL) and washed with aqueous citric acid (0.5M, 2 × 50 mL), then aqueous NaOH (1M, 3 × 50 mL), dried over Na₂SO₄, filtered, and concentrated. The residue was then purified via silica gel chromatography (330 g silica gel column, gradient of hexanes:EtOAc 0:100→50:50) to afford tert-butyl (S)-(1-(7-bromo-3-(4-chloro-3-(N-(4-methoxybenryl)methylsulfonamido)-1-methyl-1H-indazol-7-yl)-4-oxo-3,4-dihydroquinazolin-2-yl)-2-(3,5-difluorophenyl)ethyl)carbamate (6.2 g, 7.22 mmol, 57.1 % yield) as pale yellow solid foam (inseparable mixture of atropisomers). LC/MS: m/z = 801.10 [M-tBu].

### Preparation of (S)-N-((6P)-7-(2-(1-amino-2-(3,5-difluorophenyl)ethyl)-7-bromo-4-oxoquinazolin-3(4H)-y/)-4-chloro-1-methyl-1H-indazol-3-y/)methanesulfonamide

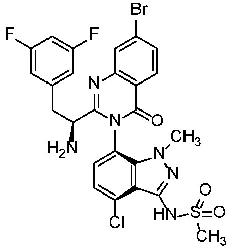

To a stirred solution of tert-butyl (S)-(1-(7-bromo-3-(4-chloro-3-(N-(4-methoxybenzyl)methylsulfonamido)-1-methyl-1H-indazol-7-yl)-4-oxo-3,4-dihydroquinazolin-2-yl)-2-(3,5-difluorophenyl)ethyl)carbamate (6.2 g, 7.22 mmol) in dichloromethane (DCM) (50 mL) was added trifluoroacetic acid (20 mL, 260 mmol) followed by trifluoromethanesulfonic acid (0.770 mL, 8.67 mmol). The resulting dark red solution was stirred at room temperature for 1 h. LCMS at this point indicates two peaks containing the desired product mass, consistent with the presence of two diastereomeric atropisomers (ratio of approximately 30:70). The mixture was concentrated *in vacuo* and the resulting residue was partitioned between EtOAc (300 mL) and aq. NaOH (1M, 30 mL). The aq. phase was tested and determined to be pH >=8.0. The organic phase was isolated and dried over Na₂SO₄, filtered, and then concentrated *in vacuo.* The residue was purified in three approximately equal portions via C18 chromatography (275 g RediSep Gold Column, Mobile Phase A: 5:95 acetonitrile:water with 0.1 % TFA; Mobile Phase B: 95:5 acetonitrile:water with 0.1 % TFA; gradient of 10-60 %B over 30 min). Fractions containing the major atropisomer (second eluting) were combined, adjusted to pH 8 via addition of aq. 1M NaOH; extracted with ethyl acetate; washed with brine (sat. aq. NaCl); dried over Na₂SO₄; filtered; and then concentrated to afford the desired major atropisomer (S)-N-((6P)-7-(2-(1-amino-2-(3,5-difluorophenyl)ethyl)-7-bromo-4-oxoquinazolin-3(4H)-yl)-4-chloro-1-methyl-1H-indazol-3-yl)methanesulfonamide (2.4 g, 3.76 mmol, 52% yield). ¹H NMR (500 MHz, DMSO-d₆) δ ppm 8.11 (d, *J*=8.55 Hz, 1 H), 8.06 (d, *J*=1.53 Hz, 1 H), 7.81 (dd, *J*=8.55, 1.83 Hz, 1 H), 7.33 (s, 2 H), 6.96 - 7.05 (m, 1 H), 6.75 (br d, *J*=7.02 Hz, 2 H), 3.67 (s, 3 H), 3.56 (dd, *J*=7.63, 5.19 Hz, 1 H), 3.25 - 3.29 (m, 1 H), 3.21 (s, 3 H), 2.81 (dd, *J*=13.43, 8.24 Hz, 1 H). LCMS: m/z = 637.05 [M+H]⁺.

### Preparation of N-((S)-1-((3P)-7-bromo-3-(4-chloro-1-methyl-3-(methylsulfonamido)-1H-indazol-7-yl)-4-oxo-3,4-dihydroguinazolin-2-yl)-2-(3,5difluorophenyl)ethyl)-2-((36S,4aR) 3-(difluoromethyl)-5,5-difluoro-3b,4,4a,5-tetrahydro-1H-cyclopropa[3,4]cyclopenta[1,2-c]pyrazol-1-y/)acetamide

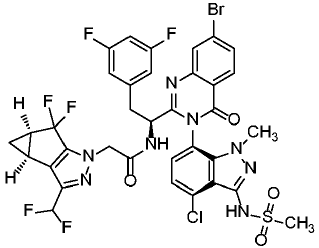

To a solution of (S)-N-((6P)-7-(2-(1-amino-2-(3,5-difluorophenyl)ethyl)-7-bromo-4-oxoquinazolin-3(4H)-yl)-4-chloro-1-methyl-1H-indazol-3-yl)methanesulfonamide (2.08 g, 3.26 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (0.861 g, 3.26 mmol) and diisopropylethylamine ("DIPEA") (1.709 mL, 9.78 mmol) in tetrahydrofuran (THF) (30 mL) was added HATU (1.364 g, 3.59 mmol). The resulting mixture was stirred at room temp for 3 h. To the mixture was added ammonia in methanol (2M, 3 mL). The mixture was stirred at room temp for 30 min. Water was then added and the mixture was extracted with ethyl acetate; washed with brine; dried over Na₂SO₄, filtered; and concentrated *in vacuo.* The resulting residue was subjected to silica gel chromatography (hexanes:EtOAc 100:0→30:70) to afford N-((S)-1-((3P)-7-bromo-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (2.5 g, 2.83 mmol, 87 % yield). ¹H NMR (500 MHz, CDCl₃)δ ppm 8.18 (d, *J*=8.24 Hz, 1 H), 7.88 (d, *J*=1.53 Hz, 1 H), 7.72 (dd, *J*=8.55, 1.83 Hz, 1 H), 7.33 (s, 1 H), 7.16 (d, *J*=7.63 Hz, 1 H), 6.57 - 6.83 (m, 4 H), 6.38 (br d, *J*=5.80 Hz, 2 H), 4.71 - 4.80 (m, 1 H), 4.63 (d, *J*=6.71 Hz, 2 H), 3.56 (s, 3 H), 3.40 (s, 3 H), 3.18 (dd, *J*=13.73, 6.10 Hz, 1 H), 2.86 (dd, *J*=13.58, 7.48 Hz, 1 H), 2.52 - 2.61 (m, 1 H), 2.41 - 2.50 (m, 1 H), 1.42 - 1.50 (m, 1 H), 1.09 - 1.16 (m, 1 H). LCMS: m/z = 883.05 [M+H]⁺.

### Preparation of tert-Butyl (S)-(1-(7-bromo-3-(4-chloro-1-(2,2-difluoroethyl)-3-(N-(4-methoxybenzyl)methylsulfonamido)-1H-indazol-7-yl)-4-oxo-3,4-dihydroquinazolin-2-yl)-2-(3,5-difluorophenyl)ethyl)carbamate

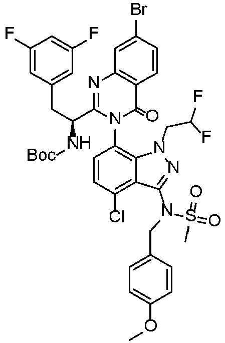

To a stirred solution of (S)-2-((tert-butoxycarbonyl)amino)-3-(3,5-difluorophenyl)propanoic acid (15 g, 49.8 mmol) and 2-amino-4-bromobenzoic acid (10.76 g, 49.8 mmol) in pyridine (150 mL) was added diphenyl phosphite (9.64 mL, 49.8 mmol) at 27 °C. The mixture was flushed with argon and the flask was then sealed. The reaction mixture was heated to 80 °C and stirred at that temperature for 2 hr. The reaction mixture was cooled to 27 °C and to the mixture was added N-(7-amino-4-chloro-1-(2,2-difluoroethyl)-1H-indazol-3-yl)-N-(4-methoxybenzyl)methanesulfonamide. The flask was sealed and the mixture was heated at 80 °C for 16 hr. The progress of the reaction was monitored by TLC (SiO₂, 30% EtOAc/Pet., Rf = 0.4, UV-active). The reaction mixture was allowed to cool to 27 °C and then was concentrated under reduced pressure. The resulting residue was subjected to silca gel column chromatography (Pet.:EtOAc 80:20→70:30) to afford tert-butyl (S)-(1-(7-bromo-3-(4-chloro-1-(2,2-difluoroethyl)-3-(N-(4-methoxybenryl)methylsulfonamido)-1H-indazol-7-yl)-4-oxo-3,4-dihydroquinazolin-2-yl)-2-(3,5-difluorophenyl)ethyl)carbamate as an off-white solid, 18 g (35%). The isolated material is a mixture of stereoisomers. LCMS: M+H = 907.18 and 909.12; purity = 89%.

### Preparation of (S)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide

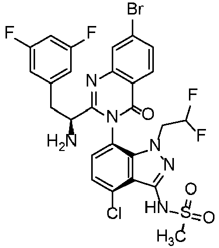

To a stirred solution of tert-butyl (S)-(1-(7-bromo-3-(4-chloro-1-(2,2-difluoroethyl)-3-(N-(4-methoxybenzyl)methylsulfonamido)-1H-indazol-7-yl)-4-oxo-3,4-dihydroquinazolin-2-yl)-2-(3,5-difluorophenyl)ethyl)carbamate (N68085-33-A2, 15 g, 14.70 mmol) in DCM (150 mL) at 27 °C under N₂ atmosphere was added TFA (150 mL, 1947 mmol). The solution was stirred for 10 min. To the reaction mixture was added triflic acid (15 mL, 169 mmol). The solution was stirred for 1 h at 27 °C. The progress of the reaction was monitored by TLC (SiO₂, 5% MeOH/DCM, Rf = 0.4, UV-active). On completion, the solvent was removed under a gentle stream of nitrogen. The residue was dissolved in EtOAc (500 mL), washed with aq saturated NaHCOs (2 × 250 mL), brine (150 mL), dried over Na₂SO₄ and filtered. The filtrate was concentrated under reduced pressure to afford an off-white solid. LCMS analysis of the solid found a 75.42%:21.47% ratio of diastereomers. The crude solid subjected to C18 reverse-phase column chromatography (Mobile Phase: A: 0.1% TFA in water and B: 0.1% TFA in MeCN). Pure fractions containing the major diastereomer (atropisomer) were combined concentrated under reduced pressure. The resulting aqueous solution was made basic via the addition of aq. sat. NaHCO₃; then was extracted with EtOAc (2 × 500 mL). The combined organic layers were washed with brine (200 mL), dried over Na₂SO₄, filtered and concentrated to afford (S)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide as an off-white solid, 8.0 g (76%). LCMS: M+H = 687.34, Purity = 96%. This material was further purified to isolate the major enantiomer as follows: (S)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide (4.5 g, 6.28 mmol) was dissolved in MeOH:MeCN (1:1, 170 mL). The solution was subjected portion-wise to SFC chiral separation as follows: column = (R, R) WHELK-01, 30x250 mm, 5 micron; solvent A = super critical CO₂; solvent B = methanol); eluent composition = 50%A:50%B; flow-rate = 100 g/min; back-pressure = 90 bar; injection volume = 1.1 mL; detection = 214 nm; Stack time = 6.8 min. For each isolated enantiomer, the resulting solution was concentrated under reduced pressure to afford an off-white solid. (*S*)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide as was isolated as the peak eluting from 6 min to 8 min and afforded 2.1 g (48%). ¹H NMR (400 MHz, DMSO-d₆) δ = 8.11-8.05 (m, 2H), 7.83-7.78 (m, 1H), 7.47-7.41 (m, 2H), 7.03-6.97 (m, 1H), 6.76-6.69 (m, 2H), 6.41-6.14 (m, 1H), 4.47-4.22 (m, 2H), 3.54-3.49 (m, 1H), 3.25-3.21 (m, 4H), 2.83-2.76 (m, 1H). LCMS: M+H = 687.04, Purity = 99%, Chiral HPLC Purity = 96%.

### Preparation of N-((S)-1-((3P)-7-Bromo-3-(4-chloro-1-(2,2-difluoroethyl)-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

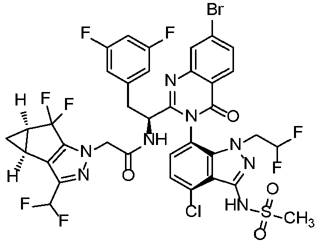

To a solution of (5)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide (1.75 g, 2.52 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (0.739 g, 2.77 mmol), HOBt hydrate (0.424 g, 2.77 mmol) and EDC.HCl (0.579 g, 3.02 mmol) in DMF (15 mL) at 27 °C under nitrogen atmosphere was added N-methylmorpholine (2.215 mL, 20.15 mmol). The solution was stirred at 27 °C for 36 h. The progress of the reaction was monitored by TLC (SiO₂, 50% EtOAc/Pet. Rf = 0.5, UV-active). The reaction mixture was diluted with ice cold water (50 mL), and stirred for 15 min. The precipitated solid was isolated via filtration, washed with water (50 mL), and dried under vacuum to obtain the crude product. This material was treated with EtOAc (20 mL), stirred for 15 min, and then the solids were isolated via filtration and dried under vacuum to afford N-((S)-1-((3P)-7-bromo-3-(4-chloro-1-(2,2-difluoroethyl)-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide as an off-white solid, 1.6g (64%). ¹H NMR (400 MHz, DMSO-d₆) δ = 10.00 (brs, 1H), 9.23 (d, *J* = 8.1 Hz, 1H), 8.13 (d, *J* = 8.6 Hz, 1H), 7.98 (d, *J* = 2.0 Hz, 1H), 7.85 (dd, *J* = 2.0, 2.1 Hz, 1H), 7.78 (d, *J* = 7.9 Hz, 1H), 7.54 (d, *J* = 7.9 Hz, 1H), 7.07-6.99 (m, 1H), 6.92 (t, *J* = 51.7 Hz, 1H), 6.61(d, *J* = 6.3 Hz, 2H), 6.11 (t, *J* = 54.6 Hz, 1H), 4.72-4.57 (m, 2H), 4.38 (tt, *J* = 107, 2.9 Hz, 1H), 4.31-4.19 (m, 1H), 3.96-3.83 (m, 1H), 3.44-3.37 (m, 1H), 3.19 (s, 3H), 3.00-2.92 (m, 1H), 2.49-2.45 (m, 2H), 1.39-1.31 (m, 1H), 0.87-0.82 (m, 1H). LCMS: M+H = 933.13, LCMS Purity = 95%, HPLC Purity = 96%, Chiral HPLC Purity = 97%.

### Preparation of tert-butyl (S)-(1-(7-bromo-3-(4-chloro-1-(2,2-difluoroethyl)-3-(N-(4-methoxybenzyl) cyclopropanesulfonamido)-1H-indazol-7-yl)-4-oxo-3,4-dihydroquinazolin-2-yl)-2-(3,5-difluorophenyl)ethyl)carbamate

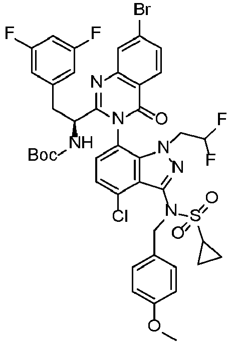

To a stirred solution of (S)-2-((tert-butoxycarbonyl)amino)-3-(3,5-difluorophenyl)propanoic acid (15 g, 49.8 mmol) and 2-amino-4-bromobenzoic acid (12.91 g, 59.7 mmol) in pyridine (150 mL) in a sealed tube at 26 °C was added diphenyl phosphite (35.7 mL, 184 mmol). The reaction mixture was degassed with N₂ bubbling for each addition of reagents. The reaction mixture was heated to 80 °C and stirred for 2 hr. The reaction mixture was cooled to 26 °C, then N-(7-amino-4-chloro-1-(2,2-difluoroethyl)-1H-indazol-3-yl)-N-(4-methoxybenzyl)cyclopropanesulfonamide (N66734-90-A2, 20.49 g, 34.9 mmol) was added. The mixture was heated at 80 °C for 16 h. The progress of the reaction was monitored by TLC (SiOz, 30% EtOAc/Pet. Rf = 0.3). The reaction mixture was cooled to 26 °C and then was concentrated under reduced pressure. The residue was diluted with water (150 mL) and extracted with ethyl acetate (2 × 500 mL). The combined organic layers were washed with aq. citric acid (5% w/v, 2 × 150 mL), then brine (250 mL); dried over anhydrous Na₂SO₄; filtered; and concentrated under reduced pressure to afford a brown gummy liquid (40 g). The above procedure was repeated, and the crude product of both iterations was combined. This material was then subjected to silica gel column chromatography (pet.:EtOAc, 60:40→55:45) to afforded tert-butyl (S)-(1-(7-bromo-3-(4-chloro-1-(2,2-difluoroethyl)-3-(N-(4-methoxybenzyl)cyclopropanesulfonamido)-1H-indazol-7-yl)-4-oxo-3,4-dihydroquinazolin-2-yl)-2-(3,5-difluorophenyl)ethyl)carbamate (mixture of diastereomers) as a yellow solid (42 g, 98%). LCMS: M+H = 933.88 & 935.88; purity = 76.91%.

### Preparation of (S)-N-((6P)-7-(2-(1-amino-2-(3,5-difluorophenyl)ethyl)-7-bromo-4-oxoguinazolin-3(4H)yl)-4-chloro-1-(2,2-difluoroethyl)-1H-indazol-3-yl)cyclopropanesulfonamide

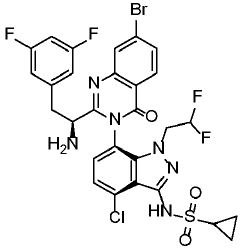

To a stirred solution of tert-butyl (S)-(1-(7-bromo-3-(4-chloro-1-(2,2-difluoroethyl)-3-(N-(4-methoxybenzyl)cyclopropanesulfonamido)-1H-indazol-7-yl)-4-oxo-3,4-dihydroquinazolin-2-yl)-2-(3,5-difluorophenyl)ethyl)carbamate (14 g, 11.53 mmol) in DCM (140 mL) at 27 °C under N2 atmosphere was added TFA (140 mL). The solution was stirred for 10 min. To the solution was added trifluoromethanesulfonic acid (7.16 mL, 81 mmol). The reaction mixture was stirred for 1 h at 27°C. The progress of the reaction was monitored by TLC (SiO₂, 50% EtOAc/pet, Rf = 0.2). The solvent was removed under a gentle stream of nitrogen. The residue was dissolved in EtOAc (500 mL) and the organic layer was washed with aq. saturated NaHCO₃ (2 × 150 mL), brine (50 mL), dried over Na₂SO₄, filtered and concentrated to dryness to the crude compound as an off white solid (12 g). The above procedure was repeated twice more and the additional crude solids (2 × 14 g) were combined with the above. The combined material was dissolved in dichloromethane (500 mL) and concentrated to afford a homogeneous crude solid. This material was washed with pet. ether: EtOAc (80:20) and then dried under vacuum to afford a brown solid (30 g). This material was then subjected to C18 reverse phase chromatography under the following conditions: Column = RediSep Gold HP C18 275 g; Mobile Phase A = Water:MeCN:TFA (950:50:1); Mobile Phase B = Water:MeCN:TFA (50:950:1); flow rate = 80 mL/min; gradient profile (time/%B) = 5/5, 5/10, 5/15, 10/20, 15/30, 20/40, 15/45, 10/50; temperature = ambient. Fractions of the major peak were pooled and concentrated under reduced pressure to remove the non-aqueous solvent. The resulting aq. solution was neutralized via the addition of sat. aq. NaHCO3 (1000 mL), then was extracted with EtOAc (4 × 500 mL). The combined organics were washed with brine (500 mL), dried over anhydrous Na₂SO₄, filtered and concentrated to afford (S)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (single diastereomer) as an off white solid. The material was then subjected to SFC purification under the following conditions: Column/dimensions = Chiralpak OX-H (30x250 mm), 5µ; Solvent A = liquid COz; Solvent B = Methanol with 0.5% diethyl amine; Eluent = A:B (70:30); Flow-rate = 100.0 g/min; Back Pressure = 100.0 bar; Detection = UV (214 nm); injection volume = 1.3 mL (93 mg/injection); 160 injections. Two peaks were collected separately and the major peak was concentrated under reduced pressure to afford (S)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (single stereoisomer) as a pale yellow solid, 7.5 g (20%). ¹H NMR (400 MHz, DMSO-d₆) δ = 8.11 - 8.04 (m, 2H), 7.82-7.78 (m, 1H), 7.47 - 7.39 (m, 2H), 7.02 - 6.95 (m, 1H), 6.76-6.69 (m, 2H), 6.38 - 6.19 (m, 1H), 4.48 - 4.37 (m, 1H), 4.32 - 4.24 (m, 1H), 3.54 - 3.48 (m, 1H), 3.3 -3.20 (m, 1 H), 2.97 - 2.90 (m, 1H), 2.83 - 2.76 (m, 1H), 1.05 - 0.99 (m, 4H). LCMS: M+H = 712.94 and 714.94; purity = 98.37%, chiral HPLC purity = 96 %.

### Preparation of N-((S)-1-((3P)-7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-olifluoro-3b,4,4a,5-tetrahydro-1H-cyclopropa[3,4]cyclopenta[1,2-c]pyrazol-1-y/)acetamide

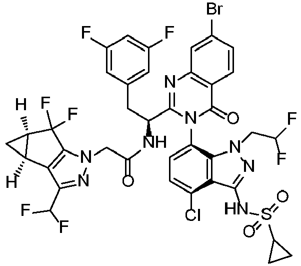

To a stirred solution of (*S*)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (500 mg, 0.700 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (N68084-15-A1, 185 mg, 0.700 mmol), and HOBt (42.9 mg, 0.280 mmol) in DMF (5 mL) at 27 °C was added N-methylmorpholine (0.308 mL, 2.80 mmol) and N-(3-dimethylaminopropyl)-N'-ethylcarbodiimide hydrochloride (242 mg, 1.261 mmol). The reaction mixture was stirred at 27 °C for 16 h. The progress of the reaction was monitored by TLC (SiO₂, 50% EtOAc/Pet., Rf = 0.3, UV-active). On completion, the reaction mixture was diluted with ice cold water (70 mL) and then stirred for 15 min at 27 °C. The precipitated solids were collected by filtration and then dried under vacuum to obtain the crude compound as an off-white solid. The crude compound was subjected to silica gel chromatography (pet.:EtOAc (98:2→50:50) to afford N-((S)-1-((3P)-7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide as an off-white solid, 550 mg (80%). ¹H NMR (400 MHz, DMSO-d₆) *δ* = 9.99 (s, 1H), 9.24 (d, *J=* 8.1 Hz, 1H), 8.13 (d, *J=* 8.8 Hz, 1H), 7.97 (d, *J=* 1.8 Hz, 1H), 7.87-7.83 (m, 1H), 7.77 (d, *J=* 7.9 Hz, 1H), 7.54 (d, *J=* 7.9 Hz, 1H), 7.06-6.79 (m, 2H), 6.64-6.58 (m, 2H), 6.23-5.98 (m, 1H), 4.74-4.57 (m, 2H), 4.41-4.35 (m, 1H), 4.29-4.16 (m, 1H), 3.94-3.84 (m, 1H), 3.38-3.34 (m, 1H), 3.02-2.93 (m, 1H), 2.90-2.83 (m, 1H), 2.48-2.35 (m, 2H), 1.37-1.30 (m, 1H), 1.02-0.90 (m, 4H), 0.87-0.82 (m, 1H). LCMS analysis method F: RT = 6.74 mins, (M+H) = 959.0 and 961.0; LCMS Purity = 98%; Chiral HPLC Purity = 98%.

### Preparation of 1-cyclopropyl-4,4-difluorobutane-1,3-dione

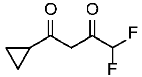

To a stirred solution of 1-cyclopropylethan-1-one (20 g, 238 mmol) in diethyl ether (2000 mL) under N₂ atmosphere at -78 °C was slowly added NaHMDS (119 mL, 238 mmol) over a period of 20 min. The solution was then stirred for 45 min at -78 °C. To the solution was added ethyl 2,2-difluoroacetate (75 mL, 713 mmol). The reaction mixture was slowly warmed to 27 °C and then stirred for 16 h. After completion, the reaction mixture was quenched with water (80 mL) and washed with diethyl ether (100 mL). The aqueous layer was acidified with aq. HCl (1N, 20 mL) and extracted with diethyl ether (2 × 100 mL). The combined organic layers were washed with brine (50 mL), dried over Na₂SO₄, filtered and concentrated under reduced pressure to afford 1-cyclopropyl-4,4-difluorobutane-1,3-dione as pale-yellow oil 25 g (65%). ¹H NMR (400 MHz, CDCl₃) δ = 6.07 - 5.87 (m, 2H), 1.84 - 1.75 (m, 1H), 1.28 - 1.19 (m, 2H), 1.10 - 1.05 (m, 2H).

### Preparation of 5-cyclopropyl-3-(difluoromethyl)-1H-pyrazole

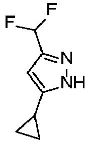

To a stirred solution of 1-cyclopropyl-4,4-difluorobutane-1,3-dione (25 g, 154 mmol) in ethanol (250 mL) at 27 °C was added hydrazine.HzO (16.13 mL, 385 mmol) followed by dropwise addition of hydrochloric acid (0.18 mL, 5.92 mmol). The reaction mixture was heated to 80 °C and stirred at that temperature for 6 h. The reaction was monitored by TLC (50% EtOAc in pet ether; RF: 0.2; Detection: KMnO₄ active). After completion, the reaction mixture was concentrated under reduced pressure. The resulting residue was dissolved in water (250 mL) and extracted with ethyl acetate (3 × 200 mL). The combined organic layers were washed with brine (50 mL), dried over Na₂SO₄, filtered and concentrated under reduced pressure to afford 5-cyclopropyl-3-(difluoromethyl)-1H-pyrazole as a yellow liquid 20 g (79%).¹H NMR (400 MHz, CDCl₃) δ = 6.79 - 6.49 (m, 1H), 6.24 - 6.08 (m, 1H), 1.96 - 1.82 (m, 1H), 1.09 - 0.91 (m, 2H), 0.79 - 0.56 (m, 2H) LCMS: M+H = 159.11, purity = 96.91%.

### Preparation of ethyl 2-(5-cyclopropyl-3-(difluoromethyl)-1H-pyrazol-1-yl) acetate:

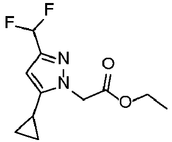

To a stirred solution of 5-cyclopropyl-3-(difluoromethyl)-1H-pyrazole (20 g, 123 mmol) in acetonitrile (200 mL) at 27 °C under N₂ atmosphere was added DIPEA (53.5 mL, 306 mmol) followed by ethyl bromoacetate (27.3 mL, 245 mmol). The reaction mixture was stirred at 65 °C for 48 hr. The progress of the reaction was monitored by TLC (SiO₂, Mobile phase: 30% ethyl acetate in pet ether; RF: 0.5 and KMnO₄ active). After completion, the reaction mixture was diluted with water (20 mL) and extracted with ethyl acetate (2 × 500mL). The combined organic layers were washed with brine solution (500 mL) and dried over anhydrous Na₂SO₄, filtered and concentrated under vacuum to afford the crude compound as brown oil (30 g). This material was subjected to silica gel chromatography (pet.:EtOAc 80:20→70:30) to afford ethyl 2-(5-cyclopropyl-3-(difluoromethyl)-1H-pyrazol-1-yl)acetate as a mixture of regioisomers, 25 g. The material was further purified by HPLC using the following conditions: Column = KROMOSIL PHENYL, 25 × 150 mm, 10 µm; Mobile phase A: 10 mM ammonium bicarbonate in water; Mobile phase B: acetonitrile; flow rate = 25 mL/min; temperature = ambient; Gradient (minute/%B) = 0/10, 2/10, 10/30, 15/30, 15.2/100, 18/100, 18.2/10. Fractions containing the desired product were pooled and then concentrated under reduced pressure to afford an aqueous mixture. This mixture was extracted with ethyl acetate (3 × 100 mL). The combined organics were dried over anhydrous Na₂SO₄, filtered, and concentrated under reduced pressure to afford ethyl 2-(5-cyclopropyl-3-(difluoromethyl)-1H-pyrazol-1-yl) acetate as a pale yellow oil, 2.1 g (24%).¹H NMR (400 MHz, DMSO-d₆) δ = 7.05 - 6.69 (m, 1H), 6.24 - 6.14 (m, 1H), 5.21 - 5.10 (m, 2H), 4.21 - 4.09 (m, 2H), 1.92 - 1.76 (m, 1H), 1.27 - 1.13 (m, 3H), 0.98 - 0.86 (m, 2H), 0.70 - 0.56 (m, 2H). LCMS: M+H = 245.31, purity = 98.89%.

### Preparation of 2-(5-cyclopropyl-3-(difluoromethyl)-1H-pyrazol-1-yl) acetic acid

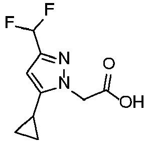

To a stirred solution of ethyl 2-(5-cyclopropyl-3-(difluoromethyl)-1H-pyrazol-1-yl)acetate (2.1 g, 8.60 mmol) in THF:methanol (5 mL:2 mL) at 27 °C was added a solution of LiOH (1.647 g, 68.8 mmol ) in water (2 mL). The reaction mixture was stirred at 27 °C for 16 hr. The progress of the reaction was monitored by TLC (SiO₂, ethyl acetate; Rf: 0.1, UV inactive and KMnO₄ active). After completion, the reaction mixture was concentrated under reduced pressure. The resulting aqueous mixture was diluted with water (50 mL) and then washed with ethyl acetate (3 × 50 mL). The aqueous layer was cooled to 0 °C and then adjusted to pH 2 via addition of aq. HCl (2N). The precipitated solid was collected via filtration and then dried under vacuum to afford 2-(5-cyclopropyl-3-(difluoromethyl)-1H-pyrazol-1-yl) acetic acid as an off white solid, 1.3 g (70%). ¹H NMR (400 MHz, DMSO-d₆) δ = 13.27 - 13.10 (m, 1H), 7.02 - 6.72 (m, 1H), 6.21 - 6.10 (m, 1H), 5.08 - 4.93 (m, 2H), 1.86 - 1.77 (m, 1H), 0.97 - 0.87 (m, 2H), 0.71 - 0.58 (m, 2H ). LCMS: M+H = 217.20, purity = 99.52%.

### Preparation of N-((S)-1-((3P)-3-(4-chloro-3-(cyclopropanesulfonamido)-1-(2,2-dif/uoroethy/)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

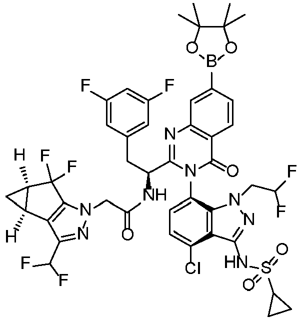

To a dry r.b. flask equipped with a stir bar was added N-((S)-1-((3P)-7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (300 mg, 0.312 mmol), 4,4,4',4',5,5,5',5'-octamethyl-2,2'-bi(1,3,2-dioxaborolane) (119 mg, 0.469 mmol), potassium acetate (92 mg, 0.937 mmol) and PdCl₂(dppf) (22.86 mg, 0.031 mmol). The flask was sealed with a septum and then placed under argon atmosphere (vac/fill × 3). To the flask was added dioxane (6.3 mL). The flask was again placed under argon atmosphere (vac/fill × 3). The resulting mixture was stirred at 60 °C for 16 h overnight. Upon cooling to ambient temperature the reaction was concentrated *in vacuo* and the resulting residue was adsorbed onto Celite. The resulting powder was subjected to silica gel column chromatography (hexanes:EtOAc 100:0→0:100 over 10 CV) to afford N-((S)-1-((3P)-3-(4-chloro-3-(cyclopropanesulfonamido)-1-(2,2-difluoroethyl)-1H-indazol-7-yl)-4-oxo-7-(4,4,5,5-tetra methyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide, 220 mg (70%). ¹H NMR (METHANOL-d₄, 500 MHz) δ 8.27 (d, 2H, J=6.2 Hz), 8.26 (s, 1H), 7.97 (dd, 1H, J=1.0, 7.9 Hz), 7.41 (d, 1H, *J*=*7.7* Hz), 7.29 (d, 1H, *J=7.7* Hz), 6.8-6.8 (m, 1H), 6.70 (br t, 1H, J=54.8 Hz), 6.55 (dd, 2H, J--2.1, 8.0 Hz), 6.01 (t, 1H, J=55.3 Hz), 4.74 (dd, 1H, *J*=4.8, 9.5 Hz), 4.68 (d, 1H, *J*=16.4 Hz), 4.59 (d, 1H, *J*=16.4 Hz), 4.38 (dd, 1H, *J*=4.2, 15.2 Hz), 4.12 (q, 1H, *J*=7.2 Hz), 3.91 (dd, 1H, *J*=3.9, 15.2 Hz), 3.68 (s, 1H), 3.06 (dd, 1H, *J*=9.4, 14.2 Hz), 2.9-2.9 (m, 1H), 2.4-2.5 (m, 2H), 2.03 (s, 2H), 1.45 (s, 12H), 1.1-1.1 (m, 2H), 1.0-1.0 (m, 3H).

### Preparation of (S)-N-(1-((3P)-7bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide

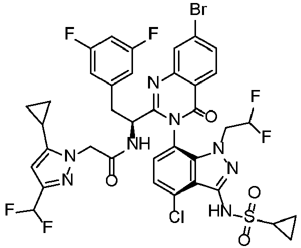

To a solution of (S)-N-((6P)-7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (600 mg, 0.826 mmol), 2-(3-cyclopropyl-5-(difluoromethyl)-1H-pyrazol-1-yl)acetic acid (179 mg, 0.826 mmol) and HOBt (50.6 mg, 0.330 mmol) in DMF (5 mL) at 27 °C was added N-methylmorpholine (0.363 mL, 3.30 mmol) and N-(3-dimethylaminopropyl)-N'-ethylcarbodiimide hydrochloride (285 mg, 1.487 mmol). Then the reaction mixture was degassed for 10 min with nitrogen gas and then stirred at 27 °C for 16 h. The progress of the reaction was monitored by TLC (SiO₂, 50% EtOAc/Pet. Rf = 0.3). The reaction mixture was diluted with ice cold water (70 mL) and then was stirred for 30 min at 27 °C. The precipitated solid was isolated via filtration and then dried under vacuum to afford (S)-N-(1-((3P)-7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide as a pale yellow solid, 550 mg (68%). ¹H NMR (400 MHz, DMSO-d₆) δ = 10.02 - 9.85 (m, 1H), 9.17 - 9.10 (m, 1H), 8.14 (d, *J=* 8.3 Hz, 1H), 7.99 - 7.95 (m, 1H), 7.87 - 7.84 (m, 1H), 7.77 (d, *J* = 7.9 Hz, 1H), 7.52 (d, *J* = 7.9 Hz, 1H), 7.07 - 7.00 (m, 1H), 6.86 - 6.59 (m, 3H), 6.20 - 5.98 (m, 2H), 4.77 - 4.67 (m, 2H), 4.50 - 4.43 (m, 1H), 4.33 - 4.22 (m, 1H), 4.00 - 3.87 (m, 1H), 3.39 - 3.32 (m, 1H), 3.06 - 2.94 (m, 2H), 2.60 - 2.55 (m, 1H), 1.46 - 1.38 (m, 1H), 1.00 - 0.91 (m, 4H), 0.75 - 0.64 (m, 2H), 0.57 - 0.46 (m, 2H ). LCMS: M+H = 910.89 and 912.91; purity = 93.59%.

### Preparation of (S)-N-(1-((3P)-3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide

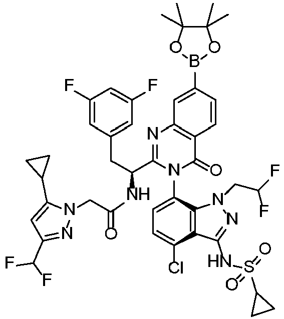

The title compound, (S)-N-(1-((3P)-3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide, was prepared following the procedure used to prepare N-((S)-1-((3P)-3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide but starting from (S)-N-(1-((3P)-7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide.

### Preparation of N-((S)-1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamid

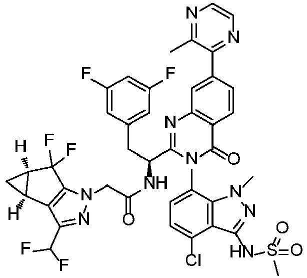

The title compound was prepared according to General Procedure B using 2-chloro-3-methylpyrazine as the coupling partner. The experiment afforded the title compound, N-((S)-1-(3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.3 min.; observed ion = 897.1 (M+H). 1H NMR (METHANOL-d4, 500 MHz) δ 8.65 (s, 1H), 8.62 (d, 1H, J=2.5 Hz), 8.44 (dd, 1H, J=0.6, 8.2 Hz), 8.12 (dd, 1H, J=0.6, 1.6 Hz), 7.91 (dd, 1H, J=1.6, 8.2 Hz), 7.33 (d, 1H, J=7.9 Hz), 7.24 (d, 1H, J=7.9 Hz), 6.8-6.8 (m, 1H), 6.64 (dd, 2H, J=2.2, 8.2 Hz), 6.69 (br t, 2H, J=54.7 Hz), 4.5-4.6 (m, 2H), 3.66 (s, 3H), 3.5-3.5 (m, 1H), 3.2-3.3 (m, 3H), 3.12 (dd, 1H, J=9.3, 14.0 Hz), 2.72 (s, 3H), 2.4-2.5 (m, 2H), 1.3-1.4 (m, 1H), 1.0-1.0 (m, 1H)

### Preparation of N-((S)-1-((3P)-3-(4-chloro-1-(2,2-difluoroethyl)-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

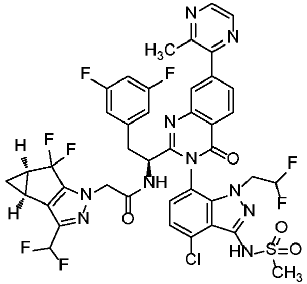

The title compound was prepared according to General Procedure D using 2-chloro-3-methylpyrazine as the coupling partner. The experiment afforded the title compound, N-((S)-1-(3-(4-chloro-1-(2,2-difluoroethyl)-3-(methylsulfonamido)-1H-indazol-7-yl)-7-(3-methyl pyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.32 min.; observed ion = 947.2 (M+H). 1H NMR (METHANOL-d4, 500 MHz) δ 8.65 (s, 1H), 8.63 (d, 1H, J=2.5 Hz), 8.43 (d, 1H, J=7.7 Hz), 8.12 (d, 1H, J=2.1 Hz), 7.91 (dd, 1H, J=1.8, 8.3 Hz), 7.40 (d, 1H, J=8.0 Hz), 7.31 (d, 1H, J=7.7 Hz), 6.6-6.8 (m, 4H), 5.9-6.2 (m, 1H), 4.77 (dd, 1H, J=4.8, 9.2 Hz), 4.62 (q, 2H, J=16.4 Hz), 4.40 (br dd, 1H, 1=4.2, 15.2 Hz), 3.9-4.0 (m, 1H), 3.4-3.5 (m, 1H), 3.3-3.3 (m, 3H), 3.09 (dd, 1H, J=9.4, 14.2 Hz), 2.73 (s, 3H), 2.4-2.5 (m, 2H), 1.3-1.4 (m, 1H), 1.0-1.0 (m, 1H)

### Preparation of N-((S)-1-((3P)-3-(4-chloro-1-methyl-3-(methylsulfonamido)-1H-indazol-7-yl)-7-(4-fluoro-2-methylphenyl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

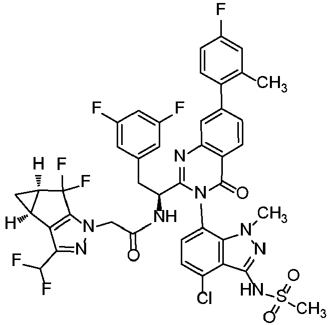

The title compound was prepared according to General Procedure T using (4-fluoro-2-methylphenyl)boronic acid as the coupling partner. The experiment afforded the title compound, N-((S)-1-(3-(4-chloro-1-methyl-3-(methylsulfonamido)-1H-indazol-7-yl)-7-(4-fluoro-2-methylphenyl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. 1H NMR (400 MHz, DMSO-d6) δ = 9.87 - 9.78 (m, 1H), 9.16 - 9.12 (m, 1H), 8.29 - 8.25 (m, 1H), 7.73 - 7.69 (m, 2H), 7.64 - 7.61 (m, 1H), 7.44 - 7.36 (m, 2H), 7.30 - 7.25 (m, 1H), 7.22 - 7.17 (m, 1H), 7.05 - 6.90 (m, 2H), 6.69 - 6.63 (m, 2H), 4.68 - 4.62 (m, 1H), 4.56 - 4.49 (m, 2H), 3.54 (s, 3H), 3.45 - 3.40 (m, 1H), 3.21 - 3.15 (m, 3H), 3.06 - 2.99 (m, 1H), 2.47 - 2.42 (m, 2H), 2.33 (s, 3H), 1.37 - 1.32 (m, 1H), 0.87 - 0.82 (m, 1H).

### Preparation of (S)-N-((6P)-7-(2-(1-amino-2-(3,5-difluorophenyl)ethyl)-4-oxo-7-(4,4,5,5-tetramethyl-1,3,2-dioxaborolan-2-yl)quinazolin-3(4H)-yl)-4-chloro-1-methyl-1H-indazol-3-yl)methanesulfonamide

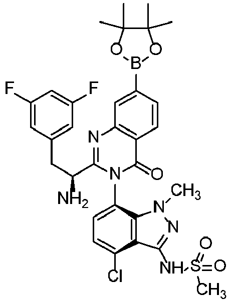

A reaction vessel was charged with (S)-N-((6P)-7-(2-(1-amino-2-(3,5-difluorophenyl)ethyl)-7-bromo-4-oxoquinazolin-3(4H)-yl)-4-chloro-1-methyl-1H-indazol-3-yl)methanesulfonamide (500 mg, 0.784 mmol), 4,4,4',4',5,5,5',5'-octamethyl-2,2'-bi(1,3,2-dioxaborolane) (299 mg, 1.18 mmol), potassium acetate (231 mg, 2.35 mmol) and PdCl₂(dppf) (57 mg, 0.078 mmol). The vessel was sealed and then placed under Ar atmosphere (vacuum evacuation followed by refill with argon, repeated three times). To the flask was added dioxane (16 ml), and the resulting mixture was degassed (brief vacuum evacuation followed by refill with argon, repeated three times). The mixture was stirred at 60 °C for 16 h. The mixture was concentrated and the resulting residue was adsorbed on Celite. The resulting powder was subjected to silica gel chromatography eluting with 0-100% ethyl acetate in hexanes over 10 CVs. The fractions containing the product were pooled and concentrated in vacuo to afford (S)-N-((6P)-7-(2-(1-amino-2-(3,5-difluorophenyl)ethyl)-4-oxo-7-(4,4,5,5-tetramethyl-1,3,2-dioxaborolan-2-yl)quinazolin-3(4H)-yl)-4-chloro-1-methyl-1H-indazol-3-yl)methanesulfonamide (365 mg, 0.533 mmol, 68.0 % yield). ¹H NMR (METHANOL-d₄, 500 MHz) δ 8.2-8.3 (m, 2H), 7.95 (d, 1H, *J=7.8* Hz), 7.30 (d, 1H, *J=8.0* Hz), 6.96 (d, 1H, *J*=7.5 Hz), 6.8-6.8 (m, 1H), 6.60 (d, 2H, *J*=7.4 Hz), 3.75 (s, 3H), 3.4-3.4 (m, 1H), 3.30 (s, 3H), 2.94 (d, 2H, *J*=7.5 Hz), 1.3-1.3 (m, 12H). The sample was analyzed using the following LCMS method: Column = Acquity UPLC BEH C18, 2.1 × 50 mm, 1.7 µm particles; Solvent A = 95:5 Water:MeCN w/ 0.1% TFA; Solvent B = 5:95 Water:MeCN w/ 0.1% TFA; Flow rate = 0.80 mL/min.; Start % B = 0, End % B = 100, Gradient time = 1.50 min. followed by a 1.0 min hold at 100 % B; Detection = 220 nm and 254 nm. LCMS retention time = 1.018 min; observed ion = 603.10 (M+H). Note, the boronic acid was also detected in the LCMS trace but is attributed to decomposition/hydrolysis during the LCMS analysis.

### Preparation of (S)-N-((6P)-7-(2-(1-amino-2-(3,5-difluorophenyl)ethyl)-7-(4,6-dimethylpyrimidin-2-yl)-4-oxoquinazolin-3(4H)-yl)-4-chloro-1-methyl-1H-indazol-3-yl)methanesulfonamide

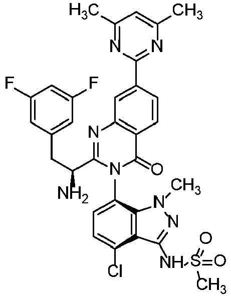

To a reaction vessel equipped with a stir bar were added Dicyclohexyl(2',6'-dimethoxy-[1,1'-biphenyl]-2-yl)phosphane (54 mg, 0.13 mmol), Pd(OAc)2 (15 mg, 0.066 mmol), tribasic potassium phosphate (418 mg, 1.97 mmol), (S)-N-(7-(2-(1-amino-2-(3,5-difluorophenyl)ethyl)-4-oxo-7-(4,4,5,5-tetramethyl-1,3,2-dioxaborolan-2-yl)quinazolin-3(4H)-yl)-4-chloro-1-methyl-1H-indazol-3-yl)methanesulfonamide (450 mg, 0.657 mmol) and 2-chloro-4,6-dimethylpyrimidine (281 mg, 1.97 mmol). The vessel was sealed with a septum cap and then placed under Ar atmosphere (vacuum evacuation followed by refill with argon, repeated three times). To the vessel was added THF (5.2 ml) and Water (1.3 ml), and the mixture was degassed (brief vacuum evacuation followed by refill with argon, repeated three times). The mixture was stirred at 60 °C for 12 hr. The reaction mixture was concentrated in vacuo and the residue was adsorbed onto Celite and then subjected to silica gel chromatography eluting with 0-100% methanol in DCM over 10 CVs to afford (S)-N-((6P)-7-(2-(1-amino-2-(3,5-difluorophenyl)ethyl)-7-(4,6-dimethylpyrimidin-2-yl)-4-oxoquinazolin-3(4H)-yl)-4-chloro-1-methyl-1H-indazol-3-yl)methanesulfonamide (403 mg, 0.454 mmol, 69 % yield). Several impurities co-eluted with the product; the material was used in downstream chemistry without additional purification. The LCMS method was: Column = Acquity UPLC BEH C18, 2.1 × 100 mm, 1.7 µm particles; Solvent A = 0.1% TFA in 95:5 Water:MeCN; Solvent B = 0.1% TFA in 5:95 Water:MeCN; Flow Rate = 0.8 mL/min.; Start % B = 0, Final % B = 100; Gradient Time = 3.5 min, then a 1 min hold at 100% B. Detection = 220 and 254 nm. LCMS retention time = 2.365 min.; observed ion = 665.10 (M+H).

### Preparation of (S)-N-((6P)-7-(2-(1-amino-2-(3,5-difluorophenyl)ethyl)-7-(3-methylpyrazin-2-yl)-4-oxoquinazolin-3(4H)-yl)-4-chloro-1-methyl-1H-indazol-3-yl)methanesulfonamide

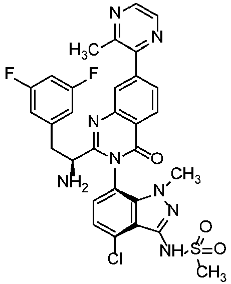

A 5 mL microwave vial equipped with a stir bar was charged with xantphos PdCl₂ (39 mg, 0.051 mmol), tribasic potassium phosphate (325 mg, 1.53 mmol), (S)-N-((6P)-7-(2-(1-amino-2-(3,5-difluorophenyl)ethyl)-4-oxo-7-(4,4,5,5-tetramethyl-1,3,2-dioxaborolan-2-yl)quinazolin-3(4H)-yl)-4-chloro-1-methyl-1H-indazol-3-yl)methanesulfonamide (350 mg, 0.511 mmol) and 2-chloro-3-methylpyrazine (197 mg, 1.53 mmol). The vial was sealed with a setum cap and then placed under Ar atmosphere (vacuum evacuation followed by refill with argon, repeated three times). To the vial was added THF (4 ml) and Water (1 ml). The mixture was degassed (brief vacuum evacuation followed by refill with argon, repeated three times). The mixture was stirred at 45°C for 5 hr. The mixture was concentrated in vacuo and the resulting residue was purified by silica chromatography using a gradient of 0-100% ethyl acetate in hexanes over 10 CVs followed by 100 % methanol. The desired product eluted during the 100% methanol elution of the column to afford (S)-N-((6P)-7-(2-(1-amino-2-(3,5-difluorophenyl)ethyl)-7-(3-methylpyrazin-2-yl)-4-oxoquinazolin-3(4H)-yl)-4-chloro-1-methyl-1H-indazol-3-yl)methanesulfonamide (200 mg, 0.307 mmol, 60.1 % yield). The sample was analyzed using the following LCMS method: Column = Acquity UPLC BEH C18, 2.1 × 50 mm, 1.7 µm particles; Solvent A = 95:5 Water:MeCN w/ 0.1% TFA; Solvent B = 5:95 Water:MeCN w/ 0.1% TFA; Flow rate = 0.80 mL/min.; Start % B = 0, End % B = 100, Gradient time = 1.50 min. followed by a 1.0 min hold at 100 % B; Detection = 220 nm and 254 nm. LCMS retention time = 1.076 min; observed ion = 651.10 (M+H). ¹H NMR (500 MHz, CD₃OD) δ (ppm) = 8.66 - 8.60 (m, 2H), 8.45 - 8.38 (m, 1H), 8.14 - 8.09 (m, 1H), 7.92 - 7.80 (m, 1H), 7.46 - 7.29 (m, 2H), 6.98 (br d, *J =* 7.5 Hz, 1H), 6.85 - 6.77 (m, 1H), 6.63 (br d, *J =* 6.6 Hz, 1H), 3.77 - 3.73 (m, 3H), 3.31 - 3.29 (m, 3H), 2.75 - 2.70 (m, 3H), 1.92 - 1.86 (m, 3H).

### Preparation of 2-(3,5-bis(trifluoromethyl)-1H-pyrazol-1-yl)acetic acid

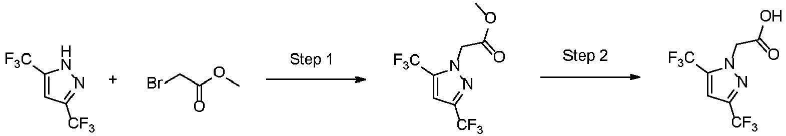

### Step 1:

To a solution of 3,5-bis(trifluoromethyl)-1H-pyrazole (1 g, 4.90 mmol) and methyl 2-bromoacetate (0.464 mL, 4.90 mmol) in N,N-Dimethylformamide (DMF) (20 mL) was added potassium carbonate (0.745 g, 5.39 mmol) and the mixture was heated to 60 °C and stirred for 3 hrs. The LCMS indicated the reaction to be complete. The reaction mixture was cooled to ambient temperature and diluted with EtOAc (75 mL) and water (125 mL). The mixture was mixed; then the phases were separated. The organic phase was washed water, then brine (50 mL). The organic phase was dried (MgSO₄), filtered and concentrated under reduced pressure. The resulting crude material was purified by silica gel chromatography (40 g column, 0-100% EtOAc in Hexanes) to afford the product methyl 2-(3,5-bis(trifluoromethyl)-1H-pyrazol-1-yl)acetate (1.14 g, 4.13 mmol, 84 % yield) as a clear liquid. ¹H NMR (CDCl₃, 500 MHz) δ 6.96 (s, 1H), 5.09 (s, 2H), 3.80 (s, 3H).

### Step 2:

To a solution of methyl 2-(3,5-bis(trifluoromethyl)-1H-pyrazol-1-yl)acetate (1.14 g, 4.13 mmol) in methanol (15 mL) was added aqueous sodium hydroxide (2.477 mL, 12.39 mmol) and the mixture was stirred at ambient temperature for 4 hrs. The reaction mixture was concentrated under reduced pressure and the resulting crude residue was diluted with aq. HCl (12.80 mL, 12.80 mmol) and water (25 mL). The mixture was extracted with EtOAc (50 mL). The organic phase was washed with brine, dried (MgSO₄), filtered, and concentrated under reduced pressure to afford the product 2-(3,5-bis(trifluoromethyl)-1H-pyrazol-1-yl)acetic acid (970 mg, 3.70 mmol, 90 % yield) as a white solid. ¹H NMR (500 MHz, CD₃OD) δ ppm 5.21 (s, 2 H), 7.25 (s, 1 H).

### Preparation of2-(3-(tert-butyl)-1H-pyrazol-1-yl)acetic acid

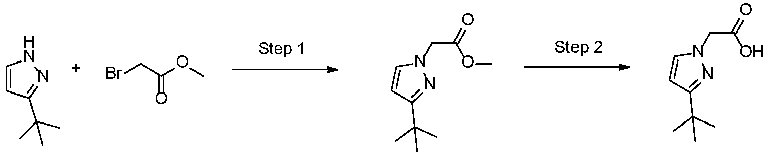

### Step 1:

To a solution of 3-(tert-butyl)-1H-pyrazole (1 g, 8.05 mmol) and methyl 2-bromoacetate (0.838 mL, 8.86 mmol) in DMF (40 mL) was added potassium carbonate (1.335 g, 9.66 mmol) and the mixture was stirred at ambient temperature for 72 hrs. The mixture was diluted with water and then extracted with EtOAc. The organic layer was washed with brine, dried (MgSO₄), filtered and concentrated under reduced pressure to afford the crude product. The crude product was purified via silica gel chromatography (40 g column, 0-100% EtOAc in Hexanes) to afford methyl 2-(3-(tert-butyl)-1H-pyrazol-1-yl)acetate contaminated with 3-(tert-butyl)-1H-pyrazole (1.5g, quantitative yield) as a clear oil. ¹H NMR (500 MHz, CDCl₃) δ ppm 1.31 - 1.34 (m, 9 H), 3.78 (s, 3 H), 4.90 (s, 2 H), 6.19 (d, *J*=2.38 Hz, 1 H), 7.37 (d, *J*=2.38 Hz, 1 H). The material was used in the next step without further purification.

### Step 2:

To a solution of methyl 2-(3-(tert-butyl)-1H-pyrazol-1-yl)acetate (1.5 g, 7.64 mmol) in Methanol (20.00 mL) and Tetrahydrofuran (THF) (20 mL) was added sodium hydroxide (4.59 mL, 22.93 mmol) and the mixture was stirred at RT for 18 hrs. The mixture was diluted with aq. HCl (22.93 mL, 22.93 mmol) and Water (25mL). The mixture was extracted with EtOAc (50 mL). The organic layer was washed with brine, dried over MgSO₄, filtered, and concentrated under reduced pressure to afford 2-(3-(tert-butyl)-1H-pyrazol-1-yl)acetic acid contaminated with 3-(tert-butyl)-1H-pyrazole (1.39 g, quantitative yield) as a white solid. ¹H NMR (500 MHz, CDCl₃) δ ppm 7.29 (d, *J=2.38* Hz, 1 H), 6.12 (d, *J*=2.38 Hz, 1 H), 4.83 (s, 2 H), 1.24 (s, 9 H).

### Preparation of 2-(3-(difluoromethyl)-1H-indazol-1-yl)acetic acid

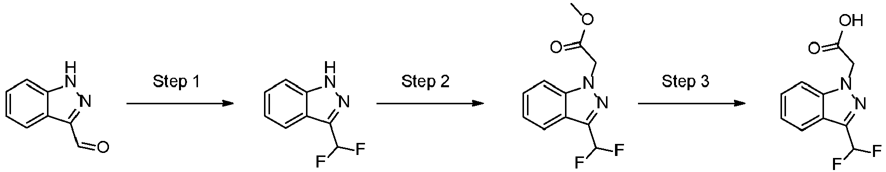

### Step 1:

To a 0 °C solution of 1H-indazole-3-carbaldehyde (2 g, 13.68 mmol) in dry dichloromethane (DCM) (50 mL) was added DAST (3.80 mL, 28.7 mmol) dropwise over 2 minutes. The solution was then allowed to warm to ambient temperature with stirring for 5 h. The reaction was cooled to 0 °C and then to the solution was added aq. tribasic potassium phosphate (0.75 M, 50 mL). The organic layer was washed with brine, dried (MgSO₄), filtered and concentrated under reduced pressure to afford the crude product which was then purified by silica gel chromatography (80 g column, 0-100% EtOAc in Hexanes) to afford 3-(difluoromethyl)-1H-indazole (275 mg, 1.635 mmol, 11.95 % yield) as a pink solid. ¹H NMR (500 MHz, CDCl₃) δ ppm 10.40 (br s, 1 H), 7.99 (d, *J*=8.05 Hz, 1 H), 7.53 - 7.57 (m, 1 H), 7.46 - 7.51 (m, 1 H), 7.30 (ddd, J=8.05, 6.85, 0.89 Hz, 1 H), 6.92 - 7.18 (m, 1 H).

### Step 2:

To a solution of 3-(difluoromethyl)-1H-indazole (275 mg, 1.635 mmol) and methyl 2-bromoacetate (0.186 mL, 1.963 mmol) in DMF (10 mL) was added potassium carbonate (271 mg, 1.963 mmol) and the mixture was stirred at ambient temperature for 18 h. The mixture was diluted with water and extracted with EtOAc. The organic layer was washed with brine, dried (MgSO₄), filtered, and concentrated under reduced pressure to afford the crude product which was purified by silica gel chromatography (40 g column, 0-100% EtOAc in Hexanes) to afford methyl 2-(3-(difluoromethyl)-1H-indazol-1-yl)acetate (216 mg, 0.899 mmol, 55.0 % yield) as a clear oil. ¹H NMR (500 MHz, CDCl₃) δ ppm 7.97 (br d, J=8.05 Hz, 1 H), 7.48 - 7.56 (m, 1 H), 7.39 (br d, J=8.64 Hz, 1 H), 7.31 (br t, *J*=7.60 Hz, 1 H), 6.85 - 7.13 (m, 1 H), 5.20 (s, 2 H), 3.79 (s, 3 H).

### Step 3:

To a solution of methyl 2-(3-(difluoromethyl)-1H-indazol-1-yl)acetate (216 mg, 0.899 mmol) in methanol (3.00 mL) and tetrahydrofuran (THF) (3 mL) was added sodium hydroxide (0.360 mL, 1.798 mmol) and the resulting mixture was stirred at ambient temperature for 1 h. The mixture was diluted with aq. HCl (1.798 mL, 1.798 mmol) and water (25mL) and then was extracted with EtOAc (50 mL). The organic layer was washed with brine, dried (MgSO₄), filtered, and concentrated under reduced pressure to afford the product 2-(3-(difluoromethyl)-1H-indazol-1-yl)acetic acid (202 mg, 0.893 mmol, 99 % yield) as a white solid. ¹H NMR (500 MHz, CDCl₃) δ ppm 7.93 - 8.00 (m, 1 H), 7.50 - 7.54 (m, 1 H), 7.39 (dt, *J*=8.64, 0.89 Hz, 1 H), 7.30 - 7.35 (m, 1 H), 6.87 - 7.11 (m, 1 H), 5.24 (s, 2 H). The sample was analyzed by LCMS Method F: retention time = 2.15 min.; observed ion = 268.1 (M+MeCN).

### Preparation of 2-(5-cyclopropyl-3-(trifluoromethyl)-1H-pyrazol-1-yl)acetic acid

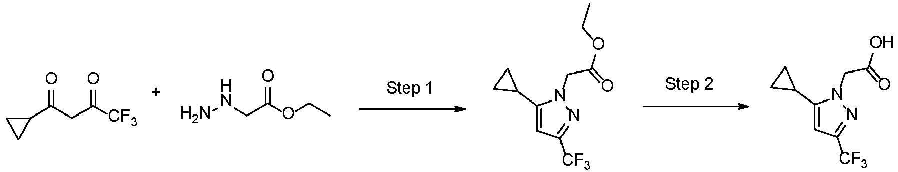

### Step 1:

To a solution of 1-cyclopropyl-4,4,4-trifluorobutane-1,3-dione (2 g, 11.10 mmol) in ethanol (50 mL) was added ethyl aminoglycinate hydrochloride (1.888 g, 12.21 mmol) at ambient temperature. The round bottom flask was then fitted with a reflux condenser and the reaction mixture was heated to 80 °C for 18 h. After 18 h, the mixture was cooled to room temperature and then was concentrated under reduced pressure. The residue was dissolved in EtOAc and washed with water. The organic layer was washed with brine, dried (MgSO₄), filtered, and concentrated in vacuo to afford the product ethyl 2-(5-cyclopropyl-3-(trifluoromethyl)-1H-pyrazol-1-yl)acetate (2.91 g, 11.10 mmol, 100 % yield) as a yellow oil. ¹H NMR (500 MHz, *METHAlVOL-d*₄*)* δ ppm 6.29 (s, 1 H), 5.14 (s, 2 H), 4.27 (q, *J*=7.15 Hz, 2 H), 1.84 (tt, *J*=8.34, 5.07 Hz, 1 H), 1.31 (t, *J*=7.15 Hz, 3 H), 1.00 - 1.07 (m, 2 H), 0.72 - 0.77 (m, 2 H). The sample was analyzed by LCMS Method F: retention time = 2.76 min.; observed ion = 263.1 (M+MeCN).

### Step 2:

To a solution of ethyl 2-(5-cyclopropyl-3-(trifluoromethyl)-1H-pyrazol-1-yl)acetate (2.91 g, 11.10 mmol) in Methanol (20.00 mL) and Tetrahydrofuran (THF) (20 mL) was added aq. sodium hydroxide (4.44 mL, 22.19 mmol) and the mixture was stirred at ambient temperature for 18 h. The mixture was concentrated under reduced pressure and the resulting residue was diluted with aq. HCl (22.19 mL, 22.19 mmol) and Water (25mL). The mixture was extracted with EtOAc (50 mL) and the organic layer was washed with brine, dried over MgSO₄, filtered, and concentrated in vacuo to afford the product 2-(5-cyclopropyl-3-(trifluoromethyl)-1H-pyrazol-1-yl)acetic acid (2.1 g, 8.97 mmol, 81 % yield) as an off-white solid. ¹H NMR (500 MHz, CD₃OD) δ ppm 6.28 (s, 1 H), 5.10 (s, 2 H), 1.80 - 1.88 (m, 1 H), 1.01 - 1.06 (m, 2 H), 0.72 - 0.77 (m, 2 H). The sample was analyzed by LCMS Method F: retention time = 2.21 min.; observed ion = 276.1 (M+MeCN).

### Preparation of 2-(3-cyclopropyl-5-methyl-1H-pyrazol-1-yl)acetic acid

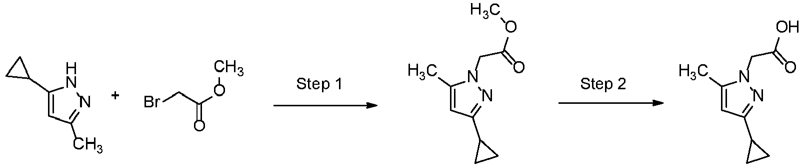

### Step 1:

To a solution of 5-cyclopropyl-3-methyl-1H-pyrazole (0.5 g, 4.09 mmol) and methyl 2-bromoacetate (0.426 mL, 4.50 mmol) in DMF (10 mL) was added potassium carbonate (0.679 g, 4.91 mmol) and the mixture was stirred at ambient temperature for 18 h. To the mixture was added methyl 2-bromoacetate (0.110 mL) and the mixture was then stirred for 5 h. The mixture was diluted with water and extracted with EtOAc. The organic layer was washed with brine, dried (MgSO₄), filtered and concentrated under reduced pressure to afford the crude product which was purified by silica gel chromatography (40 g column, 0-100% EtOAc in Hexanes) to afford the product methyl 2-(5-cyclopropyl-3-methyl-1H-pyrazol-1-yl)acetate as a clear oil. ¹H NMR (500 MHz, CDCl₃) δ ppm 5.72 (s, 1 H), 4.79 (s, 2 H), 3.78 (s, 3 H), 2.20 (d, *J*=0.89 Hz, 3 H), 1.90 (tt, *J*=8.49, 5.07 Hz, 1 H), 0.87 - 0.92 (m, 2 H), 0.67 - 0.71 (m, 2 H). The sample was analyzed by LCMS Method F: retention time = 1.88 min.; observed ion = 236.1 (M+MeCN).

### Step 2:

To a solution of methyl 2-(3-cyclopropyl-5-methyl-1H-pyrazol-1-yl)acetate (391 mg, 2.013 mmol) in Methanol (4 mL) was added sodium hydroxide (1.208 mL, 6.04 mmol) and the mixture was stirred at ambient temperature for 4 h. The mixture was concentrated under reduced pressure and the resulting crude residue was diluted with aq. HCl (6.24 mL, 6.24 mmol) and water (25mL). The mixture was extracted with EtOAc (50 mL). The organic layer was washed with brine, dried (MgSO₄), filtered, and concentrated under reduced pressure to afford the product 2-(3-cyclopropyl-5-methyl-1H-pyrazol-1-yl)acetic acid (287 mg, 1.593 mmol, 79 % yield) as a white solid. ¹H NMR (500 MHz, CD₃OD) δ ppm 5.74 (s, 1 H), 4.77 (s, 2 H), 2.19 (s, 3 H), 1.81 (tt, *J*=8.49, 5.07 Hz, 1 H), 0.84 - 0.89 (m, 2 H), 0.61 - 0.66 (m, 2 H). The sample was analyzed by LCMS Method F: retention time = 1.52 min.; observed ion = 222.1 (M+MeCN).

### Preparation of 2-(3-cyclobutyl-1H-pyrazol-1-yl)acetic acid

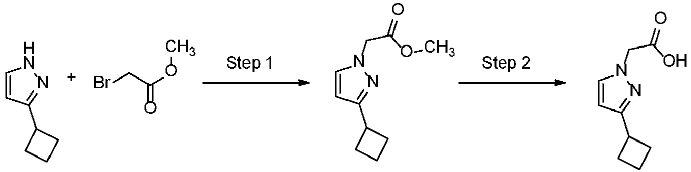

### Step 1:

To a solution of 3-cyclobutyl-1H-pyrazole (0.5 g, 4.09 mmol) and methyl 2-bromoacetate (0.426 mL, 4.50 mmol) in DMF (10 mL) was added potassium carbonate (0.679 g, 4.91 mmol) and the mixture was stirred at ambient temperature for 18 h. To the mixture was added methyl 2-bromoacetate (0.213 mL) and the mixture was then stirred for 18h. The mixture was diluted with water and extracted with EtOAc. The organic layer was washed with brine, dried over (MgSO₄), filtered, and concentrated under reduced pressure to afford the crude product which was purified by silica gel chromatography (40 g RediSep Gold column, 0-100% EtOAc in Hexanes) to afford the product methyl 2-(3-cyclobutyl-1H-pyrazol-1-yl)acetate (477 mg, 2.456 mmol, 60.0 % yield) as a clear oil. ¹H NMR (500 MHz, CDCl₃) δ ppm 7.39 (d, *J*=2.09 Hz, 1 H), 6.23 (d, *J=2.09* Hz, 1 H), 4.88 (s, 2 H), 3.78 (s, 3 H), 3.58 (quin, *J*=8.72 Hz, 1 H), 2.31 - 2.39 (m, 2 H), 2.17 - 2.27 (m, 2 H), 1.99 - 2.06 (m, 1 H), 1.86 - 1.94 (m, 1 H). The sample was analyzed by LCMS Method F: retention time = 2.08 min.; observed ion = 236.2 (M+MeCN).

### Step 2:

To solution of methyl 2-(3-cyclobutyl-1H-pyrazol-1-yl)acetate (477 mg, 2.456 mmol) in methanol (10 mL) was added sodium hydroxide (1.473 mL, 7.37 mmol) and the mixture was stirred at ambient temperature for 4 h. The mixture was concentrated under reduced pressure and the resulting crude residue was diluted with aq. HCl (7.61 mL, 7.61 mmol) and water (25 mL). The mixture was extracted with EtOAc (50 mL). The organic layer was washed with brine, dried over MgSO₄, filtered, and concentrated under reduced pressure to afford the product 2-(3-cyclobutyl-1H-pyrazol-1-yl)acetic acid (364 mg, 2.020 mmol, 82 % yield) as a white solid. ¹H NMR (500 MHz, CDCl₃) δ ppm 7.38 (d, *J*=2.38 Hz, 1 H), 6.23 (d, *J*=2.38 Hz, 1 H), 4.93 (s, 2 H), 3.59 (quin, *J*=8.64 Hz, 1 H), 2.30 - 2.41 (m, 2 H), 2.20 (quind, *J*=9.20, 9.20, 9.20, 9.20, 2.53 Hz, 2 H), 1.98 - 2.10 (m, 1 H), 1.84 - 1.96 (m, 1 H). ). The sample was analyzed by LCMS Method F: retention time = 1.77 min.; observed ion = 222.1 (M+MeCN).

### Preparation of 2-(5-methyl-3-(trifluoromethyl)-1H-pyrazol-1-yl)acetic acid

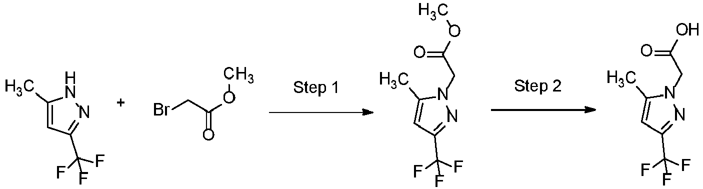

### Step 1:

To a solution of 5-methyl-3-(trifluoromethyl)-1H-pyrazole (0.500 g, 3.33 mmol) and methyl 2-bromoacetate (0.347 mL, 3.66 mmol) in N,N-Dimethylformamide (DMF) (10 mL) at ambient temperature was added potassium carbonate (0.506 g, 3.66 mmol) and the mixture was stirred for 18 h. The mixture was diluted with EtOAc (75 mL) and water (125 mL). The mixture was mixed; the layers were separated. The organic layer was washed with water followed by brine (50 mL), dried (MgSO₄), filtered, and concentrated under reduced pressure to afford the crude product which was purified by silica gel chromatography (40 g column, 0-100% EtOAc in Hexanes) to afford the product methyl 2-(5-methyl-3-(trifluoromethyl)-1H-pyrazol-1-yl)acetate (677 mg, 3.05 mmol, 91 % yield) as a clear oil. ¹H NMR (500 MHz, CDCl₃) δ ppm 6.36 (s, 1 H), 4.92 (s, 2 H), 3.81 (s, 3 H), 2.31 (s, 3 H). The sample was analyzed by LCMS Method F: retention time = 2.28 min.; observed ion = 223.1 (M+H).

### Step 2:

To a solution of methyl 2-(5-methyl-3-(trifluoromethyl)-1H-pyrazol-1-yl)acetate (677 mg, 3.05 mmol) in methanol (5 mL) was added sodium hydroxide (1.828 mL, 9.14 mmol) and the mixture was stirred at ambient temperature for 4 h. The mixture was concentrated under reduced pressure and the resulting crude residue was diluted with aq. HCl (9.45 mL, 9.45 mmol) and water (25mL). The mixture was extracted with EtOAc (50 mL). The organic layer was washed with brine, dried (MgSO₄), filtered, and concentrated under reduced pressure to afford the product 2-(5-methyl-3-(trifluoromethyl)-1H-pyrazol-1-yl)acetic acid (617 mg, 2.96 mmol, 97 % yield) as a white solid. ¹H NMR (500 MHz, CD₃OD) δ ppm 6.40 (s, 1 H), 4.98 (s, 2 H), 2.30 (s, 3 H). The sample was analyzed by LCMS Method F: retention time = 1.92 min.; observed ion = 250.1 (M+MeCN).

### Preparation of 2-(3-cyclopropyl-1H-indazol-1-yl)acetic acid

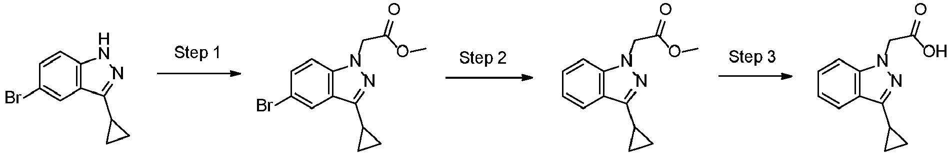

### Step 1:

To a solution of 5-bromo-3-cyclopropyl-1H-indazole (1 g, 4.22 mmol) and methyl 2-bromoacetate (0.439 mL, 4.64 mmol) in N,N-Dimethylformamide (DMF) (15 mL) was added potassium carbonate (0.641 g, 4.64 mmol) at RT and the mixture was stirred for 18 hrs. The mixture was diluted with water (125 mL) and then extracted with EtOAc (75 mL. The organic phase was washed with water followed by brine (50 mL). The organic layer was dried over MgSO₄, filtered, and concentrated in vacuo to afford the crude product which was purified by silica gel chromatography (40 g column, 0-100% EtOAc in Hexane) to afford methyl 2-(5-bromo-3-cyclopropyl-1H-indazol-1-yl)acetate (1.11 g, 3.59 mmol, 85 % yield) as a white solid. ¹H NMR (500 MHz, CDCl₃) δ ppm 1.02 - 1.05 (m, 4 H), 2.11 - 2.19 (m, 1 H), 3.73 (s, 3 H), 5.03 (s, 2 H), 7.13 (d, *J*=8.94 Hz, 1 H), 7.44 (dd, *J*=8.94, 1.79 Hz, 1 H), 7.89 (d, *J*=1.79 Hz, 1 H).

### Step 2:

To a degassed (nitrogen bubbling for 5 min.) solution of methyl 2-(5-bromo-3-cyclopropyl-1H-indazol-1-yl)acetate (1.02g, 3.30 mmol) in ethyl acetate (30 mL) and methanol (3.00 mL) was added Pd-C (0.176 g, 0.165 mmol). The flask was sealed and then purged with N₂ for 1 minute. A balloon of H₂ was connected to the flask through a needle and the flask was purged with H₂ for 30 seconds. The flask was then maintained under balloon-pressure H₂ atmosphere while the mixture was stirred at RT for 18 hrs. The mixture was sparged with N₂ for 5 minutes. Then the mixture was filtered through a pad of Celite and the filtrate was concentrated in vacuo to afford methyl 2-(3-cyclopropyl-1H-indazol-1-yl)acetate (661 mg, 2.87 mmol, 87 % yield) as a faint yellow oil. ¹H NMR (500 MHz, CDCl₃) δ ppm 1.03 - 1.08 (m, 2 H), 1.08 - 1.12 (m, 2 H), 2.21 - 2.28 (m, 1 H), 3.75 (s, 3 H), 5.09 (s, 2 H), 7.16 (ddd, *J*=8.12, 7.08, 0.89 Hz, 1 H), 7.25 - 7.28 (m, 1 H), 7.37 - 7.43 (m, 1 H), 7.77 (dt, *J=8.05,* 1.04 Hz, 1 H).

### Step 3:

To a solution of methyl 2-(3-cyclopropyl-1H-indazol-1-yl)acetate (661 mg, 2.87 mmol) in Tetrahydrofuran (THF) (7 mL) and Methanol (7.00 mL) was added 5 M aq. sodium hydroxide (1.722 mL, 8.61 mmol) and the mixture was stirred at RT for 2 hrs. The mixture was diluted with aq. HCl (1M, 10.05 mL, 10.05 mmol) and then was extracted with EtOAc. The organic layer was washed with brine, dried over MgSO₄, filtered and concentrated in vacuo to afford 2-(3-cyclopropyl-1H-indazol-1-yl)acetic acid (608 mg, 2.81 mmol, 98 % yield) as a white solid. ¹H NMR (500 MHz, *METHANOL-d*4) δ ppm 7.79 (dt, *J*=8.20, 0.97 Hz, 1 H), 7.39 - 7.47 (m, 2 H), 7.16 (ddd, *J=8.05,* 6.26, 1.49 Hz, 1 H), 5.12 (s, 2 H), 2.23 - 2.32 (m, 1 H), 1.03 - 1.08 (m, 4 H) 2-(1-isopropyl-1H-indol-3-yl)acetic acid was prepared according to the literature procedure (CN107151223, 2017). 2-(3-cyclopropyl-5-(difluoromethyl)-1H-pyrazol-1-yl)acetic acid, 2-(3-cyclopropyl-1H-pyrazol-1-yl)-2-methylpropanoic acid, 2-(3-cyclopropyl-1H-pyrazol-1-yl)-3-methylbutanoic acid, and 2-(1H-indol-1-yl)acetic acid were purchased from Enamine.

### Preparation of Example 1: (S)-2-(3,5-bis(trifluoromethyl)-1H-pyrazol-1-yl)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)acetamide.

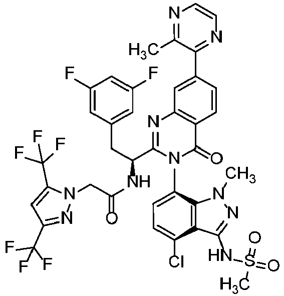

The title compound was prepared according to General Procedure C using 2-(3,5-bis(trifluoromethyl)-1H-pyrazol-1-yl)acetic acid as the coupling partner. The experiment afforded the title compound, (S)-2-(3,5-bis(trifluoromethyl)-1H-pyrazol-1-yl)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)acetamide. The sample was analyzed using LCMS Method E: retention time = 1.72 min.; observed ion = 895 (M+H). 1H NMR (500 MHz, METHANOL-d4) δ ppm 8.64 - 8.66 (m, 1 H), 8.62 (d, J=2.68 Hz, 1 H), 8.44 (d, J=8.05 Hz, 1 H), 8.13 (d, J=1.19 Hz, 1 H), 7.90 - 7.93 (m, 1 H), 7.32 - 7.36 (m, 1 H), 7.26 - 7.30 (m, 1 H), 7.15 (s, 1 H), 6.80 (tt, J=9.20, 2.27 Hz, 1 H), 6.59 - 6.63 (m, 2 H), 4.83 - 4.86 (m, 1 H), 3.65 (s, 3 H), 3.51 (dd, J=14.16, 4.62 Hz, 1 H), 3.25 (s, 3 H), 3.12 (dd, J=14.01, 9.54 Hz, 1 H), 2.72 (s, 3 H).

### Preparation of Example 2: (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-cyclopropyl-1H-indazol-1-yl)acetamide.

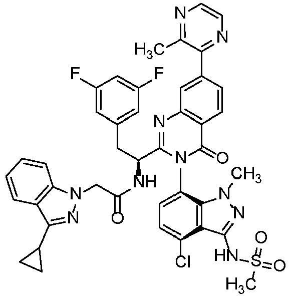

The title compound was prepared according to General Procedure C using 2-(3-cyclopropyl-1H-indazol-1-yl)acetic acid as the coupling partner modified as follows: the HATU coupling was stirred for 5 h instead of 18 h. The experiment afforded the title compound, (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-cyclopropyl-1H-indazol-1-yl)acetamide. The sample was analyzed using LCMS Method E: retention time = 1.36 min.; observed ion = 849.4 (M+H). 1H NMR (500 MHz, METHANOL-d4) δ ppm 8.64 - 8.69 (m, 1 H), 8.59 - 8.65 (m, 1 H), 8.42 (d, J=8.05 Hz, 1 H), 8.06 (d, J=1.19 Hz, 1 H), 7.91 (dd, J=8.35, 1.79 Hz, 1 H), 7.67 - 7.76 (m, 1 H), 7.31 - 7.36 (m, 1 H), 7.27 - 7.30 (m, 2 H), 7.19 - 7.25 (m, 1 H), 7.08 (ddd, J=7.97, 6.93, 0.89 Hz, 1 H), 6.76 (tt, J=9.24, 2.38 Hz, 1 H), 6.54 - 6.64 (m, 2 H), 4.92 - 4.97 (m, 1 H), 4.56 - 4.68 (m, 2 H), 3.55 (s, 3 H), 3.42 - 3.48 (m, 1 H), 3.24 (s, 3 H), 3.07 (dd, J=14.01, 9.24 Hz, 1 H), 2.72 (s, 3 H), 2.20 - 2.28 (m, 1 H), 0.96 - 1.06 (m, 4 H)

### Preparation of Example 3: (S)-2-(3,5-bis(trifluoromethyl)-1H-pyrazol-1-yl)-N-(1-((3P)-3-(4-chloro-1-methyl-3-(methylsulfonamido)-1H-indazol-7-yl)-7-(4,6-dimethylpyrimidin-2-yl)-4-oxo-3,4-dihydroquinazolin-2-yl)-2-(3,5-difluorophenyl)ethyl)acetamide.

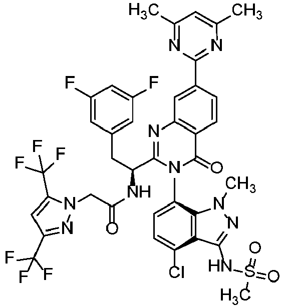

The title compound was prepared according to General Procedure C using 2-(3,5-bis(trifluoromethyl)-1H-pyrazol-1-yl)acetic acid as the coupling partner modified as follows: the amine used was (S)-N-(7-(2-(1-amino-2-(3,5-difluorophenyl)ethyl)-7-(4,6-dimethylpyrimidin-2-yl)-4-oxoquinazolin-3(4H)-yl)-4-chloro-1-methyl-1H-indazol-3-yl)methanesulfonamide. The experiment afforded the title compound, (S)-2-(3,5-bis(trifluoromethyl)-1H-pyrazol-1-yl)-N-(1-((3P)-3-(4-chloro-1-methyl-3-(methylsulfonamido)-1H-indazol-7-yl)-7-(4,6-dimethylpyrimidin-2-yl)-4-oxo-3,4-dihydroquinazolin-2-yl)-2-(3,5-difluorophenyl)ethyl)acetamide. The sample was analyzed using LCMS Method B: retention time = 1.52 min.; observed ion = 909.4 (M+H). 1H NMR (500 MHz, METHANOL-d4) δ ppm 8.94 (d, J=1.19 Hz, 1 H), 8.68 (dd, J=8.49, 1.64 Hz, 1 H), 8.40 (d, J=8.05 Hz, 1 H), 7.32 (br d, J=8.05 Hz, 1 H), 7.29 (s, 1 H), 7.24 (d, J=7.75 Hz, 1 H), 7.15 (s, 1 H), 6.80 (tt, J=9.16, 2.16 Hz, 1 H), 6.57 - 6.66 (m, 2 H), 4.90 (br d, J=12.52 Hz, 2 H), 3.63 (s, 3 H), 3.52 (dd, J=14.01, 4.77 Hz, 1 H), 3.24 (s, 3 H), 3.13 (dd, J=14.16, 9.39 Hz, 1 H), 2.63 (s, 6 H)

### Preparation of Example 4: (S)-N-(1-((3P)-3-(4-chloro-1-methyl-3-(methylsulfonamido)-1H-indazol-7-yl)-7-(4,6-dimethylpyrimidin-2-yl)-4-oxo-3,4-dihydroquinazolin-2-yl)-2-(3,5-difluorophenyl)ethyl)-2-(3-cyclopropyl-1H-indazol-1-yl)acetamide.

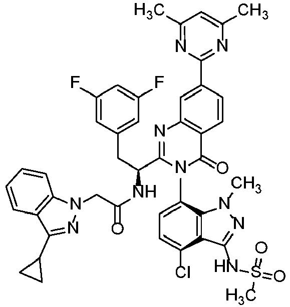

The title compound was prepared according to General Procedure C using 2-(3-cyclopropyl-1H-indazol-1-yl)acetic acid as the coupling partner modified as follows: the amine used was (S)-N-(7-(2-(1-amino-2-(3,5-difluorophenyl)ethyl)-7-(4,6-dimethylpyrimidin-2-yl)-4-oxoquinazolin-3(4H)-yl)-4-chloro-1-methyl-1H-indazol-3-yl)methanesulfonamide. The experiment afforded the title compound, (S)-N-(1-((3P)-3-(4-chloro-1-methyl-3-(methylsulfonamido)-1H-indazol-7-yl)-7-(4,6-dimethylpyrimidin-2-yl)-4-oxo-3,4-dihydroquinazolin-2-yl)-2-(3,5-difluorophenyl)ethyl)-2-(3-cyclopropyl-1H-indazol-1-yl)acetamide. The sample was analyzed using LCMS Method B: retention time = 1.55 min.; observed ion = 863.6 (M+H). 1H NMR (500 MHz, METHANOL-d4) δ ppm 8.90 (d, J=1.19 Hz, 1 H), 8.68 (dd, J=8.34, 1.79 Hz, 1 H), 8.37 (d, J=8.35 Hz, 1 H), 7.71 - 7.80 (m, 1 H), 7.23 - 7.35 (m, 4 H), 7.09 (ddd, J=8.05, 6.85, 0.89 Hz, 1 H), 6.76 (tt, J=9.16, 2.31 Hz, 1 H), 6.58 (dd, J=8.20, 2.24 Hz, 2 H), 4.89 - 4.92 (m, 1 H), 4.71 (s, 2 H), 4.56 - 4.63 (m, 1 H), 3.52 (s, 3 H), 3.44 (dd, J=14.45, 5.51 Hz, 1 H), 3.24 (s, 3 H), 3.06 (dd, J=14.01, 9.24 Hz, 1 H), 2.65 (s, 6 H), 2.24 - 2.32 (m, 1 H), 0.99 - 1.08 (m, 4 H)

### Preparation of Example 5: (S)-2-(3-(tert-butyl)-1H-pyrazol-1-yl)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)acetamide.

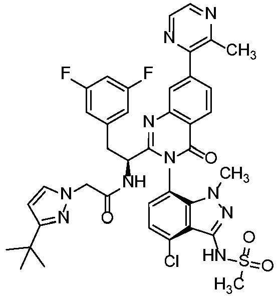

The title compound was prepared according to General Procedure C using 2-(3-(tert-butyl)-1H-pyrazol-1-yl)acetic acid as the coupling partner. The experiment afforded the title compound, (S)-2-(3-(tert-butyl)-1H-pyrazol-1-yl)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)acetamide. The sample was analyzed using LCMS Method B: retention time = 1.37 min.; observed ion = 815.4 (M+H). 1H NMR (500 MHz, METHANOL-d4) δ ppm 8.55 - 8.70 (m, 2 H), 8.43 (d, J=8.35 Hz, 1 H), 8.12 (d, J=1.79 Hz, 1 H), 7.91 (dd, J=8.20, 1.64 Hz, 1 H), 7.39 (d, J=2.38 Hz, 1 H), 7.33 (d, J=7.75 Hz, 1 H), 7.23 (d, J=7.75 Hz, 1 H), 6.77 (tt, J=9.20, 2.27 Hz, 1 H), 6.68 (dd, J=8.20, 2.24 Hz, 2 H), 6.15 (d, J=2.38 Hz, 1 H), 5.00 (dd, J=8.49, 5.81 Hz, 1 H), 4.59 (br t, J=4.77 Hz, 1 H), 4.33 - 4.49 (m, 2 H), 3.57 (s, 3 H), 3.51 (dd, J=13.86, 5.81 Hz, 1 H), 3.29 (s, 3 H), 3.08 (dd, J=14.01, 8.64 Hz, 1 H), 2.72 (s, 3 H), 1.24 (s, 9 H)

### Preparation of Example 6: 2-((3bS,4aR)-3-(difluoromethyl)-5,5-difluoro-3b,4,4a,5-tetrahydro-1H-cyclopropa[3,4]cyclopenta[1,2-c]pyrazol-1-yl)-N-((S)-2-(3,5-difluorophenyl)-1-(3-(1,4-dimethyl-3-(methylsulfonamido)-1H-indazol-7-yl)-(3P)-7-(3-methylpyrazin-2-yl)-4-oxo-3,4-dihydroquinazolin-2-yl)ethyl)acetamide.

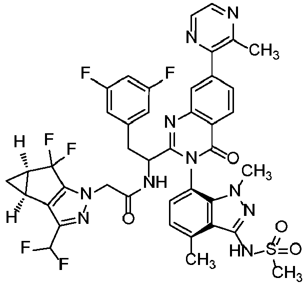

N-((S)-1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (25 mg, 0.028 mmol), RuPhos Pd G3 (2.3 mg, 2.8 µmol), 2,4,6-trimethyl-1,3,5,2,4,6-trioxatriborinane (35.0 mg, 0.279 mmol), and tribasic potassium phosphate (17.7 mg, 0.084 mmol) were combined in a 5 mL MW vial at rt. The vial was degassed (brief vacuum evacuation followed by refill with argon, repeated three times) and then to the vial was added dioxane (0.5 ml) and Water (0.1 ml). The vial was again degassed (brief vacuum evacuation followed by refill with argon, repeated three times), then the mixture was stirred at 100°C for 3 hrs. The reaction mixture was concentrated in vacuo and the residue was subjected to HPLC purification to afford the title compound, 2-((3bS,4aR)-3-(difluoromethyl)-5,5-difluoro-3b,4,4a,5-tetrahydro-1H-cyclopropa[3,4]cyclopenta[1,2-c]pyrazol-1-yl)-N-((S)-2-(3,5-difluorophenyl)-1-(3-(1,4-dimethyl-3-(methylsulfonamido)-1H-indazol-7-yl)-(3P)-7-(3-methylpyrazin-2-yl)-4-oxo-3,4-dihydroquinazolin-2-yl)ethyl)acetamide. The sample was analyzed using LCMS Method E: retention time = 1.66 min.; observed ion = 877.4 (0). 1H NMR (METHANOL-d4, 500 MHz) δ 8.6-8.7 (m, 2H), 8.43 (d, 1H, J=8.6 Hz), 8.11 (d, 1H, J=7.6 Hz), 7.90 (t, 1H, J=6.9 Hz), 7.1-7.3 (m, 2H), 6.5-6.8 (m, 4H), 4.8-4.9 (m, 2H), 4.5-4.6 (m, 2H), 3.65 (br d, 3H, J=6.0 Hz), 3.4-3.5 (m, 1H), 3.2-3.3 (m, 3H), 3.0-3.1 (m, 1H), 2.88 (s, 2H), 2.72 (s, 3H), 2.4-2.5 (m, 2H), 1.3-1.4 (m, 1H), 1.00 (br s, 1H). Note: ¹⁹F- NMR indicated that the reaction conditions racemized the amine center to afford a ∼1:1 mixture of diastereomers (as indicated).

### Preparation of Example 7: (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-(difluoromethyl)-1H-indazol-1-yl)acetamide.

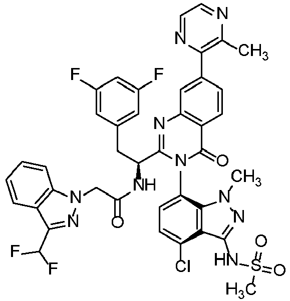

The title compound was prepared according to General Procedure C using 2-(3-(difluoromethyl)-1H-indazol-1-yl)acetic acid as the coupling partner. The experiment afforded the title compound, (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-(difluoromethyl)-1H-indazol-1-yl)acetamide. The sample was analyzed using LCMS Method B: retention time = 1.32 min.; observed ion = 857.4 (M-H). 1H NMR (500 MHz, METHANOL-d4) δ ppm 8.65 - 8.67 (m, 1 H), 8.63 (d, J=2.38 Hz, 1 H), 8.43 (d, J=8.05 Hz, 1 H), 8.10 (d, J=1.79 Hz, 1 H), 7.89 - 7.93 (m, 1 H), 7.81 (d, J=8.05 Hz, 1 H), 7.39 (ddd, J=8.42, 6.93, 1.04 Hz, 1 H), 7.29 - 7.34 (m, 3 H), 7.22 (ddd, J=8.05, 6.85, 0.89 Hz, 1 H), 6.89 - 7.13 (m, 1 H), 6.73 - 6.78 (m, 1 H), 6.66 (dd, J=8.34, 2.09 Hz, 2 H), 5.01 (dd, 1=9.69, 4.92 Hz, 1 H), 4.70 - 4.82 (m, 2 H), 3.61 (s, 3 H), 3.51 (dd, J=14.01, 4.77 Hz, 1 H), 3.21 (s, 3 H), 3.15 (dd, J=14.01, 9.54 Hz, 1 H), 2.72 (s, 3 H)

### Preparation of Example 8: (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(1-isopropyl-1H-indol-3-yl)acetamide.

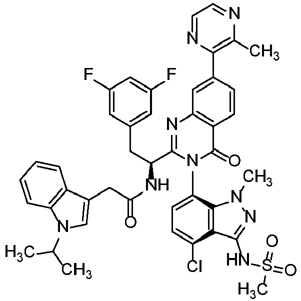

The title compound was prepared according to General Procedure C using 2-(1-isopropyl-1H-indol-3-yl)acetic acid as the coupling partner modified as follows: the amine used was (S)-N-(7-(2-(1-amino-2-(3,5-difluorophenyl)ethyl)-7-(3-methylpyrazin-2-yl)-4-oxoquinazolin-3(4H)-yl)-4-chloro-1-methyl-1H-indazol-3-yl)methanesulfonamide . The experiment afforded the title compound, (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(1-isopropyl-1H-indol-3-yl)acetamide. The sample was analyzed using LCMS Method B: retention time = 1.38 min.; observed ion = 850.5 (M+H). 1H NMR (500 MHz, METHANOL-d4) δ ppm 8.67 (d, J=2.38 Hz, 1 H), 8.64 (d, J=2.68 Hz, 1 H), 8.40 (d, J=8.05 Hz, 1 H), 7.98 (d, J=1.49 Hz, 1 H), 7.88 - 7.91 (m, 1 H), 7.32 - 7.35 (m, 2 H), 7.28 - 7.32 (m, 2 H), 7.14 (s, 1 H), 7.00 (ddd, J=8.20, 7.00, 1.19 Hz, 1 H), 6.80 (ddd, J=7.90, 7.00, 0.89 Hz, 1 H), 6.72 (tt, J=9.24, 2.38 Hz, 1 H), 6.58 - 6.63 (m, 2 H), 4.99 (dd, J=9.39, 4.92 Hz, 1 H), 4.64 (dt, J=13.41, 6.71 Hz, 1 H), 3.43 - 3.48 (m, 4 H), 3.35 (d, J=4.47 Hz, 2 H), 3.27 (s, 3 H), 3.08 (dd, J=14.01, 9.54 Hz, 1 H), 2.71 (s, 3 H), 1.45 (dd, J=7.60, 6.71 Hz, 6 H)

### Preparation of Example 9: (S)-N-(1-((3P)-3-(4-chloro-1-methyl-3-(methylsulfonamido)-1H-indazol-7-yl)-7-(4,6-dimethylpyrimidin-2-yl)-4-oxo-3,4-dihydroquinazolin-2-yl)-2-(3,5-difluorophenyl)ethyl)-2-(5-cyclopropyl-3-(trifluoromethyl)-1H-pyrazol-1-yl)acetamide.

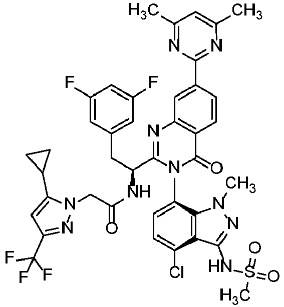

The title compound was prepared according to General Procedure C using 2-(5-cyclopropyl-3-(trifluoromethyl)-1H-pyrazol-1-yl)acetic acid as the coupling partner modified as follows: the amine used was (S)-N-(7-(2-(1-amino-2-(3,5-difluorophenyl)ethyl)-7-(4,6-dimethylpyrimidin-2-yl)-4-oxoquinazolin-3(4H)-yl)-4-chloro-1-methyl-1H-indazol-3-yl)methanesulfonamide. The experiment afforded the title compound, (S)-N-(1-((3P)-3-(4-chloro-1-methyl-3-(methylsulfonamido)-1H-indazol-7-yl)-7-(4,6-dimethylpyrimidin-2-yl)-4-oxo-3,4-dihydroquinazolin-2-yl)-2-(3,5-difluorophenyl)ethyl)-2-(5-cyclopropyl-3-(trifluoromethyl)-1H-pyrazol-1-yl)acetamide. The sample was analyzed using LCMS Method B: retention time = 1.52 min.; observed ion = 881.4 (M+H). 1H NMR (500 MHz, METHANOL-d4) δ ppm 8.90 (d, J=1.49 Hz, 1 H), 8.68 (dd, J=8.34, 1.79 Hz, 1 H), 8.54 - 8.63 (m, 1 H), 8.40 (d, J=8.34 Hz, 1 H), 7.29 - 7.34 (m, 2 H), 6.78 (tt, J=9.28, 2.20 Hz, 1 H), 6.60 - 6.68 (m, 2 H), 6.18 (s, 1 H), 4.71 (s, 2 H), 4.59 - 4.63 (m, 1 H), 3.67 (s, 3 H), 3.51 - 3.56 (m, 1 H), 3.25 (s, 3 H), 3.14 (dd, J=14.31, 9.54 Hz, 1 H), 2.64 (s, 6 H), 1.51 (ddd, J=13.49, 8.27, 5.07 Hz, 1 H), 0.92 - 0.98 (m, 1 H), 0.83 - 0.91 (m, 1 H), 0.60 (dd, J=4.92, 2.24 Hz, 2 H)

### Preparation of Example 10: (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-cyclopropyl-5-methyl-1H-pyrazol-1-yl)acetamide.

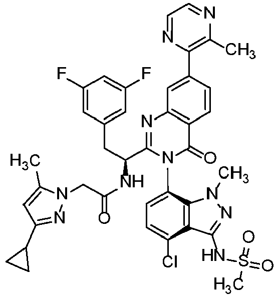

The title compound was prepared according to General Procedure C using 2-(3-cyclopropyl-5-methyl-1H-pyrazol-1-yl)acetic acid as the coupling partner. The experiment afforded the title compound, (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-cyclopropyl-5-methyl-1H-pyrazol-1-yl)acetamide. The sample was analyzed using LCMS Method B: retention time = 1.27 min.; observed ion = 813.4 (M+H). 1H NMR (500 MHz, METHANOL-d4) δ ppm 8.58 - 8.64 (m, 2 H), 8.42 (d, J=8.34 Hz, 1 H), 8.06 - 8.13 (m, 1 H), 7.87 - 7.92 (m, 1 H), 7.24 - 7.35 (m, 2 H), 6.76 (ddt, J=11.44, 7.04, 2.24, 2.24 Hz, 1 H), 6.55 - 6.61 (m, 2 H), 5.63 - 5.68 (m, 1 H), 4.89 - 4.93 (m, 1 H), 4.54 (d, J=2.38 Hz, 1 H), 4.36 - 4.40 (m, 1 H), 4.28 - 4.33 (m, 1 H), 3.62 - 3.68 (m, 3 H), 3.46 (dd, J=13.56, 4.92 Hz, 1 H), 3.23 - 3.27 (m, 3 H), 3.02 - 3.08 (m, 1 H), 2.67 - 2.72 (m, 3 H), 1.97 (s, 3 H), 1.74 - 1.80 (m, 1 H), 1.36 - 1.42 (m, 1 H), 0.76 - 0.84 (m, 2 H), 0.57 - 0.61 (m, 1 H), 0.42 - 0.53 (m, 1 H)

### Preparation of Example 11: (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-cyclobutyl-1H-pyrazol-1-yl)acetamide.

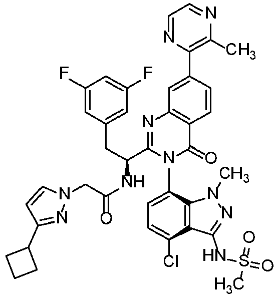

The title compound was prepared according to General Procedure C using 2-(3-cyclobutyl-1H-pyrazol-1-yl)acetic acid as the coupling partner. The experiment afforded the title compound, (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(3-cyclobutyl-1H-pyrazol-1-yl)acetamide. The sample was analyzed using LCMS Method B: retention time = 1.32 min.; observed ion = 813.5 (M+H). 1H NMR (500 MHz, METHANOL-d4) δ ppm 8.63 (d, J=2.38 Hz, 1 H), 8.60 (d, J=2.68 Hz, 1 H), 8.41 (d, J=8.05 Hz, 1 H), 8.11 (d, J=1.49 Hz, 1 H), 7.89 (dd, J=8.20, 1.64 Hz, 1 H), 7.36 (d, J=2.38 Hz, 1 H), 7.29 - 7.33 (m, 1 H), 7.23 (d, J=7.75 Hz, 1 H), 6.74 (tt, J=9.20, 2.27 Hz, 1 H), 6.62 - 6.69 (m, 2 H), 6.13 (d, J=2.38 Hz, 1 H), 4.97 (dd, J=8.64, 5.66 Hz, 1 H), 4.39 - 4.44 (m, 1 H), 4.28 - 4.33 (m, 1 H), 3.61 (s, 3 H), 3.43 - 3.53 (m, 2 H), 3.26 (s, 3 H), 3.07 (dd, J=13.71, 8.64 Hz, 1 H), 2.70 (s, 3 H), 2.19 - 2.27 (m, 2 H), 2.06 - 2.16 (m, 2 H), 1.91 - 2.01 (m, 1 H), 1.78 - 1.86 (m, 1 H)

### Preparation of Example 12: (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(5-methyl-3-(trifluoromethyl)-1H-pyrazol-1-yl)acetamide.

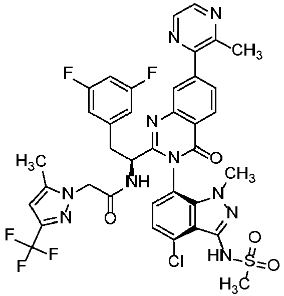

The title compound was prepared according to General Procedure C using 2-(5-methyl-3-(trifluoromethyl)-1H-pyrazol-1-yl)acetic acid as the coupling partner. The experiment afforded the title compound, (S)-N-(1-((3P)-3-(4-chloro-1-methyl-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-(5-methyl-3-(trifluoromethyl)-1H-pyrazol-1-yl)acetamide. The sample was analyzed using LCMS Method B: retention time = 1.3 min.; observed ion = 839.6 (M-H). 1H NMR (500 MHz, METHANOL-d4) δ ppm 8.61 - 8.64 (m, 1 H), 8.60 (d, J=2.38 Hz, 1 H), 8.42 (d, J=8.64 Hz, 1 H), 8.12 (d, J=1.79 Hz, 1 H), 7.89 (dd, 1=8.20, 1.64 Hz, 1 H), 7.28 - 7.32 (m, 1 H), 7.20 - 7.26 (m, 1 H), 6.74 (tt, J=9.13, 2.35 Hz, 1 H), 6.64 (dd, J=8.20, 2.24 Hz, 2 H), 6.28 (s, 1 H), 4.98 (dd, J=9.09, 5.22 Hz, 1 H), 4.49 - 4.54 (m, 1 H), 4.39 - 4.44 (m, 1 H), 3.65 (s, 3 H), 3.51 (dd, J=13.86, 5.22 Hz, 1 H), 3.24 (s, 3 H), 3.10 (dd, J=13.86, 9.09 Hz, 1 H), 2.70 (s, 3 H), 2.07 (s, 3 H)

### Preparation of Example 13: (S)-N-(1-((3P)-3-(4-chloro-3-(cyclopropanesulfonamido)-1-(2,2-difluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-indazol-1-yl)acetamide.

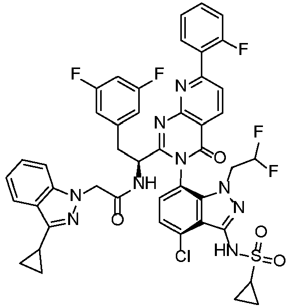

To a stirred solution of (S)-N-((6P)-7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)-N-(4-methoxybenzyl)cyclopropanesulfonamide (0.04 g, 0.047 mmol) in N,N-Dimethylformamide (1 mL) were added 2-(3-cyclopropyl-1H-indazol-1-yl)acetic acid (10.17 mg, 0.047 mmol), 2-(3H-[1,2,3]triazolo[4,5-b]pyridin-3-yl)-1,1,3,3-tetramethylisouronium hexafluorophosphate(V) (0.018 g, 0.047 mmol) and DIPEA (8.22 µl, 0.047 mmol). The mixture was stirred for 2 h and then concentrated under reduced pressure. The residue was taken up in DCM/TFA (1:1, 2 mL) and to the solution was added triflic acid (0.017 mL, 0.188 mmol). The resulting purple solution was stirred for 1 h, and then was concentrated under reduced pressure. The residue was dissolved in DCM (1.5 mL), then washed with sat NaHCO₃ (1 mL) and the organic layer was concentrated under reduced pressure. The resulting residue was dissolved in DMF, the solution was filtered, and the filtrate was subjected to prep-HPLC purification to afford the title compound, (S)-N-(1-((3P)-3-(4-chloro-3-(cyclopropanesulfonamido)-1-(2,2-difluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-indazol-1-yl)acetamide. The sample was analyzed using LCMS Method A: retention time = 1.54 min.; observed ion = 928.4 (M+H). 1H NMR (500 MHz, METHANOL-d4) δ ppm 8.73 - 8.81 (m, 1 H) 8.09 - 8.19 (m, 2 H) 7.70 - 7.76 (m, 1 H) 7.62 - 7.70 (m, 1 H) 7.36 - 7.50 (m, 4 H) 7.06 - 7.27 (m, 3 H) 6.75 - 6.83 (m, 1 H) 6.52 - 6.59 (m, 2 H) 5.84 - 6.12 (m, 1 H) 4.80 - 4.87 (m, 3 H) 4.21 - 4.37 (m, 1 H) 3.87 - 4.00 (m, 1 H) 3.39 - 3.45 (m, 1 H) 3.08 - 3.16 (m, 1 H) 2.83 - 2.91 (m, 1 H) 2.18 - 2.26 (m, 1 H) 0.89 - 1.13 (m, 8 H).

### Preparation of Example 14: (S)-2-(3-(tert-butyl)-1H-pyrazol-1-yl)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)acetamide.

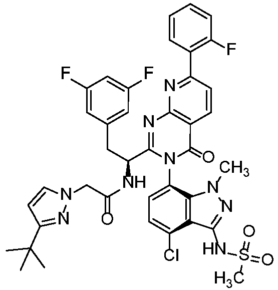

The title compound was prepared according to General Procedure A using 2-(3-(tert-butyl)-1H-pyrazol-1-yl)acetic acid as the coupling partner modified as follows: the reaction time was 2h. The experiment afforded the title compound, (S)-2-(3-(tert-butyl)-1H-pyrazol-1-yl)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)acetamide. The sample was analyzed using LCMS Method A: retention time = 1.52 min.; observed ion = 818.2 (M+H). 1H NMR (500 MHz, METHANOL-d4) δ ppm 8.73 - 8.80 (m, 1 H) 8.08 - 8.16 (m, 2 H) 7.61 - 7.68 (m, 1 H) 7.26 - 7.49 (m, 5 H) 6.66 - 6.82 (m, 3 H) 6.11 - 6.16 (m, 1 H) 4.98 - 5.04 (m, 1 H) 4.40 - 4.52 (m, 2 H) 3.60 - 3.63 (m, 3 H) 3.52 - 3.58 (m, 1 H) 3.26 - 3.29 (m, 3 H) 3.13 - 3.20 (m, 1 H) 1.20 - 1.25 (m, 9 H).

### Preparation of Example 15: (S)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-indazol-1-yl)acetamide.

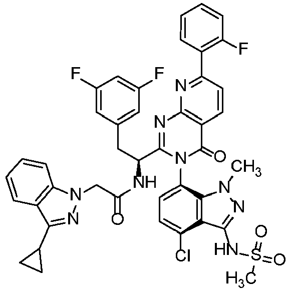

The title compound was prepared according to General Procedure A using 2-(3-cyclopropyl-1H-indazol-1-yl)acetic acid as the coupling partner modified as follows: the reaction time was 1h. The experiment afforded the title compound, (S)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-indazol-1-yl)acetamide. The sample was analyzed using LCMS Method A: retention time = 1.51 min.; observed ion = 852.4 (M+H). 1H NMR (500 MHz, METHANOL-d4) δ ppm 8.74 - 8.83 (m, 1 H) 8.09 - 8.17 (m, 2 H) 7.71 - 7.75 (m, 1 H) 7.61 - 7.67 (m, 1 H) 7.44 - 7.50 (m, 1 H) 7.31 - 7.41 (m, 3 H) 7.17 - 7.27 (m, 2 H) 7.06 - 7.11 (m, 1 H) 6.74 - 6.81 (m, 1 H) 6.60 - 6.66 (m, 2 H) 4.93 - 5.00 (m, 1 H) 4.67 - 4.74 (m, 2 H) 3.53 - 3.58 (m, 3 H) 3.47 - 3.52 (m, 1 H) 3.20 - 3.23 (m, 3 H) 3.13 - 3.17 (m, 1 H) 2.19 - 2.25 (m, 1 H) 0.97 - 1.02 (m, 4 H)

### Preparation of Example 16: (S)-N-(1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(5-cyclopropyl-3-(difluoromethyl)-1H-pyrazol-1-yl)acetamide.

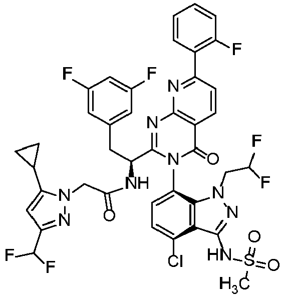

The title compound was prepared according to General Procedure A using 2-(5-cyclopropyl-3-(difluoromethyl)-1H-pyrazol-1-yl)acetic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)methanesulfonamide. The experiment afforded the title compound, (S)-N-(1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(5-cyclopropyl-3-(difluoromethyl)-1H-pyrazol-1-yl)acetamide. The sample was analyzed using LCMS Method A: retention time = 1.41 min.; observed ion = 902.3 (M+H). 1H NMR (500 MHz, METHANOL-d4) δ ppm 8.74 - 8.83 (m, 1 H) 8.08 - 8.17 (m, 2 H) 7.59 - 7.68 (m, 1 H) 7.33 - 7.50 (m, 4 H) 6.48 - 6.83 (m, 4 H) 5.89 - 6.19 (m, 2 H) 4.73 - 4.85 (m, 3 H) 4.36 - 4.51 (m, 1 H) 3.97 - 4.10 (m, 1 H) 3.43 - 3.51 (m, 1 H) 3.24 - 3.28 (m, 3 H) 3.10 - 3.20 (m, 1 H) 1.45 - 1.54 (m, 1 H) 0.73 - 0.90 (m, 2 H) 0.48 - 0.63 (m, 2 H)

### Preparation of Example 17: (S)-N-(1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-5-(difluoromethyl)-1H-pyrazol-1-yl)acetamide.

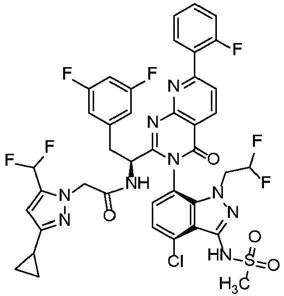

The title compound was prepared according to General Procedure A using 2-(3-cyclopropyl-5-(difluoromethyl)-1H-pyrazol-1-yl)acetic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)methanesulfonamide. The experiment afforded the title compound, (S)-N-(1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-5-(difluoromethyl)-1H-pyrazol-1-yl)acetamide. The sample was analyzed using LCMS Method A: retention time = 1.42 min.; observed ion = 902.4 (M+H). 1H NMR (500 MHz, METHANOL-d4) δ ppm 8.74 - 8.80 (m, 1 H) 8.08 - 8.19 (m, 2 H) 7.61 - 7.67 (m, 1 H) 7.32 - 7.50 (m, 4 H) 6.52 - 6.84 (m, 4 H) 5.91 - 6.24 (m, 2 H) 4.67 - 4.78 (m, 3 H) 4.30 - 4.44 (m, 1 H) 3.94 - 4.06 (m, 1 H) 3.40 - 3.49 (m, 1 H) 3.27 - 3.28 (m, 3 H) 3.09 - 3.17 (m, 1 H) 1.83 - 1.90 (m, 1 H) 0.85 - 0.91 (m, 2 H) 0.66 - 0.71 (m, 2 H)

### Preparation of Example 18: (S)-N-(1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-indazol-1-yl)acetamide.

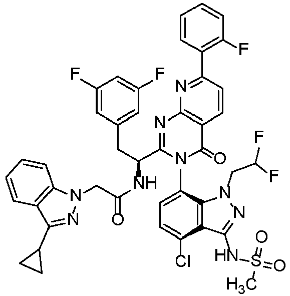

The title compound was prepared according to General Procedure A using 2-(3-cyclopropyl-1H-indazol-1-yl)acetic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)methanesulfonamide and the reaction time was 1h. The experiment afforded the title compound, (S)-N-(1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-indazol-1-yl)acetamide. The sample was analyzed using LCMS Method A: retention time = 1.51 min.; observed ion = 902.4 (M+H). 1H NMR (500 MHz, METHANOL-d4) δ ppm 8.73 - 8.82 (m, 1 H) 8.09 - 8.20 (m, 2 H) 7.71 - 7.76 (m, 1 H) 7.61 - 7.68 (m, 1 H) 7.34 - 7.50 (m, 4 H) 7.05 - 7.27 (m, 3 H) 6.74 - 6.83 (m, 1 H) 6.51 - 6.61 (m, 2 H) 5.83 - 6.11 (m, 1 H) 4.83 - 4.85 (m, 1 H) 4.79 - 4.81 (m, 2 H) 4.20 - 4.37 (m, 1 H) 3.84 - 3.99 (m, 1 H) 3.40 - 3.48 (m, 1 H) 3.19 - 3.21 (m, 3 H) 3.10 - 3.16 (m, 1 H) 2.19 - 2.26 (m, 1 H) 0.97 - 1.04 (m, 4 H)

### Preparation of Example 19: (S)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide.

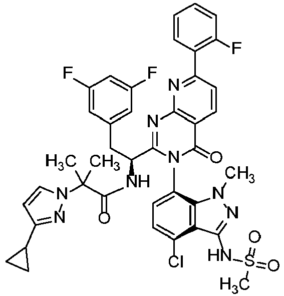

The title compound was prepared according to General Procedure A using 2-(3-cyclopropyl-1H-pyrazol-1-yl)-2-methylpropanoic acid as the coupling partner modified as follows: the reaction solvent was tetrahydrofuran (1 mL) : N,N-Dimethylformamide (0.250 mL). The experiment afforded the title compound, (S)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide. The sample was analyzed using LCMS Method H: retention time = 1.54 min.; observed ion = 830.2 (M+H). 1H NMR (500 MHz, METHANOL-d4) δ ppm 8.61 - 8.70 (m, 1 H) 7.94 - 8.07 (m, 2 H) 7.20 - 7.56 (m, 6 H) 6.61 - 6.73 (m, 1 H) 6.42 - 6.53 (m, 2 H) 5.83 - 5.90 (m, 1 H) 4.79 - 4.83 (m, 1 H) 3.51 (s, 3 H) 3.27 - 3.31 (m, 1 H) 3.18 - 3.19 (m, 3 H) 2.99 - 3.05 (m, 1 H) 1.64 - 1.77 (m, 1 H) 1.34 - 1.39 (m, 6 H) 0.59 - 0.64 (m, 2 H) 0.41 - 0.47 (m, 2 H)

### Preparation of Example 20: (S)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide.

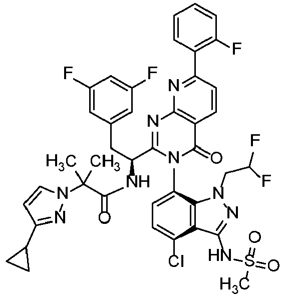

The title compound was prepared according to General Procedure A using 2-(3-cyclopropyl-1H-pyrazol-1-yl)-2-methylpropanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)methanesulfonamide and the reaction solvent was tetrahydrofuran (1 mL) : N,N-Dimethylformamide (0.250 mL). The experiment afforded the title compound, (S)-N-(1-((3P)-3-(4-chloro-1-methyl-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide. The sample was analyzed using LCMS Method H: retention time = 1.57 min.; observed ion = 880.3 (M+H). 1H NMR (500 MHz, METHANOL-d4) δ ppm 8.61 - 8.70 (m, 1 H) 7.94 - 8.07 (m, 2 H) 7.20 - 7.56 (m, 6 H) 6.61 - 6.73 (m, 1 H) 6.42 - 6.53 (m, 2 H) 5.83 - 5.90 (m, 1 H) 4.79 - 4.83 (m, 1 H) 3.51 (s, 3 H) 3.27 - 3.31 (m, 1 H) 3.18 - 3.19 (m, 3 H) 2.99 - 3.05 (m, 1 H) 1.64 - 1.77 (m, 1 H) 1.34 - 1.39 (m, 6 H) 0.59 - 0.64 (m, 2 H) 0.41 - 0.47 (m, 2 H)

### Preparation of Example 21: (S)-N-(1-((3P)-3-(4-chloro-3-(cyclopropanesulfonamido)-1-(2,2-difluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide.

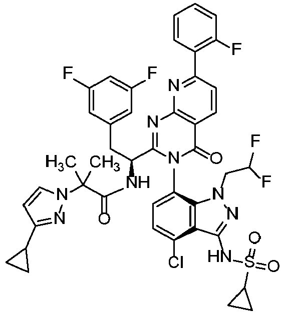

The title compound was prepared according to General Procedure A using 2-(3-cyclopropyl-1H-pyrazol-1-yl)-2-methylpropanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)cyclopropanesulfonamide and the reaction solvent was tetrahydrofuran (1 mL) : N,N-Dimethylformamide (0.250 mL). The experiment afforded the title compound, (S)-N-(1-((3P)-3-(4-chloro-3-(cyclopropanesulfonamido)-1-(2,2-difluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide. The sample was analyzed using LCMS Method H: retention time = 1.6 min.; observed ion = 906.4 (M+H). 1H NMR (500 MHz, METHANOL-d4) δ ppm 8.60 - 8.67 (m, 1 H) 7.95 - 8.08 (m, 2 H) 7.15 - 7.54 (m, 6 H) 6.62 - 6.72 (m, 1 H) 6.33 - 6.42 (m, 2 H) 5.81 - 5.89 (m, 1 H) 4.61 - 4.68 (m, 1 H) 4.19 - 4.35 (m, 1 H) 3.87 - 4.03 (m, 1 H) 3.12 - 3.17 (m, 3 H) 2.82 - 3.03 (m, 2 H) 1.69 - 1.78 (m, 1 H) 1.34 - 1.52 (m, 7 H) 0.90 - 0.98 (m, 2 H) 0.42 - 0.69 (m, 4 H)

### Preparation of Example 22: (S)-N-(1-((3P)-3-(4-chloro-3-(methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide.

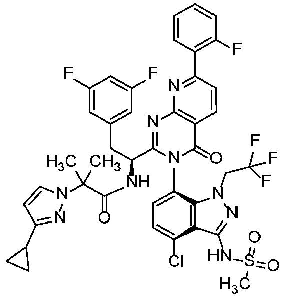

The title compound was prepared according to General Procedure A using 2-(3-cyclopropyl-1H-pyrazol-1-yl)-2-methylpropanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2,2-trifluoroethyl)-1H-indazol-3-yl)methanesulfonamide and the reaction solvent was tetrahydrofuran (1 mL) : N,N-Dimethylformamide (0.250 mL). The experiment afforded the title compound, (S)-N-(1-((3P)-3-(4-chloro-3-(methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-2-methylpropanamide. The sample was analyzed using LCMS Method H: retention time = 1.59 min.; observed ion = 898.3 (M+H). 1H NMR (500 MHz, METHANOL-d4) δ ppm 8.55 - 8.66 (m, 1 H) 7.93 - 8.08 (m, 2 H) 7.21 - 7.54 (m, 6 H) 6.59 - 6.71 (m, 1 H) 6.27 - 6.38 (m, 2 H) 5.80 - 5.89 (m, 1 H) 4.55 - 4.69 (m, 2 H) 4.17 - 4.31 (m, 1 H) 3.18 (br s, 3 H) 3.15 - 3.17 (m, 1 H) 2.87 - 3.00 (m, 1 H) 1.70 - 1.78 (m, 1 H) 1.40 - 1.50 (m, 6 H) 0.38 - 0.69 (m, 4 H)

### Preparation of Example 23: N-((S)-1-((3P)-3-(4-chloro-3-(methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide.

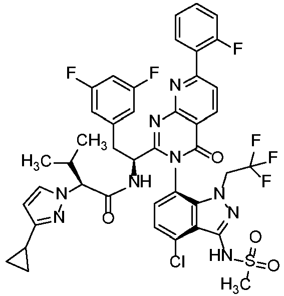

The title compound was prepared according to General Procedure A using 2-(3-cyclopropyl-1H-pyrazol-1-yl)-3-methylbutanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2,2-trifluoroethyl)-1H-indazol-3-yl)methanesulfonamide and the reaction solvent was tetrahydrofuran (1 mL) : N,N-Dimethylformamide (0.250 mL). The experiment afforded the title compound, N-((S)-1-((3P)-3-(4-chloro-3-(methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide. The sample was analyzed using LCMS Method H: retention time = 1.62 min.; observed ion = 912.3 (M+H). 1H NMR (500 MHz, METHANOL-d4) δ ppm 8.61 (d, J=8.34 Hz, 1 H) 7.98 - 8.07 (m, 2 H) 7.48 - 7.56 (m, 1 H) 7.32 - 7.45 (m, 4 H) 7.22 - 7.29 (m, 1 H) 6.62 (tt, J=9.16, 2.31 Hz, 1 H) 6.39 - 6.44 (m, 2 H) 5.80 (d, J=2.38 Hz, 1 H) 4.69 (dd, J=9.98, 4.32 Hz, 1 H) 4.15 - 4.27 (m, 1 H) 4.08 (d, J=10.13 Hz, 1 H) 3.97 (dq, J=16.35, 8.36 Hz, 1 H) 3.27 (dd, J=14.45, 4.62 Hz, 1 H) 3.02 (dd, J=14.45, 9.98 Hz, 1 H) 2.12 - 2.21 (m, 1 H) 1.77 (tt, J=8.38, 5.03 Hz, 1 H) 0.63 - 0.71 (m, 5 H) 0.44 - 0.56 (m, 5 H)

### Preparation of Example 24: N-((S)-1-((3P)-3-(4-chloro-3-(cyclopropanesulfonamido)-1-(2,2-difluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide.

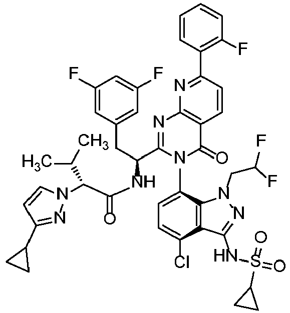

The title compound was prepared according to General Procedure A using 2-(3-cyclopropyl-1H-pyrazol-1-yl)-3-methylbutanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)cyclopropanesulfonamide and the reaction solvent was tetrahydrofuran (1 mL) : N,N-Dimethylformamide (0.250 mL). The experiment afforded the title compound, N-((S)-1-((3P)-3-(4-chloro-3-(cyclopropanesulfonamido)-1-(2,2-difluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide. The sample was analyzed using LCMS Method H: retention time = 1.56 min.; observed ion = 920.3 (M+H). 1H NMR (500 MHz, METHANOL-d4) δ ppm 8.62 (d, J=8.05 Hz, 1 H), 7.96 - 8.05 (m, 2 H), 7.50 - 7.56 (m, 1 H), 7.23 - 7.39 (m, 5 H), 6.64 - 6.71 (m, 1 H), 6.38 - 6.46 (m, 2 H), 5.82 (d, J=2.38 Hz, 1 H), 4.14 (d, J=10.43 Hz, 1 H), 3.29 (dd, J=14.45, 4.02 Hz, 1 H), 3.00 - 3.05 (m, 1 H), 2.84 - 2.91 (m, 1 H), 2.04 - 2.15 (m, 1 H), 1.69 - 1.76 (m, 1 H), 0.91 - 0.99 (m, 2 H), 0.66 - 0.74 (m, 2 H), 0.53 (d, J=6.56 Hz, 3 H), 0.45 (d, J=6.56 Hz, 3 H).

### Preparation of Example 25: N-((S)-1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide.

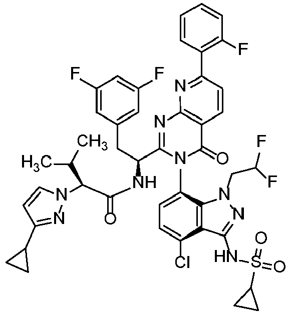

The title compound was prepared according to General Procedure A using 2-(3-cyclopropyl-1H-pyrazol-1-yl)-3-methylbutanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)methanesulfonamide and the reaction solvent was tetrahydrofuran (1 mL) : N,N-Dimethylformamide (0.250 mL). The experiment afforded the title compound, N-((S)-1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide (0.0015 g, 1.677 µmol, 2.495 % yield). The sample was analyzed using LCMS Method H: retention time = 1.58 min.; observed ion = 894.2 (M+H). 1H NMR (500 MHz, METHANOL-d4) δ ppm 8.62 - 8.64 (m, 1 H) 7.93 - 8.09 (m, 2 H) 7.20 - 7.55 (m, 7 H) 6.54 - 6.66 (m, 1 H) 6.37 - 6.48 (m, 2 H) 5.71 - 6.01 (m, 2 H) 3.98 - 4.10 (m, 1 H) 3.80 - 3.95 (m, 1 H) 3.57 - 3.72 (m, 1 H) 3.28 - 3.34 (m, 1 H) 3.18 - 3.19 (m, 3 H) 3.00 - 3.07 (m, 1 H) 2.13 (s, 1 H) 1.69 - 1.82 (m, 1 H) 0.63 - 0.69 (m, 5 H) 0.45 - 0.52 (m, 5 H)

### Preparation of Example 26: N-((S)-1-((3P)-3-(4-chloro-3-(methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide.

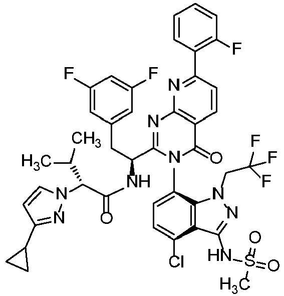

The title compound was prepared according to General Procedure A using 2-(3-cyclopropyl-1H-pyrazol-1-yl)-3-methylbutanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2,2-trifluoroethyl)-1H-indazol-3-yl)methanesulfonamide and the reaction solvent was tetrahydrofuran (1 mL) : N,N-Dimethylformamide (0.250 mL). The experiment afforded the title compound, N-((S)-1-((3P)-3-(4-chloro-3-(methylsulfonamido)-1-(2,2,2-trifluoroethyl)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide. The sample was analyzed using LCMS Method H: retention time = 1.55 min.; observed ion = 912.2 (M+H). 1H NMR (500 MHz, METHANOL-d4), 8.60 (d, J=8.05 Hz, 1 H), 7.94 - 8.07 (m, 2 H), 7.49 - 7.57 (m, 1 H), 7.22 - 7.42 (m, 5 H), 6.68 (s, 1 H), 6.41 (dd, J=8.05, 2.09 Hz, 2 H), 5.85 (d, J=2.38 Hz, 1 H), 4.17 (d, J=10.43 Hz, 1 H), 3.69 - 3.90 (m, 2 H), 3.24 - 3.29 (m, 1 H), 3.01 (dd, J=14.16, 10.28 Hz, 1 H), 2.06 - 2.17 (m, 1 H), 1.69 - 1.76 (m, 1 H), 0.68 - 0.73 (m, 2 H), 0.55 (d, J=6.56 Hz, 4 H), 0.46 - 0.46 (m, 1 H), 0.47 (d, J=6.85 Hz, 3 H).

### Preparation of Example 27: N-((S)-1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide (0.0014 g, 1.565 µmol, 2.329 % yield).

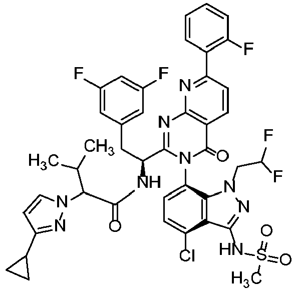

The title compound was prepared according to General Procedure A using 2-(3-cyclopropyl-1H-pyrazol-1-yl)-3-methylbutanoic acid as the coupling partner modified as follows: the amine coupling partner was (S)-N-(7-(2-(1-amino-2-(3,5-difluorophenyl)ethyl)-7-(2-fluorophenyl)-4-oxopyrido[2,3-d]pyrimidin-3(4H)-yl)-4-chloro-1-(2,2-difluoroethyl)-1H-indazol-3-yl)methanesulfonamide and the reaction solvent was tetrahydrofuran (1 mL) : N,N-Dimethylformamide (0.250 mL). The experiment afforded the title compound, N-((S)-1-((3P)-3-(4-chloro-1-(2,2-difluoroethyl)-3-(methylsulfonamido)-1H-indazol-7-yl)-7-(2-fluorophenyl)-4-oxo-3,4-dihydropyrido[2,3-d]pyrimidin-2-yl)-2-(3,5-difluorophenyl)ethyl)-2-(3-cyclopropyl-1H-pyrazol-1-yl)-3-methylbutanamide (0.0014 g, 1.565 µmol, 2.329 % yield). The sample was analyzed using LCMS Method H: retention time = 1.52 min.; observed ion = 894.3 (M+H). 1H NMR (500 MHz, METHANOL-d4) δ ppm 8.55 - 8.66 (m, 1 H) 7.88 - 8.09 (m, 2 H) 7.17 - 7.54 (m, 6 H) 6.59 - 6.73 (m, 1 H) 6.36 - 6.49 (m, 2 H) 5.60 - 5.91 (m, 2 H) 4.78 - 4.82 (m, 1 H) 4.14 (d, J=10.43 Hz, 1 H) 3.49 - 3.72 (m, 2 H) 3.26 - 3.32 (m, 1 H) 3.18 - 3.19 (m, 3 H) 2.99 - 3.05 (m, 1 H) 2.04 - 2.15 (m, 1 H) 1.68 - 1.77 (m, 1 H) 0.65 - 0.73 (m, 2 H) 0.39 - 0.57 (m, 8 H)

### Preparation of Example 28: (S)-N-(1-((3P)-3-(4-chloro-1-methyl-3-(methylsulfonamido)-1H-indazol-7-yl)-7-(2-fluorophenyl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide.

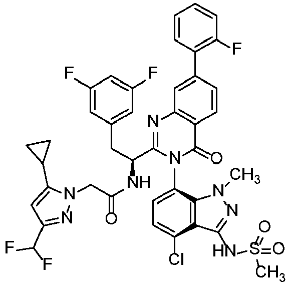

The title compound was prepared according to General Procedure I using 2-(5-cyclopropyl-3-(difluoromethyl)-1H-pyrazol-1-yl)acetic acid as the coupling partner modified as follows: the amine used was (S)-N-(7-(2-(1-amino-2-(3,5-difluorophenyl)ethyl)-7-(2-fluorophenyl)-4-oxoquinazolin-3(4H)-yl)-4-chloro-1-methyl-1H-indazol-3-yl)methanesulfonamide and immediately prior to HPLC purification the crude residue was stirred with ammonia in methanol (2M, 0.3 mL) for 10 min at room temperature. The experiment afforded the title compound, (S)-N-(1-((3P)-3-(4-chloro-1-methyl-3-(methylsulfonamido)-1H-indazol-7-yl)-7-(2-fluorophenyl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide. The sample was analyzed using LCMS Method A: retention time = 1.47 min.; observed ion = 851.7 (M+H). 1H NMR (METHANOL-d4, 500 MHz) δ 8.39 (d, 1H, J=7.7 Hz), 8.08 (t, 1H, J=1.5 Hz), 7.86 (td, 1H, J=1.5, 8.3 Hz), 7.69 (dt, 1H, J=1.8, 7.7 Hz), 7.5-7.6 (m, 1H), 7.3-7.4 (m, 4H), 6.78 (tt, 1H, J=2.2, 9.2 Hz), 6.6-6.7 (m, 2H), 6.60 (t, 1H, J=55.1 Hz), 6.06 (s, 1H), 4.9-5.0 (m, 1H), 4.6-4.7 (m, 2H), 3.6-3.7 (m, 3H), 3.5-3.5 (m, 1H), 3.2-3.3 (m, 3H), 3.1-3.2 (m, 1H), 1.4-1.5 (m, 1H), 0.8-0.9 (m, 2H), 0.5-0.6 (m, 2H)

### Preparation of Example 29: (S)-N-(1-((3P)-3-(4-chloro-1-methyl-3-(methylsulfonamido)-1H-indazol-7-yl)-7-(2-fluorophenyl)-4-oxo-3,4-dihydroquinazolin-2-yl)-2-(3,5-difluorophenyl)ethyl)-2-(3-cyclopropyl-5-(difluoromethyl)-1H-pyrazol-1-yl)acetamide.

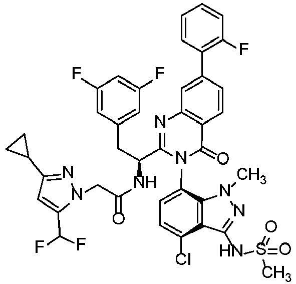

The title compound was prepared according to General Procedure I using 2-(3-cyclopropyl-5-(difluoromethyl)-1H-pyrazol-1-yl)acetic acid as the coupling partner modified as follows: the amine used was (S)-N-(7-(2-(1-amino-2-(3,5-difluorophenyl)ethyl)-7-(2-fluorophenyl)-4-oxoquinazolin-3(4H)-yl)-4-chloro-1-methyl-1H-indazol-3-yl)methanesulfonamide and immediately prior to HPLC purification the crude residue was stirred with ammonia in methanol (2M, 0.3 mL) for 10 min at room temperature. The experiment afforded the title compound, (S)-N-(1-((3P)-3-(4-chloro-1-methyl-3-(methylsulfonamido)-1H-indazol-7-yl)-7-(2-fluorophenyl)-4-oxo-3,4-dihydroquinazolin-2-yl)-2-(3,5-difluorophenyl)ethyl)-2-(3-cyclopropyl-5-(difluoromethyl)-1H-pyrazol-1-yl)acetamide. The sample was analyzed using LCMS Method A: retention time = 1.48 min.; observed ion = 851.7 (M+H). 1H NMR (METHANOL-d4, 500 MHz) δ 8.38 (br d, 1H, J=8.3 Hz), 8.09 (s, 1H), 7.86 (br d, 1H, J=8.3 Hz), 7.69 (t, 1H, J=7.7 Hz), 7.5-7.6 (m, 1H), 7.3-7.4 (m, 3H), 7.20 (br d, 1H, J=7.2 Hz), 6.6-6.8 (m, 4H), 6.23 (s, 1H), 4.6-4.6 (m, 2H), 3.61 (s, 3H), 3.4-3.5 (m, 1H), 3.2-3.3 (m, 3H), 3.19 (br s, 1H), 3.0-3.1 (m, 1H), 1.8-1.9 (m, 1H), 0.88 (br d, 2H, J=7.7 Hz), 0.6-0.7 (m, 2H)

### Preparation of Example 30: (S)-N-(1-((3P)-3-(4-chloro-1-methyl-3-(methylsulfonamido)-1H-indazol-7-yl)-7-(2-fluorophenyl)-4-oxo-3,4-dihydroquinazolin-2-yl)-2-(3,5-difluorophenyl)ethyl)-2-(1H-indol-1-yl)acetamide.

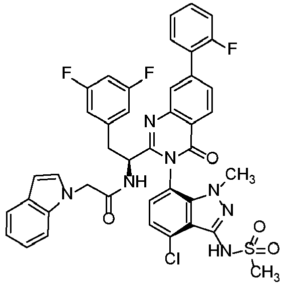

The title compound was prepared according to General Procedure I using 2-(1H-indol-1-yl)acetic acid as the coupling partner modified as follows: the amine used was (S)-N-(7-(2-(1-amino-2-(3,5-difluorophenyl)ethyl)-7-(2-fluorophenyl)-4-oxoquinazolin-3(4H)-yl)-4-chloro-1-methyl-1H-indazol-3-yl)methanesulfonamide and immediately prior to HPLC purification the crude residue was stirred with ammonia in methanol (2M, 0.3 mL) for 10 min at room temperature. The experiment afforded the title compound, (S)-N-(1-((3P)-3-(4-chloro-1-methyl-3-(methylsulfonamido)-1H-indazol-7-yl)-7-(2-fluorophenyl)-4-oxo-3,4-dihydroquinazolin-2-yl)-2-(3,5-difluorophenyl)ethyl)-2-(1H-indol-1-yl)acetamide. The sample was analyzed using LCMS Method A: retention time = 1.5 min.; observed ion = 810.8 (M+H). 1H NMR (METHANOL-d4, 500 MHz) δ 8.36 (d, 1H, J=8.0 Hz), 8.02 (t, 1H, J=1.5 Hz), 7.86 (td, 1H, J=1.5, 8.3 Hz), 7.69 (dt, 1H, J=1.8, 7.7 Hz), 7.5-7.6 (m, 2H), 7.3-7.4 (m, 4H), 7.0-7.1 (m, 2H), 6.9-7.0 (m, 2H), 6.7-6.8 (m, 1H), 6.6-6.6 (m, 2H), 6.44 (dd, 1H, J=0.9, 3.3 Hz), 4.98 (dd, 1H, J=4.8, 9.5 Hz), 4.51 (d, 2H, J=2.4 Hz), 3.5-3.5 (m, 4H), 3.25 (s, 3H), 3.10 (dd, 1H, J=9.7, 14.2 Hz)

### Preparation of Example 31: 2-((3bS,4aR)-3-(difluoromethyl)-5,5-difluoro-3b,4,4a,5-tetrahydro-1H-cyclopropa[3,4]cyclopenta[1,2-c]pyrazol-1-yl)-N-((S)-2-(3,5-difluorophenyl)-1-(3-(1,4-dimethyl-3-(methylsulfonamido)-1H-indazol-7-yl)-(3P)-7-(4-fluoro-2-methylphenyl)-4-oxo-3,4-dihydroquinazolin-2-yl)ethyl)acetamide.

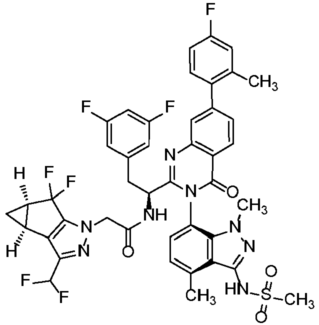

To a stirred solution of N-((S)-1-((3P)-3-(4-chloro-1-methyl-3-(methylsulfonamido)-1H-indazol-7-yl)-7-(4-fluoro-2-methylphenyl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (50 mg, 0.055 mmol) in 1,4-dioxane (3 mL) and water (0.6 mL) was added tribasic potassium phosphate (34.9 mg, 0.164 mmol) and the resulting reaction mixture was degassed with argon gas for 10 min. Then, to the reaction mixture was added 2,4,6-trimethyl-1,3,5,2,4,6-trioxatriborinane (275 mg, 1.095 mmol) followed by RuPhos-Pd-G3 (4.58 mg, 5.47 µmol) at 27 °C. The reaction mixture was heated to 100 °C and stirred for 16 hr. The progress of the reaction was monitored by TLC (SiO2, 50% EtOAc/Pet., Rf = 0.4, UV-active). On completion, the reaction mixture was diluted with EtOAc (40 mL) and filtered through a small pad of Celite. The filter pad was extracted with EtOAc (5 × 20 mL). The combined filtrate was washed with water (2 × 10 mL), dried over Na₂SO₄, filtered and then concentrated under reduced pressure to provide the crude residue as pale-yellow semi-solid (100 mg) which was purified by Prep-HPLC to afford the title compound, 2-((3bS,4aR)-3-(difluoromethyl)-5,5-difluoro-3b,4,4a,5-tetrahydro-1H-cyclopropa[3,4]cyclopenta[1,2-c]pyrazol-1-yl)-N-((S)-2-(3,5-difluorophenyl)-1-(3-(1,4-dimethyl-3-(methylsulfonamido)-1H-indazol-7-yl)-(3P)-7-(4-fluoro-2-methylphenyl)-4-oxo-3,4-dihydroquinazolin-2-yl)ethyl)acetamide. The sample was analyzed using LCMS Method I: retention time = 6.81 min.; observed ion = 893.2 (M+H). 1HNMR (400 MHz, CD3OD) δ = 8.32 (d, J = 8.4 Hz, 1H), 7.79-7.77 (m, 1H), 7.59 (dd, J = 8.1, 1.5 Hz, 1H), 7.37-7.33 (m 1H), 7.16-7.04 (m, 4H), 6.79-6.48 (m, 4H), 4.82-4.78 (m, 1H), 4.58 (q, J = 15.1 Hz, 2H), 3.60 (s, 3H), 3.48-3.40 (m, 1H), 3.19 (s, 3H), 3.04-2.98 (m, 1H), 2.86 (s, 3H), 2.42-2.36 (m, 2H), 2.35 (s, 3H), 1.34-1.31 (m, 1H), 0.99-0.95 (m, 1H)

### Preparation of Example 32: N-((S)-1-(3-(1-(2,2-difluoroethyl)-4-methyl-3-(methylsulfonamido)-1H-indazol-7-yl)-(3P)-7-(3-methylpyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

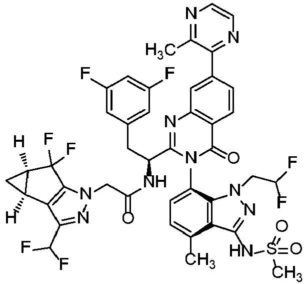

To a stirred solution of N-((S)-1-((3P)-3-(4-chloro-1-(2,2-difluoroethyl)-3-(methylsulfonamido)-1H-indazol-7-yl)-7-(3-methylpyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (20 mg, 0.021 mmol) in 1,4-dioxane (2 mL) and water (0.4 mL) was added tribasic potassium phosphate (13.44 mg, 0.063 mmol) and the resulting reaction mixture was degassed with argon gas for 10 min. Then, to the reaction mixture was added 2,4,6-trimethyl-1,3,5,2,4,6-trioxatriborinane (106 mg, 0.422 mmol) followed by RuPhos-Pd-G3 (1.766 mg, 2.111 µmol) at 27 °C. The reaction mixture was heated to 100 °C and stirred for 16 hr. The progress of the reaction was monitored by TLC (SiO2, 50% EtOAc/Pet., Rf = 0.4, UV-active). On completion, the reaction mixture was diluted with EtOAc (40 mL) and filtered through a small pad of Celite. The filter pad was extracted with with EtOAc (5 × 20 mL). The combined filterate was washed with water (2 × 10 mL), dried over Na₂SO₄, filtered and concentrated under reduced pressure to provide the crude residue as pale-yellow semi-solid (90 mg, LCMS: 60%) which was purified by Prep-HPLC to afford the title compound, N-((S)-1-(3-(1-(2,2-difluoroethyl)-4-methyl-3-(methylsulfonamido)-1H-indazol-7-yl)-(3P)-7-(3-methylpyrazin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method 1: retention time = 2.57 min.; observed ion = 927.52 (M+H). 1HNMR (400 MHz, CD3OD) δ = 8.60 (dd, J = 8.6, 2.4 Hz, 2H), 8.40 (d, J = 8.4 Hz, 1H), 8.06 (d, J = 1.3 Hz, 1H), 7.87 (dd, J = 8.3, 1.8 Hz, 1H), 7.18 (d, J = 7.4 Hz, 1H), 7.09 (d, J = 7.9 Hz, 1H), 6.79-6.51 (m, 2H), 6.44 (d, J = 6.1 Hz, 2H), 6.13-5.86 (m, 1H), 4.74-4.58 (m, 3H), 4.35-4.28 (m, 1H), 3.94-3.89 (m, 1H), 3.40-3.35 (m 1H), 3.16 (s, 3H), 3.01-2.94 (m, 1H), 2.89 (s, 3H), 2.70 (s, 3H), 2.44-2.36 (m, 2H), 1.36-1.32 (m, 1H), 0.99-0.96 (m, 1H)

### Preparation of Example 33: (S)-N-(1-((3P)-3-(4-chloro-3-(cyclopropanesulfonamido)-1-(2,2-difluoroethyl)-1H-indazol-7-yl)-4-oxo-7-(pyridin-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide.

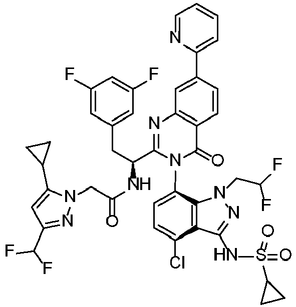

To a stirred solution of (S)-N-(1-((3P)-3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide (135 mg, 0.108 mmol) in Tetrahydrofuran (THF) (5 mL) and Water (5 mL), was added tribasic potassium phosphate (69 mg, 0.325 mmol) and 2-bromopyridine (25.7 mg, 0.163 mmol). The mixture was degassed with nitrogen gas for 10 min. To the mixture at 26°C under nitrogen atmosphere was added dichloro [9,9-dimethyl-4,5-bis(diphenylphosphino) xanthene] palladium(II) (4.10 mg, 5.42 µmol). The reaction mixture was heated to 60 °C and stirred for 5 hr. The progress of the reaction was monitored by TLC (SiO2, 80% Ethyl acetate/Pet ether. RF = 0.3). On completion of the reaction, the mixture was diluted with water (15 mL) and extracted with ethyl acetate (2 × 20 mL). The organic layer was separated and dried over sodium sulphate, filtered and concentrated under reduced pressure to provide the crude product. This material was purified by silica gel column chromatography eluting with 40% EtOAc in pet. The product-containing fractions were pooled and concentrated in vacuo to afford the title compound as a brown gummy liquid. This material was further purified by prep-HPLC to afford the title compound, (S)-N-(1-((3P)-3-(4-chloro-3-(cyclopropanesulfonamido)-1-(2,2-difluoroethyl)-1H-indazol-7-yl)-4-oxo-7-(pyridin-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide. The sample was analyzed using LCMS Method 1: retention time = 2.71 min.; observed ion = 910.35 (M+H). 1H NMR (400 MHz, METHANOL-d4) δ = 8.77 - 8.75 (m, 1H), 8.48 - 8.47 (m, 1H), 8.38 (s, 1H), 8.28 - 8.25 (m, 1H), 8.11 - 8.08 (m, 1H), 8.03 - 7.99 (m, 1H), 7.52 - 7.48 (m, 1H), 7.37 (d, J = 15.3 Hz, 2H), 6.79 - 6.55 (m, 4H), 6.06 - 6.01 (m, 2H), 4.79 - 4.78 (m, 1H), 4.74 - 4.73 (m, 2H), 4.41 - 4.36 (m, 1H), 4.01 - 3.95 (m, 1H), 3.45 - 3.40 (m, 1H), 3.13 - 3.05 (m, 1H), 2.93 - 2.87 (m, 1H), 1.50 - 1.45 (m, 1H), 1.11 - 1.07 (m, 2H), 1.00 - 0.96 (m, 2H), 0.87 - 0.78 (m, 2H), 0.58 - 0.49 (m, 2H)

### Preparation of Example 34: (S)-N-(1-((3P)-3-(4-chloro-3-(cyclopropanesulfonamido)-1-(2,2-difluoroethyl)-1H-indazol-7-yl)-4-oxo-7-(pyrimidin-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide.

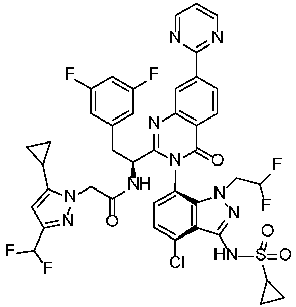

To a stirred solution of (S)-N-(1-((3P)-3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide (135 mg, 0.108 mmol) in Tetrahydrofuran (THF) (5 mL) and Water (5 mL) was added tribasic potassium phosphate (69 mg, 0.325 mmol) and 2-bromopyrimidine (25.8 mg, 0.163 mmol). The mixture was degassed with nitrogen gas for 10 min. To the mixture was added at 26°C dichloro[9,9-dimethyl-4,5-bis(diphenylphosphino) xanthene]palladium(II) (4.10 mg, 5.42 µmol) and then the reaction mixture was heated to 60 °C for 5 hr under Nitrogen atmosphere. The progress of the reaction was monitored by TLC (SiO2, 80% EtOAc/Pet. RF = 0.3). On completion of the reaction, reaction mixture was diluted with water (15 mL) and was then extracted with ethyl acetate (2 × 20 mL). The organic layer was separated and dried over sodium sulphate, filtered and the filtrated was concentrated under reduced pressure to provide the crude product as a brown gummy liquid. This material was purified by prep-HPLC to afford the title compound, (S)-N-(1-((3P)-3-(4-chloro-3-(cyclopropanesulfonamido)-1-(2,2-difluoroethyl)-1H-indazol-7-yl)-4-oxo-7-(pyrimidin-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide. The sample was analyzed using LCMS Method K: retention time = 6.64 min.; observed ion = 911.57 (M+H). 1H NMR (400 MHz, METHANOL-d4) δ = 8.99 - 8.97 (m, 2H), 8.91 - 8.89 (m, 1H), 8.71 - 8.67 (m, 1H), 8.40 - 8.37 (m, 1H), 7.51 - 7.48 (m, 1H), 7.43 - 7.37 (m, 2H), 6.79 - 6.73 (m, 1H), 6.58 - 6.46 (m, 3H), 6.12 - 5.99 (m, 2H), 4.79 - 4.74 (m, 3H), 4.42 - 4.35 (m, 1H), 4.04 - 3.94 (m, 1H), 3.45 - 3.39 (m, 1H), 3.11 - 3.04 (m, 1H), 2.94 - 2.87 (m, 1H), 1.51 - 1.46 (m, 1H), 1.12 - 1.08 (m, 2H), 1.00 - 0.96 (m, 2H), 0.89 - 0.78 (m, 2H), 0.57 - 0.52 (m, 2H)

### Preparation of Example 35: (S)-N-(1-((3P)-3-(4-chloro-3-(cyclopropanesulfonamido)-1-(2,2-difluoroethyl)-1H-indazol-7-yl)-7-(3-methylpyrazin-2-yl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide.

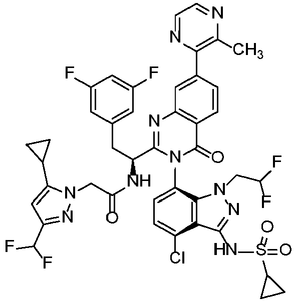

To a stirred solution of (S)-N-(1-((3P)-3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-(5-cyclopropyl-3-(difluoromethyl)-1H-pyrazol-1-yl)acetamide (70 mg, 0.045 mmol) in Tetrahydrofuran (THF) (3 mL) and Water (3 mL) were added tribasic potassium phosphate (28.3 mg, 0.134 mmol) and 2-chloro-3-methylpyrazine (8.58 mg, 0.067 mmol). The mixture was degassed with nitrogen gas for 10 min. Then to the mixture was added at 26°C under nitrogen atmosphere dichloro[9,9-dimethyl-4,5-bis(diphenylphosphino)xanthene]palladium(II) (1.683 mg, 2.226 µmol). The reaction mixture was heated at 60 °C and stirred for 5 hr. The progress of the reaction was monitored by TLC (SiO2, 80% EtOAc/Pet. Rf = 0.3). On completion of the reaction, the reaction mixture was diluted with water (15 mL) and extracted with ethyl acetate (2 × 20 mL). The organic layer was separated, dried over sodium sulphate, filtered and the filtrate was concentrated under reduced pressure to afford the crude product. This material was purified by prep-HPLC to afford the title compound, (S)-N-(1-((3P)-3-(4-chloro-3-(cyclopropanesulfonamido)-1-(2,2-difluoroethyl)-1H-indazol-7-yl)-7-(3-methylpyrazin-2-yl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide. The sample was analyzed using LCMS Method 1: retention time = 2.61 min.; observed ion = 925.31 (M+H). 1H NMR (400 MHz, METHANOL-d4) δ = 8.63 - 8.59 (m, 2H), 8.43 - 8.40 (m, 1H), 8.09 - 8.07 (m, 1H), 7.91 - 7.87 (m, 1H), 7.42 - 7.34 (m, 2H), 6.78 - 6.72 (m, 1H), 6.58 - 6.44 (m, 3H), 6.14 - 5.96 (m, 2H), 4.80 - 4.78 (m, 1H), 4.73 - 4.71 (m, 2H), 4.45 - 4.35 (m, 1H), 4.06 - 3.96 (m, 1H), 3.44 - 3.38 (m, 1H), 3.10 - 3.03 (m, 1H), 2.94 - 2.87 (m, 1H), 2.70 (s, 3H), 1.50 - 1.44 (m, 1H), 1.12 - 1.08 (m, 2H), 1.01 - 0.97 (m, 2H), 0.85 - 0.80 (m, 2H), 0.58 - 0.50 (m, 2H)

### IUPAC Chemical Names:

The IUPAC chemical names for each example are listed below. At this time these names are not recognized by common software such tools such as ChemDraw or JChem. Therefore, the chemical names used throughout the Examples section above were generated with ChemDraw with P/M nomenclature manually inserted. The chemical names can be converted to chemical structures using ChemDraw after the P/M nomenclature-e.g., "(3P)-"-is removed.

| **Example** | **IUPAC Name** |
|---|---|
| Example 1 | 2-[3,5-bis(trifluoromethyl)-1H-pyrazol-1-yl]-N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]acetamide |
| Example 2 | N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]-2-(3-cyclopropyl-1H-indazol-1-yl)acetamide |
| Example 3 | 2-[3,5-bis(trifluoromethyl)-1H-pyrazol-1-yl]-N-[(1S)-1-[(3P)-3-(4-chloro-3-methanesulfonamido-1-methyl-1H-indazol-7-yl)-7-(4,6-dimethylpyrimidin-2-yl)-4-oxo-3,4-dihydroquinazolin-2-yl]-2-(3,5-difluorophenyl)ethyl]acetamide |
| Example 4 | N-[(1S)-1-[(3P)-3-(4-chloro-3-methanesulfonamido-1-methyl-1H-indazol-7-yl)-7-(4,6-dimethylpyrimidin-2-yl)-4-oxo-3,4-dihydroquinazolin-2-yl]-2-(3,5-difluorophenyl)ethyl]-2-(3-cyclopropyl-1H-indazol-1-yl)acetamide |
| Example 5 | 2-(3-tert-butyl-1H-^{p}yrazol-1-yl)-N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]acetamide |
| Example 6 | 2-[(2S,4R)-9-(difluoromethyl)-5,5-difluoro-7,8-diazatricyclo[4.3.0.0²,⁴]nona-1(6),8-dien-7-yl]-N-[2-(3,5-difluorophenyl)-1-[(3P)-3-(3-methanesulfonamido-1,4-dimethyl-1H-indazol-7-yl)-7-(3-methylpyrazin-2-yl)-4-oxo-3,4-dihydroquinazolin-2-yl]ethyl]acetamide |
| Example 7 | N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]-2-[3-(difluoromethyl)-1H-indazol-1-yl]acetamide |
| Example 8 | N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]-2-[1-(propan-2-yl)-1H-indol-3-yl]acetamide |
| Example 9 | N-[(1S)-1-[(3P)-3-(4-chloro-3-methanesulfonamido-1-methyl-1H-indazol-7-yl)-7-(4,6-dimethylpyrimidin-2-yl)-4-oxo-3,4-dihydroquinazolin-2-yl]-2-(3,5-difluorophenyl)ethyl]-2-[5-cyclopropyl-3-(trifluoromethyl)-1H-pyrazol-1-yl]acetamide |
| Example 10 | N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]-2-(3-cyclopropyl-5-methyl-1H-pyrazol-1-yl)acetamide |
| Example 11 | N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]-2-(3-cyclobutyl-1H-pyrazol-1-yl)acetamide |
| Example 12 | N-[(1S)-1-[(3P)-3-(4-chloro-3-methanesulfonamido-1-methyl-1H-indazol-7-yl)-7-(3-methylpyrazin-2-yl)-4-oxo-3,4-dihydroquinazolin-2-yl]-2-(3,5-difluorophenyl)ethyl]-2-[5-methyl-3-(trifluoromethyl)-1H-pyrazol-1-yl]acetamide |
| Example 13 | N-[(1S)-1-[(3P)-3-[4-chloro-3-cyclopropanesulfonamido-1-(2,2-difluoroethyl)-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-indazol-1-yl)acetamide |
| Example 14 | 2-(3-tert-butyl-1H-pyrazol-1-yl)-N-[(1S)-1-[(3P)-3-(4-chloro-3-methanesulfonamido-1-methyl-1H-indazol-7-yl)-7-(2-fluorophenyl)-4-oxo-3H,4H-pyrido[2,3-d]pyrimidin-2-yl]-2-(3,5-difluorophenyl)ethyl]acetamide |
| Example 15 | N-[(1S)-1-[(3P)-3-(4-chloro-3-methanesulfonamido-1-methyl-1H-indazol-7-yl)-7-(2-fluorophenyl)-4-oxo-3H,4H-pyrido[2,3-d]pyrimidin-2-yl]-2-(3,5-difluorophenyl)ethyl]-2-(3-cyclopropyl-1H-indazol-1-yl)acetamide |
| Example 16 | N-[(1S)-1-[(3P)-3-[4-chloro-1-(2,2-difluoroethyl)-3-methanesulfonamido-1H-indazol-7-yl]-7-(2-fluorophenyl)-4-oxo-3H,4H-pyrido[2,3-d]pyrimidin-2-yl]-2-(3,5-difluorophenyl)ethyl]-2-[5-cyclopropyl-3-(difluoromethyl)-1H-pyrazol-1-yl]acetamide |
| Example 17 | N-[(1S)-1-[(3P)-3-[4-chloro-1-(2,2-difluoroethyl)-3-methanesulfonamido-1H-indazol-7-yl]-7-(2-fluorophenyl)-4-oxo-3H,4H-pyrido[2,3-d]pyrimidin-2-yl]-2-(3,5-difluorophenyl)ethyl]-2-[3-cyclopropyl-5-(difluoromethyl)-1H-pyrazol-1-yl]acetamide |
| Example 18 | N-[(1S)-1-[(3P)-3-[4-chloro-1-(2,2-difluoroethyl)-3-methanesulfonamido-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-indazol-1-yl)acetamide |
| Example 19 | N-[(1S)-1-[(3P)-3-(4-chloro-3-methanesulfonamido-1-methyl-1H-indazol-7-yl)-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-2-methylpropanamide |
| Example 20 | N-[(1S)-1-[(3P)-3-[4-chloro-1-(2,2-difluoroethyl)-3-methanesulfonamido-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-2-methylpropanamide |
| Example 21 | N-[(1S)-1-[(3P)-3-[4-chloro-3-cyclopropanesulfonamido-1-(2,2-difluoroethyl)-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-2-methylpropanamide |
| Example 22 | N-[(1S)-1-[(3P)-3-[4-chloro-3-methanesulfonamido-1-(2,2,2-trifluoroethyl)-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-2-methylpropanamide |
| Example 23 | (2S)-N-[(1S)-1-[(3P)-3-[4-chloro-3-methanesulfonamido-1-(2,2,2-trifluoroethyl)-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-3-methylbutanamide |
| Example 24 | N-[(1S)-1-[(3P)-3-[4-chloro-3-cyclopropanesulfonamido-1-(2,2-difluoroethyl)-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-3-methylbutanamide |
| Example 25 | (2S)-N-[(1S)-1-[(3P)-3-[4-chloro-1-(2,2-difluoroethyl)-3-methanesulfonamido-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-3-methylbutanamide |
| Example 26 | (2S)-N-[(1S)-1-[(3P)-3-[4-chloro-3-methanesulfonamido-1-(2,2,2-trifluoroethyl)-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-3-methylbutanamide |
| Example 27 | N-[(1S)-1-[(3P)-3-[4-chloro-1-(2,2-difluoroethyl)-3-methanesulfonamido-1H-indazol-7-yl]-7-(2-fluorophenyl)-4-oxo-3H,4H-pyrido[2,3-d]pyrimidin-2-yl]-2-(3,5-difluorophenyl)ethyl]-2-(3-cyclopropyl-1H-pyrazol-1-yl)-3-methylbutanamide |
| Example 28 | N-[(1S)-1-[(3P)-3-(4-chloro-3-methanesulfonamido-1-methyl-1H-indazol-7-yl)-7-(2-fluorophenyl)-4-oxo-3,4-dihydroquinazolin-2-yl]-2-(3,5-difluorophenyl)ethyl]-2-[5-cyclopropyl-3-(difluoromethyl)-1H-pyrazol-1-yl]acetamide |
| Example 29 | N-[(1S)-1-[(3P)-3-(4-chloro-3-methanesulfonamido-1-methyl-1H-indazol-7-yl)-7-(2-fluorophenyl)-4-oxo-3,4-dihydroquinazolin-2-yl]-2-(3,5-difluorophenyl)ethyl]-2-[3-cyclopropyl-5-(difluoromethyl)-1H-pyrazol-1-yl]acetamide |
| Example 30 | N-[(1S)-1-[(3P)-3-(4-chloro-3-methanesulfonamido-1-methyl-1H-indazol-7-yl)-7-(2-fluorophenyl)-4-oxo-3,4-dihydroquinazolin-2-yl]-2-(3,5-difluorophenyl)ethyl]-2-(1H-indol-1-yl)acetamide |
| Example 31 | 2-[(2S,4R)-9-(difluoromethyl)-5,5-difluoro-7,8-diazatricyclo[4.3.0.0²,⁴]nona-1(6),8-dien-7-yl]-N-[(1S)-2-(3,5-difluorophenyl)-1-[(3P)-7-(4-fluoro-2-methylphenyl)-3-(3-methanesulfonamido-1,4-dimethyl-1H-indazol-7-yl)-4-oxo-3,4-dihydroquinazolin-2-yl]ethyl]acetamide |
| Example 32 | N-[(1S)-1-[(3P)-3-[1-(2,2-difluoroethyl)-3-methanesulfonamido-4-methyl-1H-indazol-7-yl]-7-(3-methylpyrazin-2-yl)-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴1nona-1(6),8-dien-7-yl]acetamide |
| Example 33 | N-[(1S)-1-[(3P)-3-[4-chloro-3-cyclopropanesulfonamido-1-(2,2-difluoroethyl)-1H-indazol-7-yl]-4-oxo-7-(pyridin-2-yl)-3,4-dihydroquinazolin-2-yl]-2-(3,5-difluorophenyl)ethyl]-2-[5-cyclopropyl-3-(difluoromethyl)-1H-pyrazol-1-yl]acetamide |
| Example 34 | N-[(1S)-1-[(3P)-3-[4-chloro-3-cyclopropanesulfonamido-1-(2,2-difluoroethyl)-1H-indazol-7-yl]-4-oxo-7-(pyrimidin-2-yl)-3,4-dihydroquinazolin-2-yl]-2-(3,5-difluorophenyl)ethyl]-2-[5-cyclopropyl-3-(difluoromethyl)-1H-pyrazol-1-yl]acetamide |
| Example 35 | N-[(1S)-1-[(3P)-3-[4-chloro-3-cyclopropanesulfonamido-1-(2,2-difluoroethyl)-1H-indazol-7-yl]-7-(3-methylpyrazin-2-yl)-4-oxo-3,4-dihydroquinazolin-2-yl]-2-(3,5-difluorophenyl)ethyl]-2-[5-cyclopropyl-3-(difluoromethyl)-1H-pyrazol-1-yl]acetamide |

### Biological Methods:

HIV cell culture assay - MT-2 cells, 293T cells and the proviral DNA clone of NL₄₋₃ virus were obtained from the NIH AIDS Research and Reference Reagent Program. MT-2 cells were propagated in RPMI 1640 media supplemented with 10% heat inactivated fetal bovine serum (FBS), 100 mg/ml penicillin G and up to 100 units/mL streptomycin. The 293T cells were propagated in DMEM media supplemented with 10% heat inactivated FBS, 100 mg/mL penicillin G and 100 mg/mL streptomycin. A recombinant NL₄₋₃ proviral clone, in which a section of the nef gene was replaced with the Renilla luciferase gene, was used to make the reference virus used in these studies. The recombinant virus was prepared through transfection of the recombinant NL₄₋₃ proviral clone into 293T cells using Transit-293 Transfection Reagent from Mirus Bio LLC (Madison, WI). Supernatent was harvested after 2-3 days and the amount of virus present was titered in MT-2 cells using luciferase enzyme activity as a marker by measuring luciferase enzyme activity. Luciferase was quantitated using the EnduRen Live Cell Substrate from Promega (Madison, WI). Antiviral activities of compounds toward the recombinant virus were quantified by measuring luciferase activity in MT-2 cells infected for 4-5 days with the recombinant virus in the presence of serial dilutions of the compound.

The 50% effective concentration (EC₅₀) was calculated by using the exponential form of the median effect equation where (Fa) = 1/[1+(ED₅₀/drug conc.)m] (Johnson VA, Byington RT. Infectivity Assay. In Techniques in HIV Research. ed. Aldovini A, Walker BD. 71-76. New York: Stockton Press.1990). The 50% inhibitory concentration (EC₅₀) was calculated by using the exponential form of the median effect equation where percent inhibition = 1/[1 + (EC_{50/}drug concentration)*m*], where *m* is a parameter that reflects the slope of the concentration-response curve.

Compound cytotoxicity and the corresponding CC₅₀ values were determined using the same protocol as described in the antiviral assay except that uninfected cells were used. Cytotoxicity was assessed on day 4 in uninfected MT2 cells by using a XTT (2,3-bis[2-Methoxy-4-nitro-5-sulfophenyl]-2H-tetrazolium-5-carboxyanilide inner salt)-based colorimetric assay (Sigma-Aldrich, St Louis, Mo).

| Example | EC₅₀ nM | CC₅₀ µM |
|---|---|---|
| Example 1 | 1.9 | > 0.5 |
| Example 2 | 0.21 | > 0.5 |
| Example 3 | 3.6 | > 0.5 |
| Example 4 | 0.91 | > 0.5 |
| Example 5 | 0.65 | > 0.5 |
| Example 6 | 0.056 | > 0.5 |
| Example 7 | 0.38 | > 0.5 |
| Example 8 | 0.45 | > 0.5 |
| Example 10 | 0.21 | > 0.5 |
| Example 11 | 0.39 | > 0.5 |
| Example 12 | 0.49 | > 0.5 |
| Example 13 | 0.47 | > 0.5 |
| Example 14 | 0.53 | > 0.5 |
| Example 15 | 0.17 | > 0.5 |
| Example 16 | 0.37 | > 0.5 |
| Example 17 | 0.52 | > 0.5 |
| Example 18 | 0.17 | > 0.5 |
| Example 19 | 0.22 | > 0.5 |
| Example 20 | 0.96 | > 0.5 |
| Example 21 | 2.3 | > 0.5 |
| Example 22 | 0.80 | > 0.5 |
| Example 23 | 0.21 | > 0.5 |
| Example 24 | 3.9 | > 0.5 |
| Example 26 | 1.9 | > 0.5 |
| Example 28 | 2.2 | > 0.5 |
| Example 29 | 3.1 | > 0.5 |
| Example 30 | 3.0 | > 0.5 |
| Example 31 | 2.3 | > 0.5 |
| Example 32 | 0.063 | > 0.5 |
| Example 33 | 0.79 | > 0.5 |
| Example 34 | 0.40 | > 0.5 |
| Example 35 | 0.36 | > 0.5 |

The disclosure is not limited to the foregoing illustrative examples and the examples should be considered in all respects as illustrative and not restrictive, reference being made to the appended claims, rather than to the foregoing examples, and all changes which come within the meaning and range of equivalency of the claims are therefore intended to be embraced.

## Claims

1. A compound or salt selected from the group consisting of: 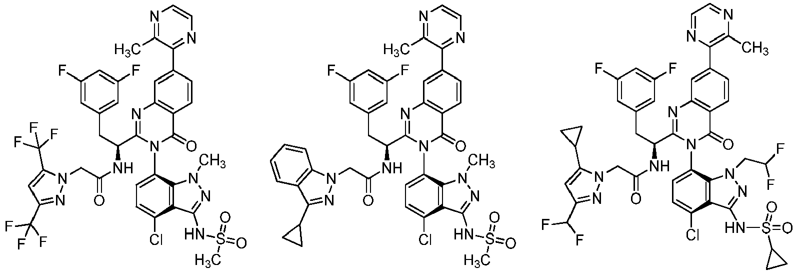 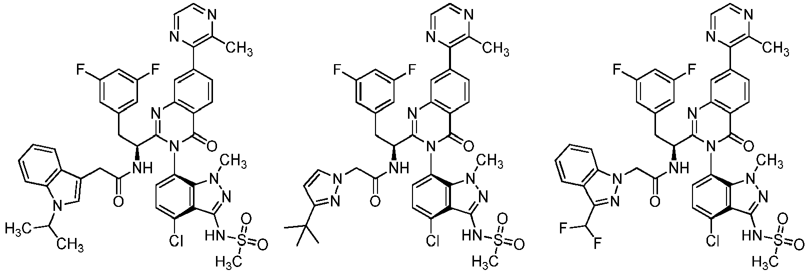 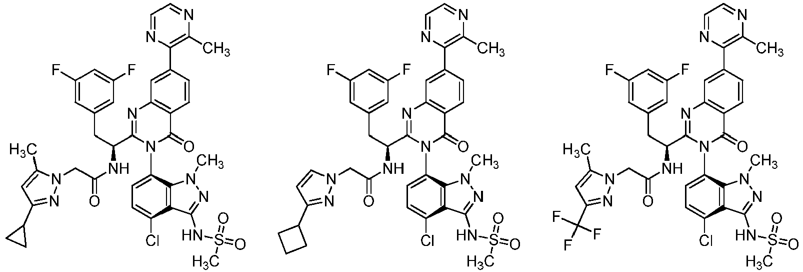 and pharmaceutically acceptable salts thereof.

2. A compound or salt selected from the group consisting of: 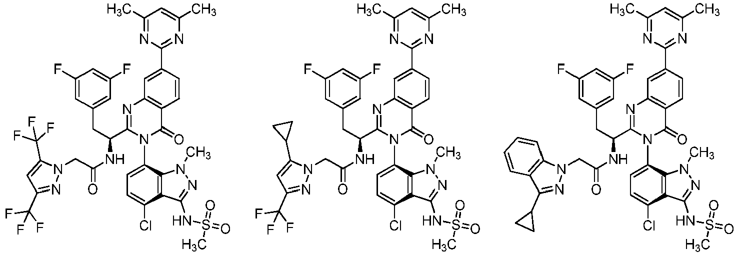 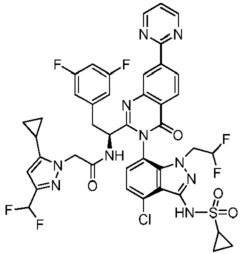 and pharmaceutically acceptable salts thereof.

3. A compound or salt selected from the group consisting of: 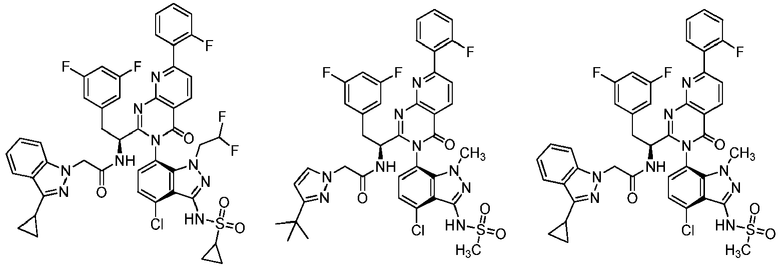 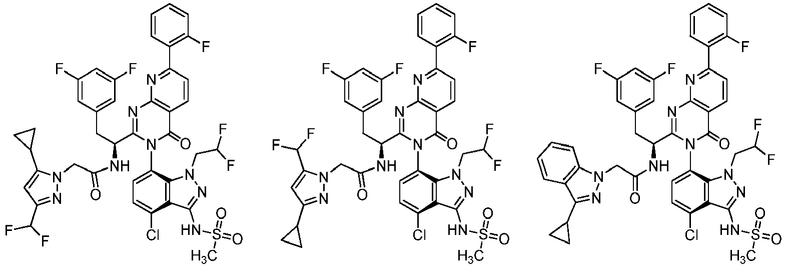 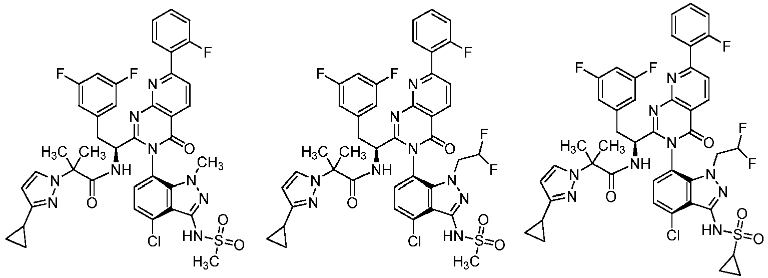 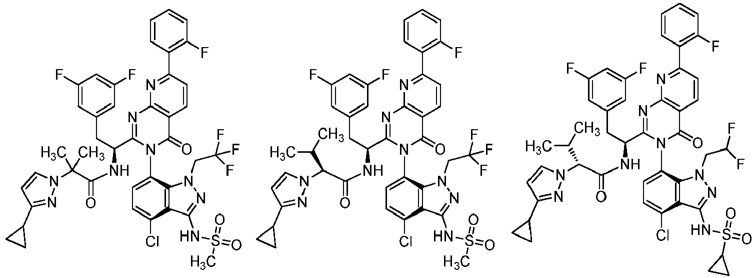 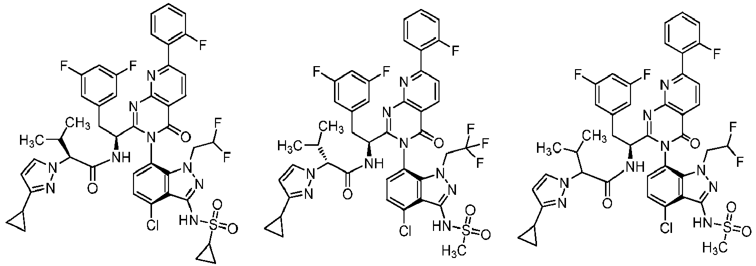 and pharmaceutically acceptable salts thereof.

4. A compound or salt selected from the group consisting of: 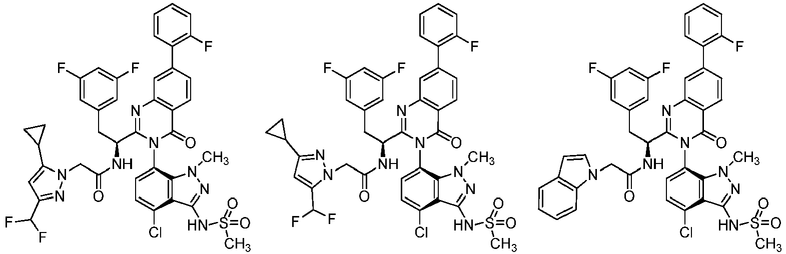 and pharmaceutically acceptable salts thereof.

5. A compound or salt selected from the group consisting of: 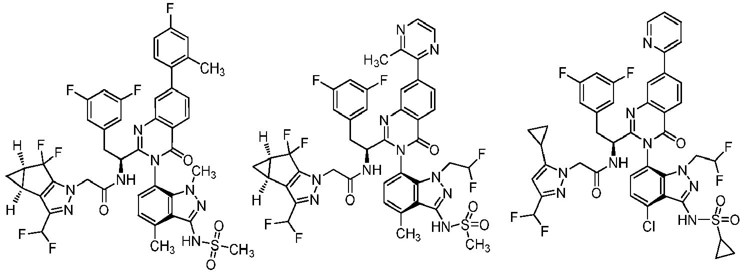 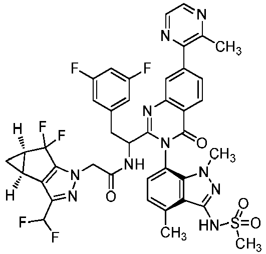 and pharmaceutically acceptable salts thereof.

6. A pharmaceutical composition comprising a compound or salt according to any of claims 1-5.

7. A pharmaceutical composition according to claim 6 further comprising a pharmaceutically acceptable excipient.

8. A pharmaceutical composition according to claim 6 or claim 7 suitable for oral administration, for intramuscular injection, or for subcutaneous injection.

9. A compound or pharmaceutically acceptable salt thereof according to any of claims 1-5 for use in therapy.

10. A compound or pharmaceutically acceptable salt thereof according to any of claims 1-5 for use in treating HIV infection in a human.

11. The compound or pharmaceutically acceptable salt thereof for use according to claim 9 or 10 wherein the compound or pharmaceutically acceptable salt thereof is administered orally.

12. The compound or pharmaceutically acceptable salt thereof for use according to claim 9 or 10 wherein administration is by intramuscular injection or subcutaneous injection.

13. The compound or pharmaceutically acceptable salt thereof for use according to claim 9 or 10 wherein said use further comprises administration of at least one other agent used for treatment of HIV infection in a human.

14. The compound or pharmaceutically acceptable salt thereof for use according to claim 13 wherein said at least one other agent is selected from the group consisting of dolutegravir, bictegravir, lamivudine, fostemsavir, and cabotegravir.

## Patentansprüche

1. Verbindung oder Salz, ausgewählt aus der Gruppe, bestehend aus: 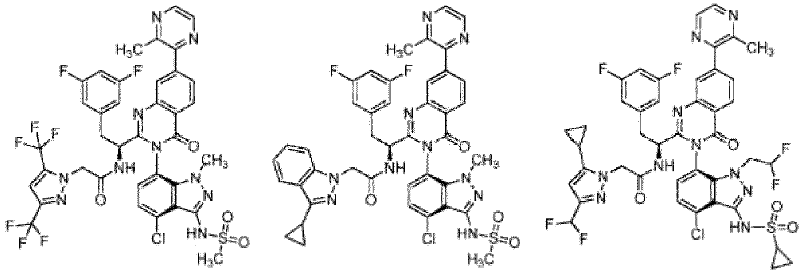 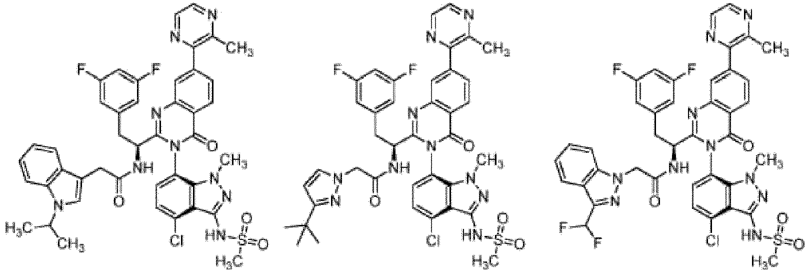 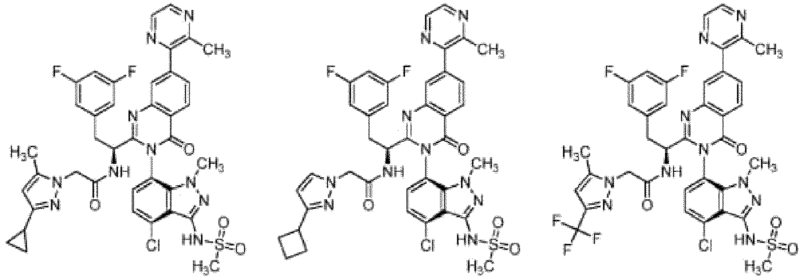 und pharmazeutisch akzeptable Salze davon.

2. Verbindung oder Salz, ausgewählt aus der Gruppe, bestehend aus: 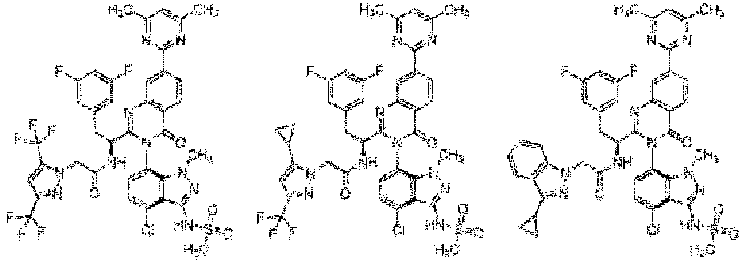 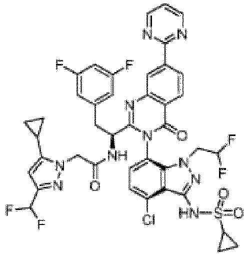 und pharmazeutisch akzeptable Salze davon.

3. Verbindung oder Salz, ausgewählt aus der Gruppe, bestehend aus: 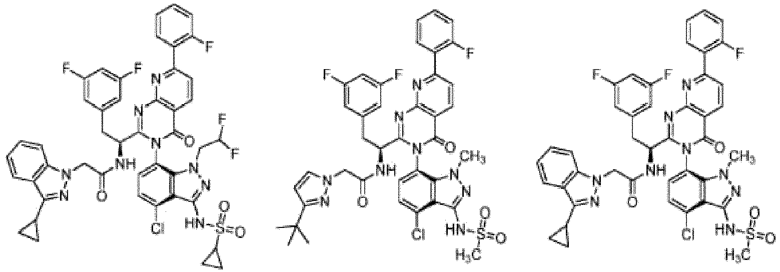 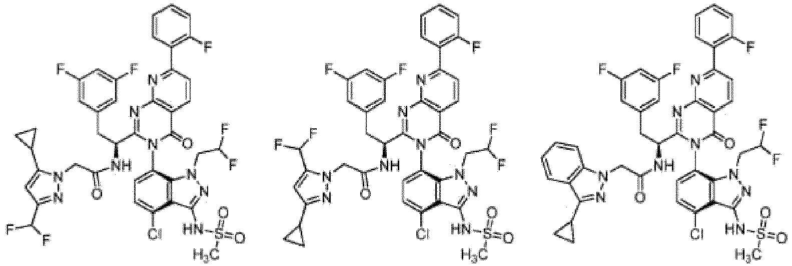 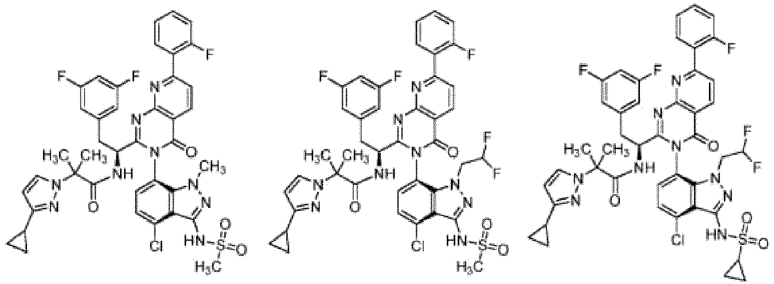 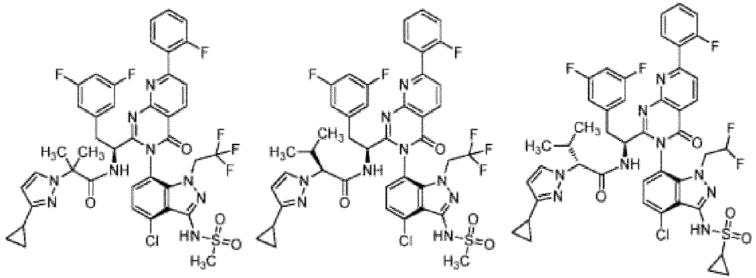 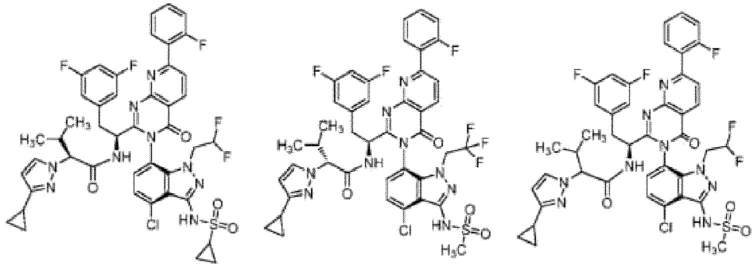 und pharmazeutisch akzeptable Salze davon.

4. Verbindung oder Salz, ausgewählt aus der Gruppe, bestehend aus: 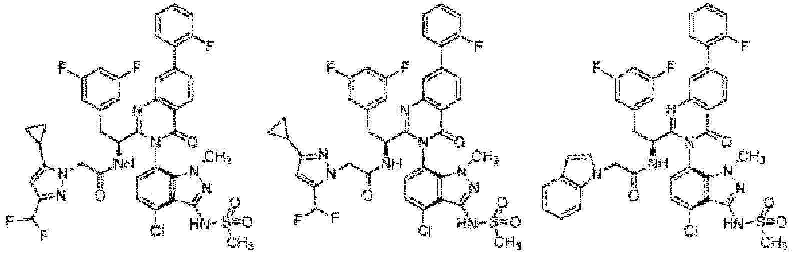 und pharmazeutisch akzeptable Salze davon.

5. Verbindung oder Salz, ausgewählt aus der Gruppe, bestehend aus: 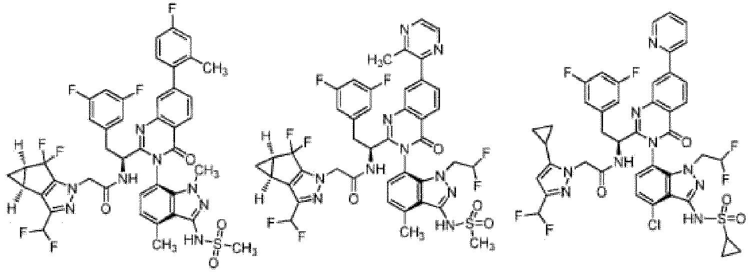 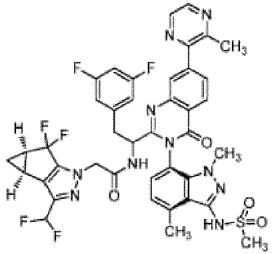 und pharmazeutisch akzeptable Salze davon.

6. Pharmazeutische Zusammensetzung, welche eine Verbindung oder ein Salz nach irgendeinem der Ansprüche 1-5 umfasst.

7. Pharmazeutische Zusammensetzung gemäß Anspruch 6, welche weiterhin einen pharmazeutisch akzeptablen Hilfsstoff umfasst.

8. Pharmazeutische Zusammensetzung gemäß Anspruch 6 oder Anspruch 7, die für die orale Verabreichung, für die intramuskuläre Injektion oder für die subkutane Injektion geeignet ist.

9. Verbindung oder pharmazeutisch akzeptables Salz davon gemäß irgendeinem der Ansprüche 1-5 zur Verwendung in der Therapie.

10. Verbindung oder pharmazeutisch akzeptables Salz davon gemäß irgendeinem der Ansprüche 1-5 zur Verwendung bei der Behandlung von HIV-Infektion in einem Menschen.

11. Verbindung oder pharmazeutisch akzeptables Salz davon zur Verwendung gemäß Anspruch 9 oder 10, wobei die Verbindung oder das pharmazeutische Salz davon oral verabreicht wird.

12. Verbindung oder pharmazeutisch akzeptables Salz davon zur Verwendung gemäß Anspruch 9 oder 10, wobei die Verabreichung durch intramuskuläre Injektion oder subkutane Injektion erfolgt.

13. Verbindung oder pharmazeutisch akzeptables Salz davon zur Verwendung gemäß0 Anspruch 9 oder 10, wobei die Verwendung weiterhin die Verabreichung wenigstens eines anderen Agens umfasst, welches für die Behandlung von HIV-Infektion in einem Menschen verwendet wird.

14. Verbindung oder pharmazeutisch akzeptables Salz davon zur Verwendung gemäß Anspruch 13, wobei das wenigstens eine andere Agens aus der Gruppe ausgewählt ist, bestehend aus Dolutegravir, Bictegravir, Lamivudin, Fostemsavir und Cabotegravir.

## Revendications

1. Composé ou sel choisi dans le groupe consistant en: 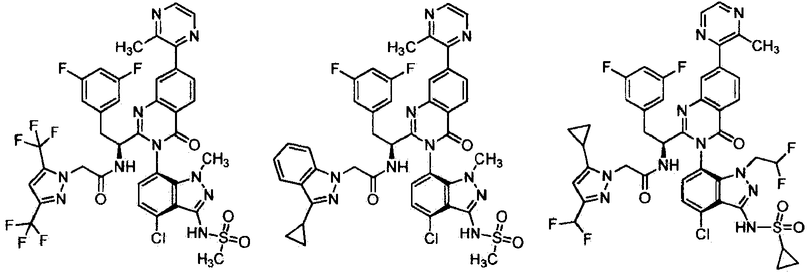 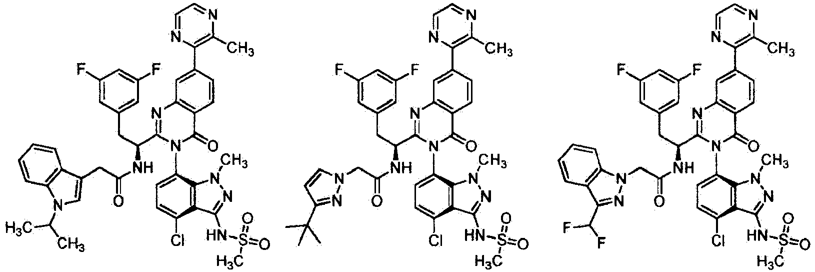 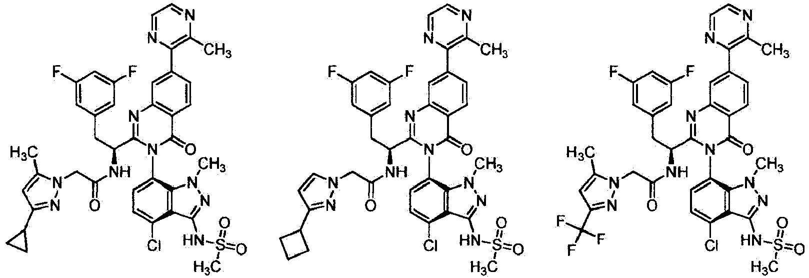 et ses sels pharmaceutiquement acceptables.

2. Composé ou sel choisi dans le groupe consistant en: 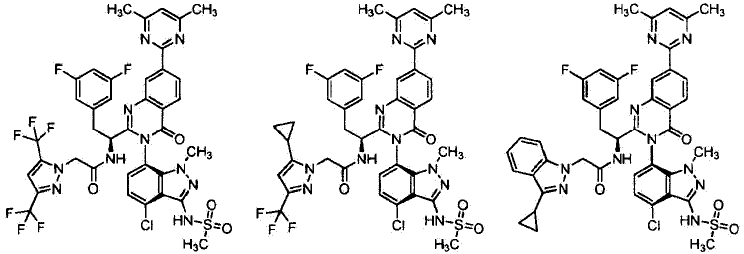 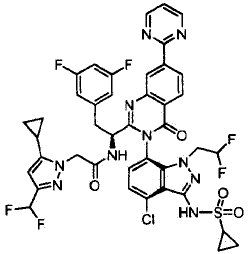 et ses sels pharmaceutiquement acceptables.

3. Composé ou sel choisi dans le groupe consistant en: 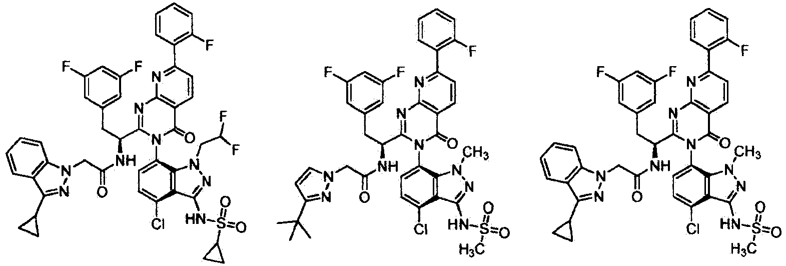 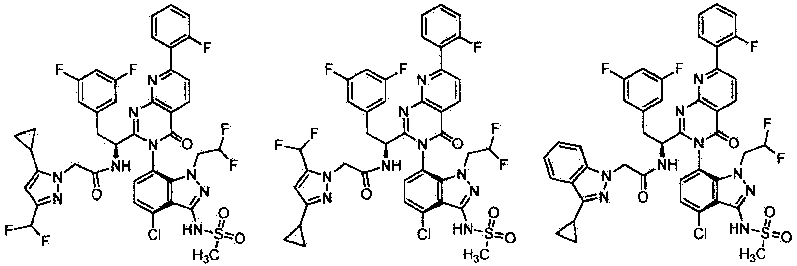 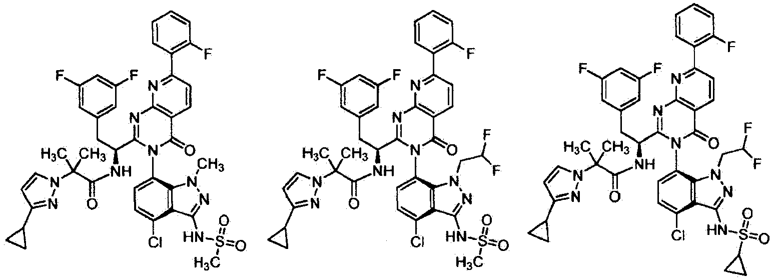 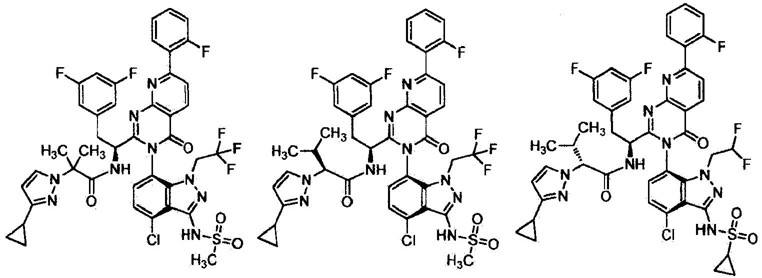 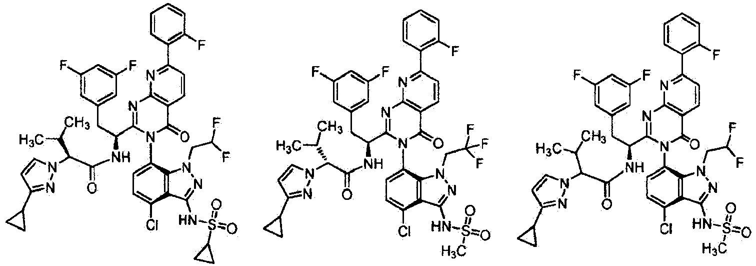 et ses sels pharmaceutiquement acceptables.

4. Composé ou sel choisi dans le groupe consistant en: 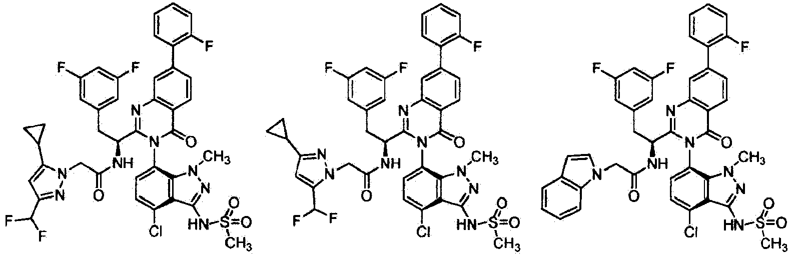 et ses sels pharmaceutiquement acceptables.

5. Composé ou sel choisi dans le groupe consistant en: 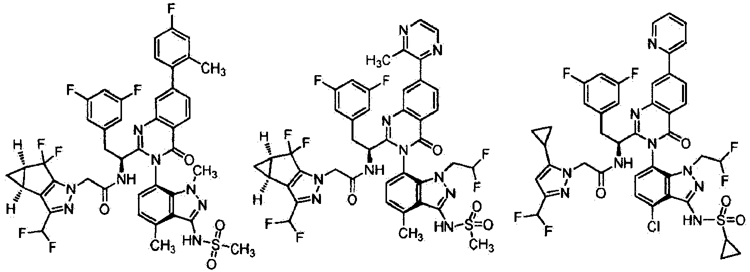 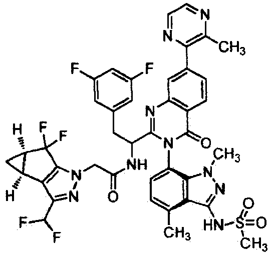 et ses sels pharmaceutiquement acceptables.

6. Composition pharmaceutique comprenant un composé ou sel selon l'une quelconque des revendications 1 à 5.

7. Composition pharmaceutique selon la revendication 6 comprenant en outre un excipient pharmaceutiquement acceptable.

8. Composition pharmaceutique selon la revendication 6 ou la revendication 7 appropriée à l'administration orale, à l'injection intramusculaire ou à l'injection sous-cutanée.

9. Composé ou sel pharmaceutiquement acceptable de celui-ci selon l'une quelconque des revendications 1 à 5 destiné à être utilisé en thérapie.

10. Composé ou sel pharmaceutiquement acceptable de celui-ci selon l'une quelconque des revendications 1 à 5 destiné à être utilisé dans le traitement d'une infection par le VIH chez un être humain.

11. Composé ou sel pharmaceutiquement acceptable de celui-ci destiné à être utilisé selon la revendication 9 ou 10 où le composé ou sel pharmaceutiquement acceptable de celui-ci est administré oralement.

12. Composé ou sel pharmaceutiquement acceptable de celui-ci destiné à être utilisé selon la revendication 9 ou 10 où l'administration est par injection intramusculaire ou injection sous-cutanée.

13. Composé ou sel pharmaceutiquement acceptable de celui-ci destiné à être utilisé selon la revendication 9 ou 10 où ladite utilisation comprend en outre l'administration d'au moins un autre agent utilisé pour le traitement d'une infection par le VIH chez un être humain.

14. Composé ou sel pharmaceutiquement acceptable de celui-ci destiné à être utilisé selon la revendication 13 où ledit au moins un autre agent est choisi dans le groupe consistant en dolutégravir, bictégravir, lamivudine, fostemsavir et cabotégravir.
